(19) Europäisches Patentamt
European Patent Office
Office européen des brevets

(11) Veröffentlichungsnummer: **0 468 231 B1**

# (12) EUROPÄISCHE PATENTSCHRIFT

(45) Veröffentlichungstag der Patentschrift: **21.09.94**

(21) Anmeldenummer: **91110928.8**

(22) Anmeldetag: **02.07.91**

(51) Int. Cl.5: **C07C 279/12**, C07C 279/14, C07D 211/26, C07D 265/30, C07D 401/12, C07D 401/14, C07D 413/12, A61K 31/495

## (54) Guanidine.

(30) Priorität: **05.07.90 CH 2250/90**
**02.05.91 CH 1315/91**

(43) Veröffentlichungstag der Anmeldung:
**29.01.92 Patentblatt 92/05**

(45) Bekanntmachung des Hinweises auf die Patenterteilung:
**21.09.94 Patentblatt 94/38**

(84) Benannte Vertragsstaaten:
**AT BE CH DE DK ES FR GB GR IT LI LU NL SE**

(56) Entgegenhaltungen:
**EP-A- 0 352 249**
**US-A- 4 227 006**

**CHEMICAL ABSTRACTS, Band 93, Nr. 21, 24. November 1980, Zusammenfassung Nr. 204281x, Columbus, Ohio, US; & JP-A-8064561**

(73) Patentinhaber: **F. HOFFMANN-LA ROCHE AG**
**Grenzacherstrasse 124**
**CH-4002 Basel (CH)**

(72) Erfinder: **Ackermann, Jean, Dr.**
**Elsässerstrasse 137**
**CH-4056 Basel (CH)**
Erfinder: **Banner, David, Dr.**
**Neubadstrasse 129**
**CH-4054 Basel (CH)**
Erfinder: **Gubernator, Klaus, Dr.**
**Carl-Maria-von-Weber-Strasse**
**W-7800 Freiburg (DE)**
Erfinder: **Hadvary, Paul, Dr.**
**Neumattenweg 8**
**CH-4105 Biel-Benken (CH)**
Erfinder: **Hilpert, Kurt, Dr.**
**Eichenstrasse 5**
**CH-4114 Hofstetten (CH)**
Erfinder: **Müller, Klaus, Prof.Dr.**
**Grellingerstrasse 5**
**CH-4142 Münchenstein (CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 468 231 B1

**CHEMICAL ABSTRACTS, Band 90, Nr. 1, 1979, Zusammenfassung Nr. 260t, Columbus, Ohio, US**

Erfinder: **Labler, Ludvik, Dr.**
**Stallenrain 12**
**CH-4103 Bottmingen (CH)**
Erfinder: **Schmid, Gérard, Dr.**
**Leibern**
**CH-4468 Kienberg (CH)**
Erfinder: **Tschopp, Thomas, Dr.**
**Juraweg 2**
**CH-4107 Ettingen (CH)**
Erfinder: **Wessel, Hans Peter, Dr.**
**Im Backacker 7**
**W-7843 Heitersheim 2 (DE)**
Erfinder: **Wirz, Beat, Dr.**
**Wiedenweg 4**
**Ch-4153 Reinach (CH)**

(74) Vertreter: **Lederer, Franz, Dr. et al**
**Lederer, Keller & Riederer**
**Patentanwälte**
**Prinzregentenstrasse 16**
**D-80538 München (DE)**

## Beschreibung

Die Erfindung betrifft neue Guanidine der Formel

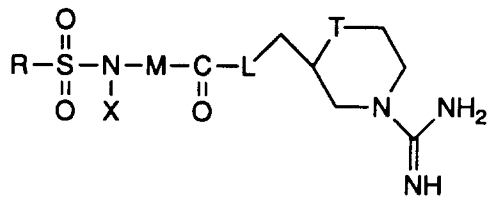

I

worin

| | |
|---|---|
| R | Aryl, Heteroaryl oder Heterocyclyl, |
| T | $CH_2$ oder O, |
| L | NH oder O und |
| -N(X)-M- | eine Gruppe $-N(SO_2-R^o)-CH_2-$ oder eine gegebenenfalls im Phenylring substituierte Isochinolylengruppe ist, und |
| $R^o$ | die gleichen Bedeutungen wie R hat, oder |
| X | H, $-CH_2COOH$, $-CH_2COO-C_{1-4}$-Alkyl, $-CH_2CO$-(Tetra- bis Heptamethylenimino) oder gegebenenfalls N-mono- oder N-di-$C_{1-4}$-alkyliertes $-CH_2CONH_2$, und |
| M | eine Gruppe $R'-(CH_2)_{1-2}CH=$, $R'-COCH_2CH=$, $R''-COCH_2CH=$, $R'-(CO)_{1-2}NHCH_2CH=$, Benzyl-$OCONHCH_2CH=$, $-CH_2[R'-(CO)_{1-2}NH]CH-$, $-CH_2$(Benzyl-OCONH)CH- oder -CH(CO-Q)$CH_2$-, |
| R' | Aryl, Heteroaryl, Cycloalkyl oder Heterocyclyl, |
| R'' | Tetra- bis Heptamethylenimino gegebenenfalls mit bis zu 2 Substituenten aus der Gruppe von Oxo, $-COO-C_{1-4}$-Alkyl, $-(CH_2)_{0-1}OH$, $-(CH_2)_{0-1}OCO-C_{1-4}$-Alkyl und gegebenenfalls mono- oder di-$C_{1-4}$-alkyliertes Carbamoyl, und |
| Q | Benzylamino oder eine gegebenenfalls durch ein O- oder S-Atom unterbrochene und gegebenenfalls durch bis zu 2 Substituenten aus der Gruppe von $C_{1-4}$-Alkyl, COOH, $-COO-C_{1-4}$-Alkyl, $-CH_2OH$ und $-CH_2O$-Benzyl substituierte Tetra- bis Heptamethyleniminogruppe ist, |

sowie Hydrate oder Solvate und physiologisch verwendbare Salze davon.

Ferner betrifft die Erfindung Verfahren zur Herstellung der obigen Verbindungen, pharmazeutische Präparate, die solche Verbindungen enthalten, sowie die Verwendung dieser Verbindungen bei der Herstellung von pharmazeutischen Präparaten.

Beispiele von physiologisch verwendbaren Salzen der Guanidine der Formel I sind Salze mit physiologisch verträglichen Mineralsäuren, wie Salzsäure, Schwefelsäure, schweflige Säure oder Phosphorsäure; oder mit organischen Säuren, wie Methansulfonsäure, p-Toluolsulfonsäure, Essigsäure, Trifluoressigsäure, Zitronensäure, Fumarsäure, Maleinsäure, Weinsäure, Bernsteinsäure oder Salicylsäure. Die Guanidine der Formel I mit freier Carboxygruppe können auch Salze mit physiologisch verträglichen Basen bilden. Beispiele solcher Salze sind Alkalimetall-, Erdalkalimetall-, Ammonium- und Alkylammoniumsalze, wie das Na-, K-, Ca- oder Tetramethylammoniumsalz. Die Guanidine der Formel I können auch in Form von Zwitterionen vorliegen.

Die Guanidine der Formel I können solvatisiert, insbesondere hydratisiert sein. Die Hydratisierung kann im Zuge des Herstellungsverfahrens erfolgen oder allmählich als Folge hygroskopischer Eigenschaften einer zunächst wasserfreien Verbindung der Formel I auftreten.

Die Guanidine der Formel I enthalten zumindest zwei asymmetrische C-Atome und können daher als Diastereomerengemisch oder als optisch reine Verbindung vorliegen.

Im Rahmen der Erfindung bezeichnet $C_{1-4}$-Alkyl geradkettige ($C_{1-4}$-n-Alkyl) oder verzweigte Gruppen, wie Methyl, Aethyl, Propyl, Isopropyl, Butyl, Isobutyl und t-Butyl.

Aryl bezeichnet Gruppen, wie Phenyl, Naphthyl und Anthryl, gegebenenfalls mit 1 bis 4 Substituenten, wie Halogen, $NO_2$, $NH_2$, CN, OH, $CH_3$, Isopropyl, t-Butyl, Phenyl, Phenylsulfonyl, $OCH_3$, Benzyloxy, Formamido, COOH, $COO-C_{1-4}$-n-Alkyl oder gegebenenfalls mono- oder di-$C_{1-4}$-alkyliertes Amino.

Weitere mögliche Substituenten einer Phenylgruppe sind $-NHSO_2$-Ar oder -NHCO-Ar (worin Ar Phenyl mit bis zu 3 Substituenten, wie Halogen, $NO_2$, $CF_3$, COOH und $C_{1-4}$-Alkyl, z.B. $CH_3$, ist), $-NHCOCH_2CH_2COO$(H, $C_{1-4}$-Alkyl oder Benzyl), Acetamido, $-NHCOCH_2OCH_2CH_2OCH_3$, -NHCOO(H oder $C_{1-4}$-Alkyl), -NHCOCOO (H oder $C_{1-4}$-Alkyl), $-NHCH_2COO$-Aethyl, $-NHCOCOC_6H_5$ und Tetra- bis Hepta-

methyleniminocarbonyl.

Beispiele von Cycloalkylgruppen sind Cyclohexyl und Decalyl. Heteroarylgruppen bestehen aus einem oder 2 Ringen und enthalten 3 bis 9 C-Atome und 1 oder 2 Heteroatome. Beispiele davon sind Imidazolyl, Thienyl, Benzothienyl, Chinolyl und Indolyl. Sie können substituiert sein, z.B. durch $CH_3$, Halogen, $-CH_2COOH$ oder 1-Methyl-5-trifluormethylpyrazolyl.

Heterocyclyl bezeichnet 1 oder 2 Ringe, 4 bis 7 C-Atome und 1 oder 2 Heteroatome enthaltende Gruppen, wie Tetrahydrochinolyl, Azepinyl, Piperidinyl, Pyrrolidinyl, Benzodiazepinyl, Benzoxazolyl und Benzopyrrolidinyl, gegebenenfalls mit 1 oder 2 Substituenten, wie Methyl, Halogen, Oxo, COOH oder $COOCH_3$.

Beispiele von Tetra- bis Heptamethyleniminogruppen sind Piperidino, Hexahydroazepin und Heptahydroazocin. Beispiele von Substituenten in einer solchen Gruppe R'' sind Acetoxy, Acetoxymethyl, Carbomethoxy, Carbäthoxy, Diäthylcarbamoyl, Butyryloxymethyl und Isobutyryloxymethyl.

Beispiele von durch ein O- oder S-Atom unterbrochene und gegebenenfalls substituierten Tetra- bis Heptamethyleniminogruppen Q sind Carboxyhexahydrooxazepin und Hexahydrothiazepin.

Als Substituent des Phenylrings in einer Isochinolylengruppe -N(X)-M kommen z.B. Cl, $NO_2$, $NH_2$ und OH in Frage.

Beispiele von Verbindungen der Formel I sind diejenigen der Formel

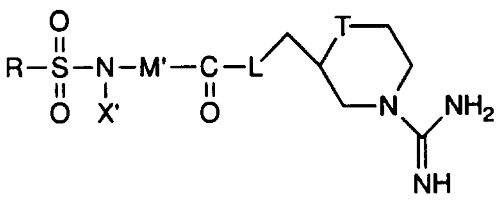

IA

worin

| | |
|---|---|
| -N(X')-M- | eine gegebenenfalls im Phenylring substituierte Isochinolylengruppe, oder |
| X' | H, $-CH_2COOH$, $-CH_2COO-C_{1-4}$-Alkyl, $-CH_2CO-$ (Tetra- bis Heptamethylenimino) oder gegebenenfalls N-mono- oder N-di-$C_{1-4}$-alkyliertes $-CH_2CONH_2$, und |
| M' | eine Gruppe $R'-(CH_2)_{1-2}CH=$, $R'-COCH_2CH=$ oder $-CH(CO-Q')CH_2-$, |
| Q' | Benzylamino, Morpholino oder Tetra- bis Heptamethylenimino ist, und |
| R, R', L und T | die gleichen Bedeutungen wie oben haben, |

sowie Hydrate oder Solvate und physiologisch verwendbare Salze davon.

Bevorzugt unter den Verbindungen IA sind diejenigen, worin X' H ist und R, M', L und T die gleichen Bedeutungen wie oben haben.

Bevorzugt unter den Verbindungen I sind solche der Formel

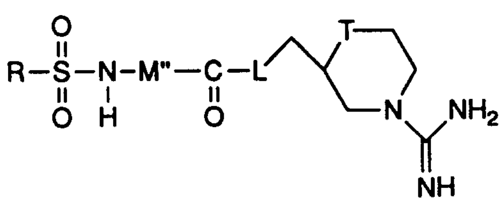

IB

worin

| | |
|---|---|
| M'' | eine Gruppe $R''-COCH_2CH=$, $R'-(CO)_{1-2}NHCH_2CH=$, Benzyl-$OCONHCH_2CH=$, $-CH_2[R'-(CO)_{1-2}NH]CH-$, $-CH_2$(Benzyl-OCONH)CH- oder $-CH(CO-Q'')CH_2-$, |
| Q'' | eine gegebenenfalls durch ein O- oder S-Atom unterbrochene und gegebenenfalls durch bis zu 2 Substituenten aus der Gruppe von $C_{1-4}$-Alkyl, COOH, $-COO-C_{1-4}$-Alkyl, $-CH_2OH$ und $-CH_2O$-Benzyl substituierte Tetra- bis Heptamethyleniminogruppe ist, und |
| R, R', R'', L und T | die gleichen Bedeutungen wie oben haben. |

Beispiele von Arylgruppen R sind Naphthyl, Hydroxynaphthyl, 4-Biphenyl, 2-Anthryl, Jodphenyl, Nitrophenyl, Benzyloxyphenyl, Dimethoxyphenyl, 4-Methoxy-2,3,6-trimethylphenyl, 2,4,6-Triisopropylphenyl, Carboxyphenyl, Methoxycarbonylphenyl, Benzyloxynaphthyl, Phenylsulfonylphenyl, Hexahydroazepinoylphenyl und t-Butylphenyl.

Beispiele von Heteroarylgruppen R sind 3-Methyl-8-chinolyl, 5-(1-Methyl-5-trifluormethylpyrazol-3-yl)-2-thienyl und Benzothienyl.

Ein Beispiel für die Heterocyclylgruppe R ist 3-Methyl-1,2,3,4-tetrahydro-8-chinolyl.

Ein Beispiel für eine Sulfonamidgruppe $-N(SO_2-R^o)-CH_2-$ als Bedeutung von -N(X)-M- ist N-Dimethylaminonaphthylsulfonyl-aminomethylen.

Bevorzugte Verbindungen der Formel I sind ferner diejenigen, worin X H oder $-CH_2COOH$ ist.

Beispiele von Resten M in der Bedeutung von $R'-(CH_2)_{1-2}CH=$ sind 3-Indolyläthyliden, 2,3-Dioxo-1-indolinyläthyliden, Phenäthyliden, 1,4-Dioxo-5H-2,5-benzodiazepin-5-yläthyliden, (Fluor, Chlor, Jod, Cyano, Nitro, Amino, Carboxy, $C_{1-4}$-Alkoxycarbonyl oder Hydroxy)-phenäthyliden, Cyclohexylpropyliden, Decalyläthyliden, Imidazolyläthyliden, Thienyläthyliden, (Methyl, Brom, Fluor oder Carboxymethyl)-3-indolyläthyliden, Naphthyläthyliden, (Aethoxycarbonylcarbonylamino, Methoxycarbonyläthylcarbonylamino, Benzyloxycarbonyläthylcarbonylamino, Aethoxycarbonylamino, Benzoylcarbonylamino, Carboxybenzoylamino, Methoxyäthoxyacetamido, Acetamido, Carboxycarbonylamino, Carboxypropionylamino, Tolylsulfonamido, Jodophenylsulfonamido, Carboxyphenylsulfonamido oder Aethoxycarbonyl-methylamino)phenäthyliden, Oxobenzoxazolinäthyliden oder 5-Bromo- oder 5-Methyl-2,3-dioxo-1-indolinyläthyliden.

Beispiele von Resten M in der Bedeutung von (R' oder R")$COCH_2CH=$ sind Hexahydroazepinoyläthyliden, (Methoxycarbonyl oder Carboxy)-pyrrolidinoyläthyliden, 3,4-Dihydro-2(1H)-isochinolinoyläthyliden, (Nitro, Amino, Jodo oder Formamido)benzoyläthyliden, Morpholinoäthyliden, Heptahydroazocinoyläthyliden, (Aethoxycarbonyl, Acetoxymethyl, Dimethylcarbamoyl, Isobutyryloxymethyl oder Butyryloxymethyl)-piperidinoyläthyliden, 3-Methoxycarbonyl-4-oxopiperidinoyläthyliden oder 4-Acetoxy-3-äthoxycarbonylpiperidinoyläthyliden.

Beispiele von Resten M in der Bedeutung $R'-(CO)_{1-2}NHCH_2CH=$ sind Benzoylcarboxamidoäthyliden, Thienoylcarboxamidoäthyliden, Benzoylamidoäthyliden oder Benzyloxycarboxamidoäthyliden.

Beispiele von Resten M in der Bedeutung von $-CH_2[R'-(CO)_{1-2}NH]CH-$sind 2-(Carboxybenzoylamido)-äthylen, 2-(Benzyloxybenzoylamido)äthylen, 2-(2-Piperidincarboxamido)äthylen, 2-(Hydroxybenzoylamido)-äthylen und 2-(Aminobenzoylamido)äthylen.

Beispiele von Resten M in der Bedeutung von $-CH(CO-Q)CH_2-$ sind 1-(Benzylaminocarbonyl)äthylen, 1-(Hexahydroazepinoyl)äthylen, 1-(Morpholinoyl)äthylen, 1-(Heptahydroazocinoyl)äthylen, 1-[2-(Benzyloxymethylmorpholinoyl)]äthylen, 1-[2-(Hydroxymethylmorpholinoyl)]äthylen, 1-(2-Aethoxycarbonyl-4-methylpiperidinoyl)äthylen und 1-(2-Carboxy-4-methylpiperidinoyl)äthylen und 1-(3-Carboxyhexahydro-1,4-oxazepinoyl)-äthylen ist.

In den obigen Formeln ist R vorzugsweise Aryl, insbesondere Naphthyl oder Nitro- oder Jodphenyl, L NH und das asymmetrische C-Atom im Piperidin- oder Morpholinring ist vorzugsweise in (S)-Konfiguration.

Bevorzugte Verbindungen der Formel I sind ferner diejenigen der Formel

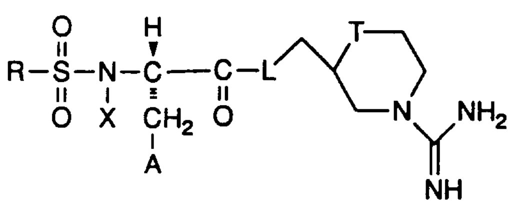

IC

worin A Aryl, Aroyl oder Heterocyclyl ist, insbesondere Phenyl, Nitrophenyl, Indolyl, 2,3-Dioxo-1-indolinyl oder Aminobenzoyl bedeuten.

Beispiele von bevorzugten Verbindungen sind folgende:

(R)-N-[(RS)-1-Amidino-3-piperidinylmethyl]-α-(2-naphthylsulfonamido)-2,3-dioxo-1-indolinpropionamid,

(R)-N-[(RS)-1-Amidino-3-piperidinylmethyl]-α-(2-naphthylsulfonamido)-o-nitrohydrocinnamamid,

(R)-N-[(RS)-1-Amidino-3-piperidinylmethyl]-α-(o-nitrobenzolsulfonamido)indol-3-propionamid,

(R)-N-[(S)-1-Amidino-3-piperidinylmethyl]-α-(p-jodbenzolsulfonamido)-indol-3-propionamid,

(R)-N-[(S)-1-Amidino-3-piperidinylmethyl]-α-(p-jodbenzolsulfonamido)-p-nitrohydrocinnamamid,

(R)-N-[(RS)-1-Amidino-3-piperidinylmethyl]-3-(o-aminobenzoyl)-(2-naphthylsulfonamido)propionamid,

N-[(R)-α-[[(S)-1-Amidino-3-piperidinyl]methylcarbamoyl]phenäthyl]-N-(2-naphthylsulfonyl)glycin,

(R)-N-[(S)-1-Amidino-3-piperidinylmethyl]-1,2,3,4-tetrahydro-2-(2-naphthylsulfonyl)-3-isochinolincarboxamid,

(S)-N-[(RS)-1-Amidino-3-piperidinylmethyl]hexahydro-β-(2-naphthylsulfonamido)-γ-oxo-1H-1-azepinbutyramid,

(R)-N-[(S)-1-Amidino-3-piperidinylmethyl]-α-(2-naphthylsulfonamido)-2,3-dioxo-1-indolinpropionamid,

4'-[(R)-2-[[[(S)-1-Amidino-3-piperidinyl]methyl]carbamoyl]-2-(2-naphthylsulfonamido)äthyl]oxanilsäure,

(S)-N-[[(RS)-1-Amidino-3-piperidinyl]methyl]hexahydro-$\beta$-2-naphthylsulfonamido-$\gamma$-oxo-1(2H)-azocinbutyramid,

(2RS,4R)-1-[N[4]-[[(S)-1-Amidino-3-piperidinyl]methyl]-N[2]-(2-naphthylsulfonyl)-L-asparaginyl]-4-methyl-2-piperidincarbonsäure`

4'-[(R)-2-[[[(S)-1-Amidino-3-piperidinyl]methyl]carbamoyl]-2-(2-naphthylsulfonamido)äthyl]-succinanilidsäure.

Die obigen Verbindungen kann man dadurch herstellen, dass man

a) eine Säure der Formel

$R\text{-}SO_2N(X)\text{-}M\text{-}COOH$ II

unter intermediärem Schutz eines in der Gruppe X, R oder M enthaltenen Carboxygruppe mit einem Amin oder Alkohol der Formel

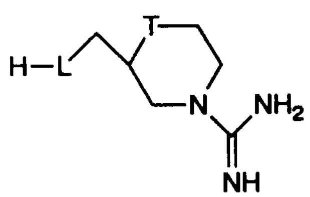

III

oder einem Salz davon umsetzt oder

b) eine Verbindung der Formel

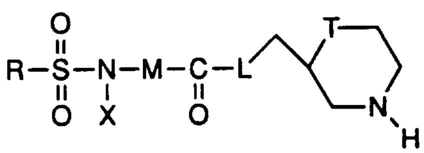

IV

mit einem Amidinierungsreagenz umsetzt oder

c) ein Amin der Formel

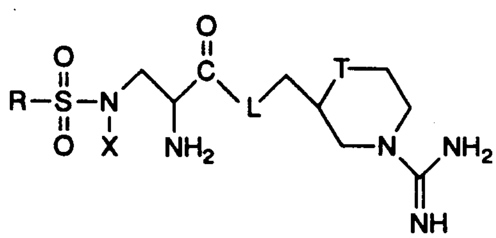

V

mit einer Säure der Formel R'-COOH oder einem funktionellen Derivat davon umsetzt, und

d) gewünschtenfalls eine in der Gruppe M einer Verbindung der Formel I enthaltene reaktionsfähige Gruppe funktionell abwandelt, und

e) gewünschtenfalls eine Verbindung der Formel I in ein physiologisch verträgliches Salz überführt oder ein Salz einer Verbindung der Formel I in die freie Säure oder Base überführt.

Zweckmässig wird die Säure II in einem Lösungsmittel, wie Dimethylformamid (DMF), in Gegenwart einer Base, wie 4-Aethylmorpholin oder Aethyldiisopropylamin, mit einem Salz einer Verbindung der Formel III, z.B. einem Trifluoracetat, Bisulfit, Nitrat, Hydrochlorid oder Hydrojodid, und mit Benzotriazol-1-yloxy-tris-(dimethylamino)phosphonium-hexafluorophosphat bei Raumtemperatur umgesetzt. Eine in der Gruppe X, R oder M enthaltene z.B. als t-Butylester geschützte Carboxygruppe wird dabei freigesetzt.

In der Verfahrensvariante b) kann man die Verbindung IV in einem Lösungsmittel, wie DMF, in Gegenwart einer Base, wie Triäthylamin, mit Formamidinsulfonsäure oder 3,5-Dimethyl-1-pyrazolyl-formamidiniumnitrat, zweckmässig bei einer Temperatur bis zu 50 °C umsetzen.

Nach der Verfahrensvariante c) kann man das Amin V mit einer Säure R'-COOH oder einem funktionellen Derivat, z.B. einem Säureanhydrid, in einem Lösungsmittel, wie DMF, in Gegenwart von einer Base, wie 4-Aethylmorpholin, bei erhöhter Temperatur, z.B. bis zu 50 oder 80 °C, umsetzen.

Als funktionelle Abwandlungen in der Variante d) kann man folgende nennen:

1. die Reduktion einer in der Gruppe M enthaltenen Nitroarylgruppe zur Aminoarylgruppe, in einem Lösungsmittel, wie Aethanol, mit Pd/C;
2. die Verseifung einer Estergruppe, wie Aethoxycarbonyl, z.B. in Aethanol oder Methanol, mittels einer Base, wie wässriges Natriumhydroxid;
3. die Verseifung einer Estergruppe, wie Benzyloxycarbonyl, z.B. in Aethanol mit Pd/C;
4. die Spaltung eines Benzyläthers, z.B. in Aethanol mit Pd/C in Gegenwart einer Säure, wie Salzsäure oder Essigsäure.

Die N-sulfonierten Aminosäuren II lassen sich durch Reaktion eines entsprechenden reaktionsfähigen Sulfonsäurederivats, wie dem Sulfochlorid $R-SO_2Cl$, mit der entsprechenden freien Aminosäure HN(X)-M-COOH, in Gegenwart einer Base, wie einem Alkalimetallhydroxid, in einem Lösungsmittel, wie einem Aether, z.B. Diäthyläther oder Dioxan, und Wasser, herstellen.

In einer Variante kann man eine Säure II durch Oxidation des entsprechenden Alkohols

$R-SO_2-N(X)-M-CH_2OH$ VI

herstellen, z.B. in einem Lösungsmittel, wie Aceton, mit einem Oxidationsmittel, wie dem Jones-Reagens. Dabei wird ein in der Gruppe M des Alkohols VI enthaltenes Heterocyclylradikal R', wie 2-Oxo-1-indolinyl, in 2,3-Dioxo-1-indolinyl oxidiert.

Ein Alkohol V lässt sich herstellen, indem man den entsprechenden $\alpha$-Aminoalkohol $HN(X)-M-CH_2OH$ in Gegenwart von wässrigem Natriumhydroxid und Natriumbicarbonat mit dem Sulfochlorid $R-SO_2Cl$ in Dioxan umsetzt.

Ein Aminoalkohol $HN(X)-M-CH_2OH$, worin M z.B. eine Gruppe $=CH(CH_2-R')$ ist, kann man herstellen durch Spaltung eines entsprechenden am N-Atom geschützten, in 4-Stellung durch $-CH_2-R'$ substituierten Oxazolidins, z.B. des (R)-2,2-Dimethyl-4-($CH_2R'$)-3-oxazolidincarbonsäure-t-butylesters, in Methanol mit Salzsäure.

Das obige Oxazolidin lässt sich in an sich bekannter Weise herstellen, z.B. falls R' ein über ein N-Atom gebundenes Heterocyclylradikal, wie 2-Oxo-1-indolinyl ist, durch Umsetzung des entsprechenden cyclischen Amins H-R' mit (S)-2,2-Dimethyl-4-(p-tolylsulfonyloxymethyl)-3-oxazolidincarbonsäure-t-butylester in Gegenwart einer Suspension von Natriumhydrid in DMF.

Zur Herstellung einer Aminosäure II, worin R ein Hydroxyarylradikal ist, kann man zunächst das entsprechende Sulfochlorid $R-SO_2Cl$, worin die OH-Gruppe in geschützter Form, z.B. als Acetoxygruppe vorliegt, in Aceton mit der Aminosäure HN(X)-M-COOH in Gegenwart von wässrigem Natriumhydroxid umsetzen und dann die Schutzgruppe, z.B. Acetyl, mit einer Lösung von Natriumhydroxid in Wasser und Methanol abspalten.

Zur Herstellung eines Guanidins der Formel I, worin X $-CH_2COOH$ ist, kann man zunächst das Sulfochlorid $R-SO_2Cl$ mit einem Salz, z.B. das p-Toluolsulfonat eines Esters $H_2N$-M-COO-Benzyl in einem Lösungsmittel, wie Methylenchlorid in Gegenwart einer Base, wie Triäthylamin umsetzen. Das erhaltene Sulfonamid $R-SO_2NH$-M-COO-Benzyl wird dann in einem Lösungsmittel, wie THF, bei etwa -80 °C in Gegenwart von Butyllithium mit Bromessigsäure-t-butylester umgesetzt. Danach wird die Benzylgruppe selektiv, z.B. durch katalytische Hydrierung mit Pd/C in Aethanol, aus dem erhaltenen Diester

$R-SO_2N(CH_2COO$-t-Butyl)-M-COO-Benzyl

abgespalten. Wie weiter oben beschrieben, wird bei der Umsetzung der entstandenen Säure mit einer Verbindung der Formel III die als t-Butylester geschützte Carboxygruppe in der Seitenkette freigesetzt.

Die Ausgangssäuren $R-SO_2NHCH(CO-Q)-CH_2COOH$ lassen sich herstellen durch

a) Umsetzung eines Asparaginsäurederivats, z.B.

t-Butoxy-$CONHCH(COOH)CH_2COO$-Benzyl

mit dem der Gruppe Q entsprechenden Amin H-Q in einem Lösungsmittel, wie DMF, in Gegenwart von Benzotriazol-1-yloxy-tris(dimethylamino)phosphonium-hexafluorophosphat,

b) Reaktion des erhaltenen Amids

t-Butoxy-CONHCH(CO-Q)-$CH_2$COO-Benzyl

in einem Lösungsmittel, wie Essigester, mit einer Säure, wie Salzsäure, und dann in Dioxan mit Natronlauge, Natriumbicarbonat und einem Sulfochlorid R-$SO_2$Cl und
c) Abspaltung der Benzylgruppe im entstandenen Sulfonamid

R-$SO_2$NHCH(CO-Q)-$CH_2$COO-Benzyl

in einem Lösungsmittel, wie Methanol mittels Natronlauge.

Die Verbindungen III, IV und V sind neu und als solche Gegenstand der Erfindung. Die Piperidinderivate der Formel III lassen sich wie in den nachstehenden Beispielen 1, 2 und 3 beschrieben aus 3-Picolylamin, falls L = NH ist, oder aus 3-Hydroxymethylpiperidin, falls L = O ist, herstellen. Aminomethylmorpholinderivate der Formel III erhält man durch Umsetzung des 2-Aminomethyl-4-benzylmorpholins(J. Med. Chem. 33, 1990, 1406-1413) mit Di-t-butyldicarbonat in Dioxan, Hydrierung des erhaltenen mit Boc geschützten Amins in Aethanol in Gegenwart von Pd/C zum 2-(t-Butoxycarbonylaminomethyl)morpholin und Amidinierung des letzteren. Hydroxymethylmorpholinderivate der Formel III lassen sich durch Amidinierung des 2-Hydroxymethylmorpholins herstellen.

Die Verbindungen der Formel IV stellt man her durch Umsetzung der entsprechenden Säure II mit der Verbindung der Formel

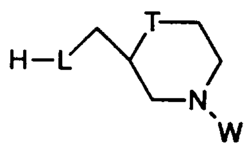

III'

worin W eine Schutzgruppe, wie Boc oder Z, ist,
z.B. wie oben beschrieben für die Umsetzung von II mit III, gefolgt durch die Abspaltung der Schutzgruppe, z.B. mittels Trifluoressigsäure in Methylenchlorid, p-Toluolsulfonsäure in Acetonitril oder einer Lösung von Chlorwasserstoff in Aethylacetat.

Alkohole der Formel III' lassen sich wie in Beispiel 3a) beschrieben durch Einführung der Schutzgruppe am N-Atom des 3-Hydromethylpiperidins bzw. wie im Beispiel 19a) bis c) beschrieben ausgehend von Benzyloxymethyloxiran über das 2-Benzyloxymethylmorpholin und das z.B. mit Boc geschützte 2-Benzyloxymethylmorpholin herstellen. Wie in den Beispielen 6a) bis d) und 19d) und e) beschrieben kann man den erhaltenen Alkohol in das entsprechende Amin III' überführen.

Die Amine V kann man durch Abspaltung der Benzyloxycarbonylgruppe in der entsprechenden Verbindung I, worin z.B. M die Gruppe

-$CH_2$(Benzyl-OCONH)CH-

ist, z.B. in Aethanol in Gegenwart von Salzsäure mit Pd/C, herstellen.

Die Guanidine der Formel I, ihre Solvate und ihre Salze hemmen sowohl die durch Thrombin induzierte Plättchenaggregation als auch die durch Thrombin induzierte Gerinnung von Fibrinogen im Blutplasma. Die besagten Verbindungen beeinflussen sowohl die Plättchen induzierte als auch die plasmatische Blutgerinnung. Sie verhindern damit insbesondere die Entstehung von Gerinnungsthromben aber auch von plättchenreichen Thromben und können bei der Bekämpfung bzw. Verhütung von Krankheiten, wie Thrombose, Apoplexie, Herzinfarkt, Entzündung und Arteriosklerose verwendet werden. Ferner haben diese Verbindungen einen Effekt auf Tumorzellen und verhindern die Bildung von Metastasen. Somit können sie auch als Antitumor-Mittel eingesetzt werden.

Eine unterschiedliche Hemmung von Thrombin und anderen Serin-Proteasen durch die obigen Verbindungen ist erstrebenswert, um Verbindungen mit einer möglichst hohen Spezifität zu erhalten und damit mögliche Nebenwirkungen zu vermeiden. Nebst anderen getesteten Serin-Proteasen wurde das Verhältnis der Hemmung von Trypsin zur Hemmung von Thrombin als generelles Mass der Spezifität einer Verbindung genommen (q in der nachstehenden Tabelle), weil Trypsin als unspezifischste Serin-Protease durch verschiedenste Hemmer leicht gehemmt werden kann. Um die Hemmung von Thrombin und Trypsin, trotz Benutzung unterschiedlicher Substrate, direkt vergleichen zu können, wurde als Mass der Hemmung die von Substrat- und Enzymkonzentration unabhängige Hemmkonstante $K_i$ ermittelt.

Zum Nachweis der Hemmung der katalytischen Aktivität der obigen Proteasen können spezifische chromogene Peptidsubstrate benutzt werden. Die Hemmung der amidolytischen Aktivität von Thrombin und Trypsin durch die obigen Guanidine wurde wie nachstehend beschrieben nachgewiesen.

Die Messungen wurden auf Mikrotiterplatten bei Raumtemperatur durchgeführt. Dafür wurden pro Vertiefung der Platte 150 µl Puffer (50 mM Tris, 100 mM NaCl, 0,1% Polyäthylenglykol; pH 7,8) mit 50 µl der in DMSO gelösten und im Puffer verdünnten Hemmsubstanz vermischt und 25 µl humanes Thrombin (0,5 nM Endkonz.) zugegeben. Nach 10 Minuten Inkubation wurde die Reaktion durch Zugabe von chromogenem Substrat S-2238 (H-D-Phe-Pip-Arg-Paranitroanilin von Kabivitrum; 10 oder 50 µM Endkonz.) gestartet und die Hydrolyse des Substrates spektrophotometrisch auf einem kinetischen Mikrotiter-Plattenleser während 5 Minuten verfolgt. Nach graphischer Darstellung der Hemmkurven wurden die Ki-Werte nach der in Biochem. J. 55, 1955, 170-171 beschriebenen Methode bestimmt. Die Hemmung von Trypsin erfolgte analog, jedoch unter Verwendung des Substrates S-2251 (H-D-Val-Leu-Lys-Paranitroanilin) in 200 und 750 µM Endkonzentration.

Die Resultate sind aus folgender Tabelle ersichtlich:

| **Produkt von Beispiel** | **8** | **9** | **13a** | **14i** | **17b** |
|---|---|---|---|---|---|
| Ki (nM) Thrombin | 8,55 | 135 | 20,8 | 25 | 20,7 |
| Ki (nM) Trypsin | 20075 | 168700 | 29200 | 38000 | 6300 |
| q | 2350 | 1250 | 1400 | 1520 | 304 |
| **Produkt von Beispiel** | **17c** | **17f** | **17j** | **18f** | |
| Ki (nM) Thrombin | 58 | 41,3 | 26,1 | 16 | |
| Ki (nM) Trypsin | 10000 | 18300 | 14800 | 18850 | |
| q | 172 | 455 | 565 | 1180 | |

Die Guanidine der Formel I haben eine geringe Toxizität. So haben die Produkte von Beispiel 8, 13a, 14i und 18f bei intravenöser Verabreichung eine LD50 von 30-50 mg/kg an der Maus.

Wie eingangs erwähnt, sind Arzneimittel enthaltend ein Guanidin der Formel I, ein Solvat oder Salz davon, ebenfalls Gegenstand der vorliegenden Erfindung, weiterhin auch ein Verfahren zur Herstellung derartiger Arzneimittel, welches dadurch gekennzeichnet ist, dass man eine oder mehrere der besagten Verbindungen und, falls erwünscht, einen oder mehrere andere therapeutisch wertvolle Stoffe in eine galenische Darreichungsform bringt. Die Arzneimittel können enteral, z.B. oral in Form von Tabletten, Lacktabletten, Dragées, Hart- und Weichgelatinekapseln, Lösungen, Emulsionen oder Suspensionen, oder rektal, z.B. in Form von Suppositorien, oder als Spray verabreicht werden. Die Verabreichung kann aber auch parenteral, z.B. in Form von Injektionslösungen, erfolgen.

Zur Herstellung von Tabletten, Lacktabletten, Dragées und Hartgelatinekapseln kann der Wirkstoff mit pharmazeutisch inerten, anorganischen oder organischen Excipientien vermischt werden. Als solche Excipientien kann man für Tabletten, Lacktabletten, Dragées und Hartgelatinekapseln z.B. Lactose, Maisstärke oder Derivate davon, Talk, Stearinsäure oder deren Salze, verwenden. Für Weichgelatinekapseln eignen sich als Excipientien z.B. vegetabile Oele, Wachse, Fette, halbfeste und flüssige Polyole; je nach Beschaffenheit des Wirkstoffs sind jedoch bei Weichgelatinekapseln überhaupt keine Excipientien erforderlich. Zur Herstellung der Lösungen und Sirupe eignen sich als Excipientien z.B. Wasser, Polyole, Saccharose, Invertzucker und Glucose, für Injektionslösungen eignen sich z.B. Wasser, Alkohole, Polyole, Glycerin und vegetabile Oele, und für Suppositorien eignen sich natürliche oder gehärtete Oele, Wachse, Fette, halbflüssige oder flüssige Polyole. Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten.

Für die Bekämpfung bzw. Verhütung der weiter oben genannten Krankheiten kann die Dosierung des Wirkstoffes innerhalb weiter Grenzen variieren und ist natürlich in jedem Einzelfall den individuellen Gegebenheiten anzupassen. Im allgemeinen dürfte bei oraler oder parenteraler Verabreichung eine Dosis von etwa 0,1 bis 20 mg/kg, vorzugsweise von etwa 0,5 bis 4 mg/kg, pro Tag für den Ewachsenen angemessen sein, wobei aber die soeben angegebene obere Grenze auch über- oder unterschritten werden kann, wenn sich dies als angezeigt erweisen sollte.

Beispiel 1

a) Zu einer Lösung von 30 g 3-Picolylamin in 300 ml Dioxan wird eine Lösung von 66,6 g Di-t-butyldicarbonat in 200 ml Dioxan zugegeben, so dass die Temperatur 25 °C nicht übersteigt. Das Reaktionsgemisch wird 3 Stunden bei Raumtemperatur gerührt und eingedampft. Nach Filtration über Kieselgel mit Essigester, erhält man 55,5 g 3-t-Butyloxycarbonyl-picolylamin.

b) 45,5 g 3-t-Butyloxycarbonyl-picolylamin werden in 220 ml Methanol gelöst und katalytisch mit 4,6 g Ruthenium auf Aluminium (5%) bei 60 °C unter 100 bar Wasserstoff hydriert. Nach Filtrierung des Katalysators und Eindampfen des Lösungsmittels erhält man 46 g rac-3-t-Butyloxycarbonyl-aminomethylpiperidin.

c) 38,8 g des Produkts von b) werden in 900 ml DMF gelöst und mit 74,5 ml Triäthylamin versetzt. Nach Zugabe von 26,5 g Formamidinsulfonsäure wird das Reaktionsgemisch 15 Stunden gerührt und anschliessend filtriert. Die Mutterlauge wird eingedampft, der Rückstand in Wasser aufgenommen und mit Essigester ausgeschüttelt. Die wässerige Phase wird eingedampft und das Produkt mit Aethanol, Toluol und Dichloräthan azeotropisch verdampft. Der Rückstand wird in Aether aufgeschlämmt und filtriert. Man erhält 44,0 g rac-3-t-Butoxycarbonyl-aminomethyl-1-amidinopiperidin-hemisulfit, MS: 257 ($M^+ + 1$), 201, 157, 126, 96.

d) 27,8 g des Produkts von c) werden in 70 ml Methylenchlorid gelöst, mit 70 ml Trifluoressigsäure bei 0 °C versetzt und 1 Stunde gerührt. Das Reaktionsgemisch wird eingedampft und mit Toluol und Aethanol azeotropisch verdampft. Man erhält 27,6 g rac-3-Aminomethyl-1-amidinopiperidin-sulfit, MS: 156 ($M^+$), 126 ($M^+$-$CH_2NH_2$), 69,45.

Beispiel 2

Eine Lösung von 5,3 g rac-3-Hydroxymethylpiperidin in 100 ml DMF wird mit 19 ml Triäthylamin versetzt. Nach Zugabe von 6,8 g Formamidinsulfonsäure wird das Reaktionsgemisch 15 Stunden gerührt. Die Suspension wird filtriert, der Niederschlag mit Aether gewaschen und getrocknet. Man erhält 10,3 g rac-3-Hydroxymethyl-1-amidinopiperidin-hemisulfit, MS: 158 (M + 1), 143, 116, 102; Smp. 100 °C.

Beispiel 3

a) Zu einer Lösung von 92,9 g rac-3-Hydroxymethylpiperidin in 1500 ml Dioxan wird eine Lösung von 211,2 g Di-t-butyldicarbonat in 500 ml Dioxan zugegeben, so dass die Temperatur 25 °C nicht übersteigt. Das Reaktionsgemisch wird 15 Stunden bei Raumtemperatur gerührt und eingedampft. Der Rückstand wird in 800 ml Hexan aufgeschlämmt und filtriert. Man erhält 120,7 g rac-N-t-Butyloxycarbonyl-3-hydroxymethylpiperidin, Smp. 78 °C.

b) Eine Lösung von 100 g des Produkts von a) in 4000 ml Methylenchlorid wird mit 56,2 ml Pyridin versetzt und auf 0 °C abgekühlt. Dazu tropft man 58,3 ml Buttersäurechlorid, so dass die Temperatur 10 °C nicht übersteigt. Das Reaktionsgemisch wird anschliessend 15 Stunden bei Raumtemperatur gerührt. Die Suspension wird abfiltriert, das Filtrat eingedampft und der Rückstand in Essigester aufgenommen. Die organische Phase wird mit wässriger 10% $CuSO_4$-Lösung gewaschen, getrocknet und eingedampft. Der Rückstand wird durch Kieselgel filtriert und mit Hexan/Essigester (8/2) eluiert. Man erhält 119,7 g rac-3-(Butyroxymethyl)-1-piperidincarbonsäure-t-butylester.

c) 116,6 g des Produkts von b) werden in 2 l 0,1M Natriumchloridlösung und 80 ml 0,1M Natriumphosphat-Puffer pH 7,0 emulgiert. Der pH wird mit 1,0N Natronlauge auf 7,0 gestellt und die Reaktion durch Zugabe von 1,00 g aus Pseudomonas fluorescens gewonnener Lipoproteinlipase (Lipase P-30, Amano) in 10 ml 0,1M Natriumchloridlösung gestartet. Der pH wird unter Rühren durch Zugabe von 2,0N Natronlauge bei 7,0 gehalten. Nach 14 Stunden wird die Reaktion durch Zugabe von 500 ml Methylenchlorid beendet, das Reaktionsgemisch mit Methylenchlorid extrahiert und die organische Phase getrocknet und eingedampft. Chromatographie des Rückstands über Kieselgel mit Hexan/Essigester ergibt 36,6 g [S]-3-Hydroxymethy-1-piperidincarbonsäure-t-butylester, Smp. 89-90 °C,

$$[\alpha]^{25}_{365} = +53{,}5°$$

(c = 1,0, EtOH).

d) Die 65,7 g Ester-Fraktion von c) werden in 1,15 l 0,1M Natriumchloridlösung und 45 ml 0,1M Natriumphosphat-Puffer (pH 7,0) emulgiert und mit 0,50 g Lipase P-30 in 5 ml 0,1M Natriumchloridlösung versetzt. Der pH wird unter Rühren durch Zugabe von 2,0N Natronlauge bei 7,0 gehalten. Nach 40 Stunden wird die Reaktion durch Zugabe von 400 ml Methylenchlorid beendet, das Reaktionsgemisch mit Methylenchlorid extrahiert und die organische Phase getrocknet und eingedampft. Chromatographie des Rückstands über Kieselgel mit Hexan/Essigester ergibt 49,5g [R]-3-(Butyryloxymethyl)-1-piperidincarbonsäure-t-butylester. Dieser wird in 250 ml Aethanol gelöst, mit 88 ml 2N Natronlauge versetzt, über Nacht gerührt und dann eingedampft. Der Rückstand wird in 200 ml Methylenchlorid aufgenommen und mit Wasser gewaschen, die wässrige Phase mit Methylenchlorid extrahiert und die organische Phase getrocknet und eingeengt. Chromatographie des Rückstands über Kieselgel mit Hexan/Essigester ergibt 33,7 g [R]-3-Hydroxymethyl-1-piperidincarbonsäure-t-butylester,

$$[\alpha]^{25}_{365} = -60,7°$$

(c = 1,0, EtOH).

e) Eine Lösung von 5,0 g des Produkts von d) in 20 ml Methylenchlorid wird auf 0 ° C abgekühlt und mit 15 ml Trifluoressigsäure versetzt. Nach 2 Stunden wird das Reaktionsgemisch eingedampft und der Rückstand in Wasser aufgenommen und mit Natriumbicarbonat bis auf pH 7,5 versetzt. Diese wässrige Phase wird eingedampft und die Kristallmasse in 100 ml Methylenchlorid/Aethanol (9/1) aufgeschlämmt. Die Salze werden abfiltriert und das Filtrat eingedampft. Der Rückstand wird in 30 ml DMF gelöst und mit 9,7 ml Triäthylamin versetzt. Nach Zugabe von 3,5 g Formamidinsulfonsäure wird das Reaktionsgemisch 15 Stunden gerührt. Der Niederschlag wird abfiltriert und das Lösungsmittel eingedampft. Der Rückstand wird durch reversen Phasen Chromatographie über silyliertem Kieselgel RP-18 mit Wasser gereinigt. Man erhält 2,6 g (R)-3-Hydroxymethyl-1-amidinopiperidinhemisulfat, FAB-MS: 158 (M + 1).

Beispiel 4

Analog Beispiel 3e) wird aus [S]-3-Hydroxymethyl–1-piperidincarbonsäure-t-butylester (S)-3-Hydroxymethyl-1-amidinopiperidin-hemisulfat hergestellt, FAB-MS: 158 (M + 1).

Beispiel 5

Eine Lösung von 1,5 g N-(2-Naphthylsulfonyl)-D-tryptophan in 30 ml DMF wird mit 1,5 ml Aethylmorpholin versetzt. Nach Zugabe von 0,7 g rac-3-Aminomethyl-1-amidinopiperidin-sulfit (Beispiel 1) und 1,7 g Benzotriazol-1-yloxy-tris(dimethylamino)phosphonium-hexafluorphosphat wird das Reaktionsgemisch 15 Stunden gerührt. Das Lösungsmittel wird eingedampft, der Rückstand in 50 ml Wasser aufgenommen und mit 100 ml Essigester extrahiert. Die Essigester-Phase wird mit Wasser gewaschen, getrocknet und eingedampft. Das Rohmaterial wird auf einer RP-18 Säule mit Wasser/Acetonitril (0-30%) gereinigt. Man erhält 0,8 g (R)-N-[(RS)-1-Amidino-3-piperidinylmethyl]-α-(2-naphthylsulfonamido)indol-3-propionamid-bisulfit, FAB-MS: 533 $(M + H)^+$.

Beispiel 6

a) Eine Lösung von 5,0 g (S)-3-Hydroxymethyl-1-piperidincarbonsäure-t-butylester (Beispiel 3c) in 100 ml Pyridin wird mit 5,4 g p-Chlorsulfonylchlorid versetzt. Das Reaktionsgemisch wird 15 Stunden gerührt, eingedampft, in 200 ml Essigester aufgenommen und mit Wasser und wässriger 10% $CuSO_4$-Lösung gewaschen. Die organische Phase wird getrocknet und eingedampft. Der Rückstand wird über Kieselgel filtriert und mit Hexan/Essigester (8/2) eluiert. Man erhält 6,5 g (S)-3-(p-Chlorphenylsulfonyloxymethyl)-1-piperidincarbonsäure-t-butylester.

b) Eine Lösung des Produkts von a) in 50 ml DMF wird mit 3,25 g Natriumazid versetzt. Das Reaktionsgemisch wird 15 Stunden bei 50 ° C gerührt und eingedampft. Der Rückstand wird in Wasser und Aether aufgenommen und mit Wasser gewaschen. Die Aether-Phase wird getrocknet und eingedampft. Man erhält 4,0 g (S)-3-Azidomethy-1-piperidincarbonsäure-t-butylester.

c) Eine Lösung des Produkts von b) in 100 ml Aethanol wird in Gegenwart von 0,6 g Platinoxid unter 1 bar Wasserstoff hydriert. Dann wird das Reaktionsgemisch über Kieselgel filtriert und mit Methanol eluiert. Man erhält 3,4 g (R)-3-Aminomethyl-1-piperidincarbonsäure-t-butylester, $[\alpha]^{25}_{D}$ = + 23,0 ° (c = 0,4, EtOH).

d) Mit einem identischen Verfahren erhält man aus (R)-3-Hydroxymethyl-1-piperidincarbonsäure-t-butylester den (S)-3-Aminomethyl-1-piperidincarbonsäure-t-butylester, $[\alpha]^{25}_{D}$ = -17,7 ° (c = 0,6 EtOH)

e) Eine Lösung von 1,5 g N-(2-Naphthylsulfonyl)-D-tryptophan in 50 ml DMF wird mit 0,8 ml Aethyldiisopropylamin versetzt. Nach Zugabe von 1,0 g des Produkts von d) und 1,7g Benzotriazol-1-yloxy-tris-(dimethylamino)phosphonium-hexafluorphosphat wird das Reaktionsgemisch 15 Stunden gerührt. Das Lösungsmittel wird eingedampft, der Rückstand in Wasser aufgenommen und mit Essigester extrahiert. Die Essigester-Phase wird mit Wasser gewaschen, getrocknet und eingedampft. Chromatographie über Kieselgel mit Essigester ergibt 2,3 g (R)[(S)-1-(t-Butoxycarbonyl)-3-piperidinylmethyl]-α-naphthylsulfonamidoindol-3-propionamid.

f) Eine Lösung des Produkts von e) in 10 ml Methylenchlorid wird bei 0°C mit 3 ml Trifluoressigsäure versetzt. Das Reaktionsgemisch wird 1 Stunde bei 0°C gerührt, eingedampft und mit Aethylenchlorid azeotropiert. Der Rückstand wird in Wasser und Essigester aufgenommen, mit 10-%igem Natriumcarbonat auf pH 9 gesetzt und ausgeschüttelt. Die organische Phase wird getrocknet und eingedampft. Man erhält 2,5 g (R)-α-Naphthylsulfonamido-N-[(S)-3-piperidinylmethyl]-indol-3-propionamid.

g) Eine Lösung des Produkts von f) in 50 ml DMF wird mit 1,7 ml Triäthylamin versetzt. Nach Zugabe von 0,7 g Formamidinsulfonsäure wird das Reaktionsgemisch 15 Stunden gerührt. Das Lösungsmittel wird eingedampft, der Rückstand in Essigester und Methanol aufgenommen. Die organische Phase wird mit Wasser gewaschen, getrocknet und eingedampft. Reinigung des Produkts auf einer RP-18 Säule mit Wasser/ Acetonitril ergibt 1,85 g (R)-N-[(S)-1-Amidino-3-piperidinylmethyl]-α-(2-naphthylsulfonamido)-indol-3-propionamid-bisulfit, FAB-MS: 533 $(M+H)^+$.

Beispiel 7

a) Eine Lösung von 1,0 g N-2-Naphthylsulfonyl-(D)-tryptophan in 20 ml DMF wird mit 0,5 ml Aethyldiisopropylamin versetzt. Nach Zugabe von 0,6 g (S)-3-Hydroxymethyl-1-piperidincarbonsäure-t-butylester (Beispiel 3c) und 1,1 g Benzotriazol-1-yloxy-tris(dimethylamino)phosphonium-hexafluorphosphat wird das Reaktionsgemisch 15 Stunden gerührt. Das Lösungsmittel wird eingedampft, der Rückstand in Wasser aufgenommen und mit Essigester extrahiert. Die Essigester-Phase wird mit Wasser gewaschen, getrocknet und eingedampft. Chromatographie über Kieselgel mit Essigester ergibt 1,0 g N-(2-Naphthylsulfonyl)-D-tryptophan-(S)-1-t-butoxycarbonyl-3-piperidinylmethylester.

b) Eine Lösung des Produkts von a) in 10 ml Acetonitril wird mit 0,9 g p-Toluolsulfonsäure versetzt. Das Reaktionsgemisch wird während 15 Stunden gerührt. Nach Eindampfen wird der Rückstand in Essigester aufgenommen und mit gesättigter Natriumbicarbonatlösung gewaschen. Die organische Phase wird getrocknet und eingedampft. Reinigung über einer RP Säule mit Wasser/Acetonitril ergibt 0,47 g N-(2-Naphthylsulfonyl)-D-tryptophan-(S)-3-piperidinylmethylester.

c) Eine Lösung des Produkts von b) in 20 ml DMF wird mit 0,4 ml Triäthylamin versetzt. Nach Zugabe von 0,2 g Formamidinsulfonsäure wird das Reaktionsgemisch 15 Stunden gerührt. Das DMF wird eingedampft, der Rückstand in Essigester aufgenommen und mit Wasser gewaschen. Die organische Phase wird getrocknet und eingedampft. Reinigung auf einer RP-Säule mit Wasser/Acetonitril ergibt 0,27 g N-(2-Naphthylsulfonyl)-D-tryptophan-(S)-1-amidino-3-piperidinylmethylester-bisulfit, FAB-MS: 534 (M+1).

Beispiel 8

a) Eine Lösung von 10,7 g (R)-4-Hydroxymethyl-2,2-dimethyl-3-oxazolidincarbonsäure-t-butylester (J. Org. Chem. 52, 1987, 2361-64) in 107 ml Pyridin wird mit 9,7 g p-Toluolsulfonsäurechlorid versetzt. Das Reaktionsgemisch wird 17 Stunden gerührt, dann in Essigester aufgenommen und mit Wasser gewaschen. Nach Trocknen und Eindampfen wird der Rückstand über Kieselgel mit Hexan-Essigester (3:1) gereinigt. Man erhält 15,1 g (S)-2,2-Dimethyl-4-(p-tolylsulfonyloxymethyl)-3-oxazolidincarbonsäure-t-butylester.

b) Einer Suspension von 0,82 g Natriumhydrid in 50 ml DMF gibt man 4,6 g 2-Indolinon zu und rührt 90 Minuten. Dann gibt man 6,6 g des Produkts von a) in 60 ml DMF zu und rührt das Reaktionsgemisch bei 50°C über Nacht. Nach Abdampfen des Lösungsmittels wird der Rückstand in Essigester aufgenommen und mit Wasser gewaschen. Nach Trocknen und Eindampfen des Lösungsmittels wird der Rückstand über Kieselgel mit Essigester/Hexan (1/4) gereinigt. Man erhält 2,5 g (R)-2,2-Dimethyl-4-(2-oxo-1-indolinylmethyl)-3-oxazolidincarbonsäure-t-butylester.

c) Eine Lösung von 2,5 g des Produkts von b) in 30 ml Methanol wird mit 36 ml 2N Salzsäure versetzt und über Nacht gerührt. Nach Abdampfen des Lösungsmittels wird der Rückstand mit Toluol azeotropiert. 1,56 g des erhaltenen 1-[(R)-2-Amino-3-hydroxypropyl]-2-indolinons werden in 2 Aequivalent 1N Natronlauge gelöst. Dazu gibt man 0,8 g Natriumbicarbonat in 8,1 ml Wasser und anschliessend eine

Lösung von 1,46 g 2-Naphthylsulfochlorid in 28 ml Dioxan und rührt über Nacht. Das Reaktionsgemisch wird auf 200 ml wässriges 5% Kaliumhydrogensulfat/10% Kaliumsulfat gegossen und mit Essigester extrahiert. Nach Waschen der organischen Phase mit Wasser und Trocknen wird das Lösungsmittel abdestilliert. Der Rückstand wird über Kieselgel mit Methylenchlorid/Methanol (98/2) gereinigt. Man erhält 1,9 g N-[(R)-2-Hydroxy-1-(2-oxo-1-indolinylmethyl)äthyl]-2-naphthylsulfonamid.

d) Eine Lösung von 1,82 g des Produkts von c) in 70 ml Aceton wird bei 0 °C mit 17,4 ml Jones-Reagens versetzt und 4 Stunden gerührt. Das Reaktionsgemisch wird auf Eis gegossen und mit Essigester extrahiert. Nach Waschen der organischen Phase mit Wasser, Trocknen und Eindampfen wird das Produkt über Kieselgel mit Methylenchlorid/Methanol (19/1) und 1% Essigsäure gereinigt. Man erhält 1,5 g N-(2-Naphthylsulfonyl)-3-(2,3-dioxo-1-indolinyl)-D-alanin.

e) Analog Beispiel 5 erhält man aus dem Produkt von d) das (R)-N-[(RS)-1-Amidino-3-piperidinylmethyl]-α-(2-naphthylsulfonamido-2,3-dioxo-1-indolinpropionamid-acetat (Epimere 1:1), FAB-MS: 671 $(M+H)^+$.

Beispiel 9

a) Zu einer Lösung von 7,3 g (R)-Phenylalanin in 120 ml 1N Natronlauge werden 20 g 2-Naphthylsulfochlorid in 150 ml Aether gegeben. Das Reaktionsgemisch wird 15 Stunden gerührt und abdekantiert. Die wässrige Phase wird abgetrennt, mit Aether gewaschen, mit HCl auf pH 3 angesäuert und mit Essigester extrahiert. Die organische Phase wird getrocknet und eingedampft. Die ausgefallenen Kristalle werden in Aether aufgeschlämmt und filtriert. Man erhält 14,4g N-(2-Naphthylsulfonyl)-3-phenyl-D-alanin, Smp. 146 °C.

b) Mit einem identischen Verfahren werden folgende N-sulfonierte Aminosäuren hergestellt:

N-(2-Naphthylsulfonyl)-3-p-chlorphenyl-D-alanin, Smp. 154 °C
N-(2-Naphthylsulfonyl)-3-p-cyanophenyl-D-alanin, Smp. 182 °C
N-(2-Naphthylsulfonyl)-3-p-nitrophenyl-D-alanin, Smp. 218 °C
N-(2-Naphthylsulfonyl)-3-benzyl-D-alanin, Smp. 138 °C
N-(2-Naphthylsulfonyl)-D-tryptophan, Smp. 167 °C
N-(4-Biphenylsulfonyl)-D-tryptophan, Smp. 227-230 °C
N-(2-Anthrylsulfonyl)-D-tryptophan, Smp. 210 °C
N-(4-Nitrophenylsulfonyl)-D-tryptophan, MS: $M^+$ 389
N-(2-Nitrophenylsulfonyl)-D-tryptophan, Smp. 70-80 °C
N-(3-Nitrophenylsulfonyl)-D-tryptophan, Smp. 80-84 °C
N-(4-Benzyloxyphenylsulfonyl)-D-tryptophan, Smp. 193 °C
N-(3,4-Dimethoxyphenylsulfonyl)-D-tryptophan, Smp. 182-184 °C
N-(2,3,6-Trimethyl-4-methoxyphenylsulfonyl)-D-tryptophan, MS: $M^+$ 416
N-(2,4,6-Triisopropylphenylsulfonyl)-D-tryptophan, MS: $M^+$ 470
N-(2-Naphthylsulfonyl)-5-methyltryptophan, Smp. 192 °C
(R)-2-(2-Naphthylsulfonyl)-1,2,3,4-tetrahydro-3-isochinolincarbonsäure,
m/e 322, 191, 176, 150, 127.

c) Analog Beispiel 5 erhält man aus dem Produkt von a) das (R)-N-[(RS)-1-Amidino-3-piperidinylmethyl]-α-(2-naphthylsulfonamido)hydrocinnamidbisulfit (Epimere 1:1), FAB-MS: 494 $(M+H)^+$.

Beispiel 10

a) 1,1 g Natriumhydrid-Dispersion (60%) werden in 50 ml DMF aufgeschlämmt. Dazu werden 5,23 g 4-Methyl-3H-1,4-benzodiazepin-2,5(1H,4H)-dion so zugegeben, dass die Temperatur 35 °C nicht übersteigt und 90 Minuten bei Raumtemperatur gerührt. Anschliessend gibt man eine Lösung von 5,3 g (S)-2,2-Dimethyl-4-(p-tolylsulfonyloxymethyl)-3-oxazolidincarbonsäure-t-butylester (Beispiel 6a) in 50 ml DMF zu und rührt über Nacht bei 50 °C. Das Reaktionsgemisch wird eingedampft, der Rückstand in Essigester aufgenommen, mit Wasser gewaschen und getrocknet. Nach Eindampfen des Lösungsmittels wird der Rückstand über Kieselgel mit Essigester/Hexan (1/1) gereinigt. Man erhält 1,85 g (R)-(2,3,4,5-Tetrahydro-4-methyl-2,5-dioxo-1H-1,4-benzodiazepin-1-ylmethyl)-2,2-dimethyl-3-oxazolidincarbonsäure-t-butylester.

b) Eine Lösung des Produkts von a) in 37 ml Methanol wird mit 23 ml 2N Salzsäure versetzt. Nach Rühren über Nacht wird das Reaktionsgemisch eingedampft. 1,37 g des erhaltenen 1-[(R)-2-Amino-3-hydroxypropyl]-3,4-dihydro-4-methyl-2H-1,4-benzodiazepin-2,5(1H)-dions werden in 15 ml Pyridin gelöst und mit 2,08 g 2-Naphthylsulfochlorid versetzt. Nach 3 Stunden wird das Reaktionsgemisch auf 2N Salzsäure gegossen und mit Essigester extrahiert. Man wäscht mit Wasser, trocknet und dampft das Lösungsmittel ab. Der Rückstand wird über Kieselgel mit Methylenchlorid/Methanol (97/3) gereinigt. Man

isoliert 0,5 g N-[(R)-1-Hydroxymethyl-2-(2,3,4,5-tetrahydro-4-methyl-2,5-dioxo-1H-1,4-benzodiazepin-1-ylmethyl)äthyl]-2-naphthylsulfonamid.

c) Eine Lösung von 0,4 g des Produkts von b) in 12 ml Aceton wird bei 0 °C mit 3 ml Jones-Reagens versetzt. Nach 3 Stunden bei Raumtemperatur wird das Reaktionsgemisch auf Eis gegossen und mit Essigester extrahiert. Nach Waschen mit Wasser und Trocknen erhält man nach Eindampfen 0,4 g N-(2-Naphthylsulfonyl)-3-(2,3,4,5-tetrahydro-4-methyl-2,5-dioxo-1H-1,4-benzodiazepin-1-yl)-D-alanin.

d) Analog Beispiel 5 erhält man au dem Produkt von c) das (R)-N-[(RS)-1-Amidino-3-piperidinylmethyl]-1,2,3,4-tetrahydro-2-methyl-α-(2-naphthylsulfonamido)-1,4-dioxo-5H-2,5-benzodiazepin-5-propionamid (Epimere 1:1), FAB-MS: 606 $(M+H)^+$.

## Beispiel 11

a) Einer Lösung von 0,9 g D-Tryptophan in 4,8 ml 1N NaOH und 4,8 ml Aceton wird eine Lösung von 1,25 g 6-Acetoxynaphthyl-2-sulfonylchlorid (Monatsh. 49, 1928, 96) in 4,4 ml Aceton zugetropft und das pH durch Zugabe von 1N NaOH auf 9 gehalten. Dann wird das Aceton eingedampft, die resultierende Suspension mit 1N HCl angesäuert und das Produkt mit Essigester extrahiert. Das Produkt wird auf Kieselgel mit Essigester/Aceton (1:1) chromatographiert. Die Hauptfraktion wird in 8,8 ml einer methanolischen Lösung von 0,5N NaOH aufgenommen und 17 Stunden gerührt. Die Lösung wird abgedampft, mit Wasser und 1N HCl versetzt und mit Essigester extrahiert. Reinigung auf Kieselgel mit Essigester-Aceton (3:2) ergibt 0,54 g N-(6-Hydroxy-2-naphthylsulfonyl)-D-tryptophan, MS: $M^+$ 410, m/e 207,130.

b) Analog Beispiel 5 erhält man aus dem Produkt von a) das (R)-N-[(RS)-1-Amidino-3-piperidinylmethyl]-α-(6-hydroxy-2-naphthylsulfonamido)indol-3-propionamid-hydrochlorid (Epimere 1:1), FAB-MS: 549 $(M+H)^+$.

## Beispiel 12

Analog Beispiel 5 und 9 stellt man folgende Verbindungen her:

a) (R)-N-[(RS)-1-Amidino-3-piperidinylmethyl]-p-fluor-α-(2-naphthylsulfonamido)hydrocinnamamid-bisulfit (Epimere 1:1), FAB-MS: 512 $(M+H)^+$.

b) (R)-N-[(RS)-1-Amidino-3-piperidinylmethyl]-p-chlor-α-(2-naphthylsulfonamido)hydrocinnamamid-bisulfit (Epimere 1:1), FAB-MS: 528 $(M+H)^+$.

c) (R)-N-[(RS)-1-Amidino-3-piperidinylmethyl]-p-cyano-α-(2-naphthylsulfonamido)hydrocinnamamid-bisulfit (Epimere 1:1), FAB-MS: 519 $(M+H)^+$.

d) (R)-N-[(RS)-1-Amidino-3-piperidinylmethyl]-α-(2-naphthylsulfonamido)-p-nitrohydrocinnamamid-bisulfit (Epimere 1:1), FAB-MS: 539 $(M+H)^+$.

e) (R)-p-Amino-N-[(RS)-1-amidino-3-piperidinylmethyl]-α-(2-naphthylsulfonamido)hydrocinnamamid-bisulfit (Epimere 1:1), FAB-MS: 509 $(M+H)^+$.

f) (R)-N-[(RS)-1-Amidino-3-piperidinylmethyl]-3-(p-hydroxyphenyl)-2-(2-naphthylsulfonamido)propionamid-bisulfit (Epimere 1:1), FAB-MS: 510 $(M+H)^+$.

## Beispiel 13

Analog Beispiel 5 und 9 stellt man folgende Verbindungen her:

a) (R)-N-[(RS)-1-Amidin-3-piperidinylmethyl]-α-(2-naphthylsulfonamido)-o-nitrohydrocinnamamid-bisulfit (Epimere 1:1), FAB-MS: 539 $(M+H)^+$.

b) (R)-N-[(RS)-1-Amidino-3-piperidinylmethyl]-α-(2-naphthylsulfonamido)cyclohexanpropionamid-bisulfit (Epimere 1:1), FAB-MS: 500 $(M+H)^+$.

c) (R)-N-[(RS)-1-Amidino-3-piperidinylmethyl]-2-(2-naphthylsulfonamido)-4-phenylbutyramid-bisulfit (Epimere 1:1), FAB-MS: 508 $(M+H)^+$.

d) (R)-N-[(RS)-1-Amidino-3-piperidinylmethyl]-α-(2-naphthylsulfonamido)cyclohexanbutyramid-bisulfit (Epimere 1:1), FAB-MS: 514 $(M+H)^+$.

e) (R)-N-[(RS)-1-Amidino-3-piperidinylmethyl]-α-(2-naphthylsulfonamido)-1-naphthylpropionamid-bisulfit, FAB-MS: 586 $(M+H)^+$.

f) (R)-N-[(RS)-1-Amidino-3-piperidinylmethyl]-α-(2-naphthylsulfonamido)-2-naphthylpropionamid-bisulfit (Epimere 1:1), FAB-MS: 544 $(M+H)^+$.

g) (αR,2S,4aR und/oder S,8aR und/oder S)-N-[(RS)-1-Amidino-3-piperidinylmethyl]decahydro-α-(2-naphthylsulfonamido)-2-naphthylpropionamid-bisulfit (Gemisch von Diastereomeren), FAB-MS: 554 (M+H).

h) (R)-N-[(RS)-1-Amidino-3-piperidinylmethyl]-α-(2-naphthylsulfonamido)-4-imidazolpropionamid-bisulfit (Epimere 1:1), FAB-MS: 484 $(M+H)^+$.

i) (R)-N-[(RS)-1-Amidino-3-piperidinylmethyl]-α-(2-naphthylsulfonamido)-2-thienylpropionamid-bisulfit (Epimere 1:1), FAB-MS: 500 $(M+H)^+$.

Beispiel 14

Analog Beispiel 5 und 11 stellt man folgende Verbindungen her:

a) (R)-N-[(RS)-1-Amidino-3-piperidinylmethyl]-α-(3-methyl-8-chinolinsulfonamido)indol-3-propionamid-bisulfit (Epimere 1:1), FAB-MS: 548 $(M+H)^+$.

b) (R)-N-[(RS)-1-Amidino-3-piperidinylmethyl]-α-[(RS)-3-methyl-1,2,3,4-tetrahydro-8-chinolinsulfonamido]-indol-3-propionamid-bisulfit, FAB-MS: 552 $(M+H)^+$.

c) (R)-N-[(RS)-1-Amidino-3-piperidinylmethyl]-α-(4-biphenylsulfonamido)indol-3-propionamid-hydrochlorid (Epimere 1:1), FAB-MS: 559 $(M+H)^+$.

d) (R)-N-[(RS)-1-Amidino-3-piperidinylmethyl]-α-(2-anthrylsulfonamido)indol-3-propionamid-hydrochlorid (Epimere 1:1), FAB-MS: 583 $(M+H)^+$.

e) (R)-N-[(RS)-1-Amidino-3-piperidinylmethyl]-α-[5-(1-methyl-5-trifluormethylpyrazol-3-yl)-2-thienylsulfonamido]indol-3-propionamid-bisulfit (Epimere 1:1), FAB-MS: 637 $(M+H)^+$.

f) (R)-N-[(RS)-1-Amidino-3-piperidinylmethyl]-α-(p-jodbenzolsulfonamido)indol-3-propionamid-hydrochlorid (Epimere 1:1), FAB-MS: 609 $(M+H)^+$.

g) (R)-N-[(RS)-1-Amidino-3-piperidinylmethyl]-α-(o-jodbenzolsulfonamido)indol-3-propionamid-hydrochlorid (Epimere 1:1), FAB-MS: 608 $(M+H)^+$.

h) (R)-N-[(RS)-1-Amidino-3-piperidinylmethyl]-α-(p-nitrobenzolsulfonamido)indol-3-propionamid-hydrochlorid (Epimere 1:1), FAB-MS: 528 $(M+H)^+$.

i) (R)-N-[(RS)-1-Amidino-3-piperidinylmethyl]-α-(o-nitrobenzolsulfonamido)indol-3-propionamid-hydrochlorid (Epimere 1:1), FAB-MS: 528 $(M+H)^+$.

j) (R)-N-[(RS)-1-Amidino-3-piperidinylmethyl]-α-(m-nitrobenzolsulfonamido)indol-3-propionamid-hydrochlorid (Epimere 1:1), FAB-MS: 528 $(M+H)^+$.

k) (R)-N-[(RS)-1-Amidino-3-piperidinylmethyl]-α-(p-benzyloxybenzolsulfonamido)indol-3-propionamid-hydrochlorid (Epimere 1:1), FAB-MS: 589 $(M+H)^+$.

l) (R)-N-[(RS)-1-Amidino-3-piperidinylmethyl]-α-(3,4-dimethoxybenzolsulfonamido)indol-3-propionamid-hydrochlorid (Epimere 1:1), FAB-MS: 543 $(M+H)^+$.

m) (R)-N-[(RS)-1-Ainidino-3-piperidinylmethyl]-α-(4-methoxy-2,3,6-trimethylbenzolsulfonamido)indol-3-propionamid-hydrochlorid (Epimere 1:1), FAB-MS: 555 $(M+H)^+$.

n) (R)-N-[(RS)-1-Amidino-3-piperidinylmethyl]-α-(2,4,6-triisopropylbenzolsulfonamido)indol-3-propionamid-hydrochlorid (Epimere 1:1), FAB-MS: 609 $(M+H)^+$.

Beispiel 15

Analog Beispiel 5 stellt man folgende Verbindungen her:

a) (RS)-N-[(RS)-1-Amidino-3-piperidinylmethyl]-5-methyl-α-(2-naphthylsulfonamido)indol-3-propionamid-bisulfit, FAB-MS: 547 $(M+H)^+$.

b) (RS)-N-[(RS)-1-Amidino-3-piperidinylmethyl]-5-fluor-α-(2-naphthylsulfonamido)indol-3-propionamid-bisulfit, FAB-MS: 551 $(M+H)^+$.

c) 3-[(R)-2-[(RS)-1-Amidino-3-piperidinylmethylcarbamoyl]-2-(2-naphthylsulfonamido)äthyl]indol-1-essigsäure-bisulfit (Epimere 1:1), FAB-MS: 591 $(M+H)^+$.

Beispiel 16

Analog Beispiel 6 bzw. 7 erhält man:

a) (R)-N-[(R)-1-Amidino-3-piperidinylmethyl]-α-(2-naphthylsulfonamido)indol-3-propionamid-bisulfit, FAB-MS: 533 $(M+H)^+$ bzw.

b) N-(2-Naphthylsulfonyl)-D-tryptophan-(R)-1-amidino-3-piperidinylmethylester-p-toluolsulfonat (1:1), FAB-MS: 534 $(M+H)^+$.

Beispiel 17

Analog Beispiel 6 und 7 erhält man folgende Verbindungen:

a) (R)-N-[(R)-1-Amidino-3-piperidinylmethyl]-α-(p-jodbenzolsulfonamido)indol-3-propionamid-p-toluolsulfonat (1:1), FAB-MS: 609 $(M+H)^+$.

b) (R)-N-[(S)-1-Amidino-3-piperidinylmethyl]-α-(p-jodbenzolsulfonamido)indol-3-propionamid-p-toluolsulfonat (1:1), FAB-MS: 609 $(M+H)^+$.

c) N-(p-Jodphenylsulfonyl)-D-tryptophan-(R)-1-amidino-3-piperidinylmethylester-p-toluolsulfonat (1:1), FAB-MS: 609 $(M+H)^+$.

d) N-(p-Jodphenylsulfonyl)-D-tryptophan-(S)-1-amidino-3-piperidinylmethylester-p-toluolsulfonat (1:1), FAB-MS: 610 $(M+H)^+$.

e) (R)-N-[(R)-1-Amidino-3-piperidinylmethyl]-α-(p-jodbenzolsulfonamido)-2-naphthylpropionamid-p-toluolsulfonat (1:1), FAB-MS: 620 $(M+H)^+$.

f) (R)-N-[(S)-1-Amidino-3-piperidinylmethyl]-α-(p-jodbenzolsulfonamido)-2-naphthylpropionamid-p-toluolsulfonat (1:1), FAB-MS: 620 $(M+H)^+$.

g) N-(p-Jodphenylsulfonyl)-3-(2-naphthyl)-D-alanin-(R)-1-amidino-3-piperidinylmethylester-p-toluolsulfonat (1:1), FAB-MS: 621 $(M+H)^+$.

h) N-(p-Jodphenylsulfonyl)-3-(2-naphthyl)-D-alanin-(S)-1-amidino-3-piperidinylmethylester-p-toluolsulfonat (1:1), FAB-MS: 621 $(M+H)^+$.

i) (R)-N-[(R)-1-Amidino-3-piperidinylmethyl]-α-(p-jodbenzolsulfonamido)-p-nitrohydrocinnamamid-bisulfit (2:1), FAB-MS: 615 $(M+H)^+$.

j) (R)-N-[(S)-1-Amidino-3-piperidinylmethyl]-α-(p-jodbenzolsulfonamido)-p-nitrohydrocinnamamid-p-toluolsulfonat (1:1), FAB-MS: 615 $(M+H)^+$.

k) N-(p-Jodphenylsulfonyl)-3-(p-nitrophenyl)-D-alanin-(R)-1-amidino-3-piperidinylmethylester-p-toluolsulfonat (1:1), FAB-MS: 616 $(M+H)^+$.

l) N-(p-Jodphenylsulfonyl)-3-(p-nitrophenyl)-D-alanin-(S)-1-amidino-3-piperidinylmethylester-p-toluolsulfonat (1:1), FAB-MS: 616 $(M+H)^+$.

Beispiel 18

Analog Beispiel 5 stellt man folgende Verbindungen her:

a) (R)-N-[(RS)-1-Amidino-3-piperidinylmethyl]hexahydro-α-2-naphthylsulfonamido-γ-oxo-1H-azepin-1-butyramid-bisulfit(Epimere 1:1), FAB-MS: 543 $(M+H)^+$.

b) 1-[(R)-3-[(RS)-1-Amidino-3-piperidinylmethylcarbamoyl]-3-(2-naphthylsulfonamido)propionyl]-L-prolinmethylester (Epimere 1:1), FAB-MS: 573 $(M+H)^+$.

c) 1-[(R)-3-[(RS)-1-Amidin-3-piperidinylmethylcarbamoyl]-3-(2-naphthylsulfonamido)propionyl]-L-prolin (Epimere 1:1), FAB-MS: 559 $(M+H)^+$.

d) (R)-N-[(RS)-1-Amidino-3-piperidinylmethyl]-3,4-dihydro-α-(2-naphthylsulfonamido)-γ-oxo-2(1H)-isochinolinbutyramid (Epimere 1:1), FAB-MS: 577 $(M+H)^+$.

e) (RS)-N-[(RS)-1-Amidino-3-piperidinylmethyl]-2-(2-naphthylsulfonamido)-3-(o-nitrobenzoyl)propionamid-bisulfit, FAB-MS: 567 $(M+H)^+$.

f) (R)-N-[(RS)-1-Amidino-3-piperidinylmethyl]-3-(o-aminobenzoyl)-2-(naphthylsulfonamido)propionamid (Epimere 1:1), FAB-MS: 537 $(M+H)^+$.

g) (R)-N-[(RS)-1-Amidino-3-piperidinylmethyl]-3-anthraniloyl-2-(p-jodbenzolsulfonamido)propionamid-trifluoracetat (Epimere 1:1), FAB-MS: 613 $(M+H)^+$.

h) (R)-N-[(RS)-1-Amidino-3-piperidinylmethyl]-3-(o-formamidobenzoyl)-α-(2-naphthylsulfonamido)-propionamidacetat (Epimere 1:1), FAB-MS: 565 $(M+H)^+$.

Beispiel 19

a) Zu 164 ml 70%iger Natronlauge gibt man 199 g 2-Aminoäthylhydrogensulfat, erwärmt die Lösung auf 50 °C und tropft eine Lösung von (RS)-Benzyloxymethyloxiran in 280 ml Methanol zu. Nach 1 Stunde bei 50 °C gibt man weitere 280 ml 70%ige Natronlauge zu und rührt die Lösung über Nacht. Dann wird das Reaktionsgemisch auf Eis gegossen und mit Toluol extrahiert. Die organische Phase wird mit Wasser gewaschen, getrocknet und eingedampft. Man erhält nach Destillation 26,8 g rac-2-Benzyloxymethylmorpholin, MS: $M^+$ -91 = 116 (Benzyl).

b) Zu 26,8 g des Produkts von a) in 287 ml Dioxan gibt man eine Lösung von 31,0 g Di-t-butyldicarbonat in 287 ml Dioxan und rührt das Reaktionsgemisch über Nacht. Nach Eindampfen des Lösungsmittels

und Chromatographie des Rückstandes an Kieselgel mit Essigester-Hexan erhält man 15 g t-Butyl-rac-2-benzyloxymethyl-4-morpholincarboxylat, MS: $M^+$ -56 = 251 (Isobutylen).

c) Man hydriert eine Lösung von 15 g des Produkts von b) in 300 ml Aethanol in Gegenwart von 1,5 g Pd/C bei Normalbedingungen und erhält nach Filtration und Eindampfen des Lösungsmittels quantitativ t-Butyl-rac-2-hydroxymethyl-4-morpholincarboxylat, MS: $M^+$ = 217.

d) Eine Lösung von 8,8 g des Produkts von c) in 44 ml Pyridin wird mit 9,4g p-Chlorbenzolsulfochlorid versetzt. Nach 5 Stunden Rühren wird das Reaktionsgemisch eingedampft, der Rückstand in Essigester aufgenommen und mit 10%iger Kupfersulfatlösung gewaschen. Nach Trocknen der organischen Phase, Eindampfen des Lösungsmittels und Chromatographie des Rückstandes an Kieselgel mit Essigester-Hexan erhält man 14,7 g t-Butyl-rac-2-(p-chlorphenylsulfonyloxymethyl)-4-morpholincarboxylat, MS: 392 $(M+H)^+$.

e) Einer Lösung von 14,7g des Produkts von d) in 91 ml DMF gibt man 7,3 g Natriumazid zu. Nach 24 Stunden Rühren bei 50 °C wird das Reaktionsgemisch auf Eis gegossen und mit Aether extrahiert. Die organische Phase wird mit Wasser gewaschen, getrocknet und eingedampft. Man erhält 8,1 g t-Butyl-rac-2-azidomethyl-4-morpholincarboxylat.

f) Man hydriert eine Lösung von 8,1 g des Produkts von e) in 92 ml Aethanol in Gegenwart von 0,8 g Platinoxid während 4 Stunden bei Normalbedingungen und isoliert 6,5 g t-Butyl-rac-2-aminomethyl-4-morpholincarboxylat, MS: $(M+H)^+$ -56 = 159 (Isobutylen).

g) Eine Lösung von 3,0 g N-(2-Naphthylsulfonyl)-D-tryptophan (Beispiel 9) in 35 ml DMF wird mit 1,2 ml 4-Aethylmorpholin und mit 3,4 g Benztriazol-1-yloxy-tris-(dimethylamino)-phosphonium-hexafluorphosphat versetzt. Dazu gibt man eine Lösung von 2,0 g des Produkts von f) in 2 ml DMF und rührt die Lösung über Nacht. Dann wird das Lösungsmittel abdestilliert, der Rückstand in Essigester aufgenommen und mit Wasser gewaschen. Nach Trocknen, Eindampfen des Lösungsmittels und Chromatographie des Rückstandes an Kieselgel mit Essigester erhält man 4,5 g t-Butyl-(RS)-2-N-(2-naphthylsulfonyl)-D-tryptophylaminomethyl-4-morpholincarboxylat, MS: $(M+H)^+$ -56 = 537 (Isobutylen).

h) Eine Lösung von 2 g des Produkts von g) in 20 ml Essigester wird mit 20 ml einer 4 molaren Lösung von HCl in Essigester versetzt. Nach Rühren dampft man die Lösung zur Trockene ein. Der Rückstand wird in 20 ml DMF gelöst, mit 1,4 ml Triäthylamin und 0,5 g Formamidinsulfonsäure versetzt und 17 Stunden bei Raumtemperatur gerührt. Dann wird das Lösungsmittel verdampft, der Rückstand in 50 ml Essigester und 10 ml Methanol aufgenommen und diese Lösung mit Wasser gewaschen. Nach Trocknen der organischen Phase, Eindampfen des Lösungsmittels und Chromatographie des Rückstandes an einer RP-Säule (alkylsilyliertes Kieselgel) mit Wasser-Acetonitril erhält man 0,1 g (R)-N-[(RS)-4-Amidino-2-morpholinylmethyl]-α-(2-naphthylsulfonamido)indol-3-propionamid-bisulfit, MS: 535 $(M+H)^+$.

Beispiel 20

Analog Beispiel 19g) und h) erhält man aus t-Butyl-rac-2-hydroxymethyl-4-morpholincarboxylat (Beispiel 19c) und N-(2-Naphthylsulfonyl)-D-tryptophan N-(2-Naphthylsulfonyl)-D-tryptophan-(RS)-4-amidino-2-morpholinylmethylester-bisulfit, MS: 536 $(M+H)^+$.

Beispiel 21

Analog Beispiel 19 erhält man

a) (R)-N-[(RS)-4-Amidino-2-morpholinylmethyl]-α-(2-naphthylsulfonyl)-p-nitrohydrocinnamamid-bisulfit, MS: 541 $(M+H)^+$

b) (R)-N-[(RS)-4-Amidino-2-morpholinylmethyl]-α-(2-naphthylsulfonamido)-2-thienylpropionamid-bisulfit, MS: 502 $(M+H)^+$

c) (R)-N-[(RS)-4-Amidin-2-morpholinylmethyl]-3-(o-aminobenzoyl)-2-(2-naphthylsulfonamido)propionamid-bisulfit, MS: 539 $(M+H)^+$.

Beispiel 22

a) Eine Lösung von 10 g (D)-Phenylalaninbenzylester-p-toluolsulfonat in 150 ml Methylenchlorid wird mit 8,2 ml Triäthylamin versetzt und auf 0 °C gekühlt. Man gibt 5,8 g 2-Naphthylsulfochlorid zu und rührt 12 Stunden bei Raumtemperatur. Das Reaktionsgemisch wird eingedampft und in 200 ml Essigester aufgenommen, das ausgefallene Triäthylammoniumchloridsalz wird abgenutscht und die Mutterlauge mit Wasser gewaschen. Nach Eindampfen der organischen Phase und Auskristallisieren aus Hexan erhält man 9,7 g N-(2-Naphthylsulfonyl)-3-phenyl-D-alaninbenzylester, Smp. 107 °C.

b) Eine Lösung von 3 g des Produkts von a) in 40 ml THF wird bei -80 °C mit 5 ml 1.6M Butyllithium in Hexan und dann mit 1,2 ml Bromessigsäure-t-butylester versetzt. Nach 2-stündigem Rühren bei Raumtemperatur wird das Reaktionsgemisch in 200 ml Essigester aufgenommen, mit Wasser gewaschen und die organische Phase eingedampft. Das Produkt wird über Kieselgel mit Hexan/Essigester (9:1) gereinigt. Man erhält 1,35 g N-(t-Butoxycarbonylmethyl)-N-(2-naphthylsulfonyl)-3-phenyl-D-alaninbenzylester, NMR ($CDCl_3$) 1,45 (s, 9H, t-Butyl).

c) Eine Lösung von 1,35 g des Produkts von b) in 50 ml Aethanol wird in Gegenwart von 0,3 g Pd/C hydriert. Nach Eindampfen des Reaktionsgemisches und Azeotropieren mit Toluol erhält man 0,95 g N-(t-Butoxycarbonylmethyl)-N-(2-naphthylsulfonyl)-3-phenyl-D-alanin.

d) Analog Beispiel 5 erhält man N-[(R)-α-[[(S)-1-Amidino-3-piperidinyl]methylcarbamoyl]phenäthyl]-N-(2-naphthylsulfonyl)glycin, FAB-MS: 552 $(M+H)^+$.

Beispiel 23

a) Zu einer Lösung von 10,0 g N-Boc-L-Asparaginsäure-β-benzylester in 200 ml DMF gibt man 13,7 g Benzotriazol-1-yloxy-tris(dimethylamino)phosphonium-hexafluorophosphat und 16,9 ml Benzylamin und rührt das Reaktionsgemisch über Nacht bei Raumtemperatur. Dann wird das Lösungsmittel abgedampft, der Rückstand in Essigester aufgenommen und mit Wasser gewaschen. Nach Trocknen, Eindampfen und Chromatographie an Kieselgel mit Essigester-Hexan (1:4) erhalt man 5,4 g (S)-3-(1,1-Dimethyläthoxycarbonylamino)-4-benzylamino-4-oxobuttersäurebenzylester.

b) Eine Lösung von 2,0 g des Produkts von a) in 10 ml Essigester wird mit 10 ml einer 4 molaren Lösung von HCl in Essigester versetzt. Nach 3 Stunden Rühren wird eingedampft und der Rückstand in 30 ml Dioxan suspendiert. Man gibt 5 ml 1N Natronlauge, 0,8 g Natriumbicarbonat, 20 ml Wasser und dann eine Lösung von 1,1 g 2-Naphthylsulfochlorid in 15 ml Dioxan zu und rührt über Nacht bei Raumtemperatur. Das Reaktionsgemisch wird auf eine 5%ige Kaliumhydrogensulfat - 10%ige Kaliumsulfatlösung gegossen und mit Essigester extrahiert. Nach Trocknen und Eindampfen der organischen Phase wird der Rückstand aus Essigester-Hexan umkristallisiert. Man erhält 1,8 g (S)-2-Benzylcarbamoyl-N-(2-naphthylsulfonyl)-β-alaninbenzylester.

c) Eine Lösung von 1,0 g des Produkts von b) in 20 ml Methanol wird mit 2 ml 1N Natronlauge versetzt und über Nacht bei Raumtemperatur gerührt. Man säuert an, extrahiert mit Essigester und kristallisiert den nach Eindampfen erhaltenen Rückstand aus Methanol-Methylenchlorid-Hexan. Man erhält 0,3 g (S)-2-Benzylcarbamoyl-N-(2-naphthylsulfonyl)-β-alanin.

d) Analog Beispiel 5 erhält man (S)-N-[(RS)-1-Amidino-3-piperidinylmethyl]-3-benzylcarbamoyl-3-(2-naphthylsulfonamido)propionamid-triflouracetat (Epimere 1:1), FAB-MS: 551 $(M+H)^+$.

Beispiel 24

Analog Beispiel 23 erhält man via (R)-2-Benzylcarbamoyl-N-(2-naphthylsulfonyl)-β-alanin (R)-N-[(RS)-1-Amidino-3-piperidinylmethyl]-3-benzylcarbamoyl-3-(2-naphthylsulfonamido)propionamid-hydrochlorid, FAB-MS: 551 $(M+H)^+$.

Beispiel 25

Analog Beispiel 23 erhält man mittels Hexamethylenimin an Stelle von Benzylamin das (S)-N-[(RS)-1-Amidino-3-piperidinylmethyl]hexahydro-β-(2-naphthylsulfonamido)-γ-oxo-1H-1-azepinbutyramid-trifluoracetat, FAB-MS: 543 $(M+H)^+$.

Beispiel 26

Analog Beispiel 6 erhält man das (R)-N-[(S)-1-Amidino-3-piperidinylmathyl]-1,2,3,4-tetrahydro-2-(2-naphthylsulfonyl)-3-isochinolincarboxamidhydrochlorid, FAB-MS: 506 $(M+H)^+$.

Beispiel 27

Analog Beispiel 5 und 9 erhält man Methyl-o-[(R)-α-[(RS)-1-amidino-3-piperidinylmethylcarbamoyl]-p-nitrophenäthylsulfamoyl]benzoat, FAB-MS: 547 $(M+H)^+$.

Beispiel 28

Analog Beispiel 5 und 9 erhält man das (RS)-N-[[(RS)-1-Amidino-3-piperidinyl]methyl]-5-bromo-α-2-naphthylsulfonamidoindol-3-propionamidsulfit. FAB-MS: 611 $(M+H)^+$.

Beispiel 29

Analog Beispiel 6 und 9 stellt man folgende Verbindungen her:

a) (R)-N-[[(RS)-1-Amidino-3-piperidinyl]methyl]-p-jodo-α-2-naphthylsulfonamidohydrocinnamid-p-toluolsulfonat. FAB-MS: 620 $(M+H)^+$.

b) (R)-N-[[(S)-1-Amidino-3-piperidinyl]methyl]-α-2-naphthylsulfonamido-p-nitrohydrocinnamid-p-toluolsulfonat. FAB-MS: 539 $(M+H)^+$.

Beispiel 30

a) Eine auf 0° abgekühlte Lösung von 30,1 g (R)-4-Hydroxymethy-2,2-dimethyl-3-oxazolidincarbonsäure-t-butylester in 400 ml Methylenchlorid wird mit 27,2 ml Triäthylamin versetzt. Nach Zugabe von 12,1 ml Methansulfochlorid bei 0° wird das Reaktionsgemisch während 1 Stunde gerührt. Die Salze werden abfiltriert, das Filtrat eingedampft und das Produkt in Hexan auskristallisiert. Man erhält 32,7 g (S)-2,2-Dimethyl-4-(methansulfonyloxymethyl)-3-oxazolidincarbonsäure-t-butylester, $H^1$-NMR ($CDCl_3$): 1,48, s, 9H, t-Butyl; 3,04, s, 3H, Mesyl.

b) Eine Lösung von 29,1 g des Produkts von a) in 200 ml DMF wird mit 24,5 g Natriumazid versetzt. Die Suspension wird auf 50° erwärmt und während 24 Stunden gerührt. Die Salze werden dann abfiltriert und das Filtrat eingedampft. Das Rohprodukt wird in 400 ml Essigester gelöst und mit Wasser gewaschen. Die organische Phase wird getrocknet und eingedampft. Man erhält 20,2 g (R)-2,2-Dimethyl-4-(azidomethyl)-3-oxazolidin-carbonsäure-t-butylester, IR ($CHCl_3$) 2140 $cm^{-1}$ (Azid-Bande).

c) Eine Lösung von 20,2 g des Produkts von b) in 400 ml Dioxan wird mit 4 g Platinoxid versetzt. Das Reaktionsgemisch wird bei Raumtemperatur und normalem Druck hydriert. Nach Filtration und Eindampfen des Filtrats erhält man 17,9 g (R)-2-2-Dimethyl-4-(aminomethyl)-3-oxazolidincarbonsäure-t-butylester.

d) Eine Lösung des Produkts von c) in 300 ml Methylenchlorid wird bei 0° mit 19,9 ml Hünigbase (Aethyldiisopropylamin) und 12,2 ml Benzylchloroformiat versetzt. Nach Rühren wird das Reaktionsgemisch eingedampft, das Rohprodukt in 400 ml Essigester gelöst und mit 100 ml Wasser gewaschen. Die organische Phase wird getrocknet und eingedampft und das Rohmaterial auf Kieselgel mit Essigester/Hexan 1/9 gereinigt. Man erhält 26,4 g (R)-2,2-Dimethyl-4-(carbobenzoxy-aminomethyl)-3-oxazolidincarbonsäure-t-butylester.

e) Eine Lösung des Produkts von d) in 300 ml Methanol wird mit 36,2 ml 2N HCl versetzt. Nach 24 Stunden wird die Lösung eingedampft, in 400 ml Dioxan gelöst und mit 72,43 ml 1N NaOH versetzt. Nach Zugabe von 30,4 g Natriumbicarbonat wird die Suspension mit einer Lösung von 24,6 g 2-Naphthylsulfochlorid in 100 ml Dioxan versetzt. Die Suspension wird 15 Stunden gerührt, das Reaktionsgemisch dann abfiltriert, eingedampft, in Essigester aufgenommen, mit Wasser gewaschen und getrocknet. Die organische Phase wird eingedampft und das Rohprodukt auf Kieselgel mit Essigester/Hexan 3/7 gereinigt. Man erhält 25,85 g [(R)-3-Hydroxy-2(2-naphthylsulfonamido)propyl]carbamidsäurebenzylester, MS: 415 $(M+H)^+$, 371 ($-CO_2$), 347, 325, 281, 269, 225, 191, 135.

f) Eine Lösung von 5 g des Produkts von e) in 100 ml Aethanol wird nach Zugabe von 1 g 10% Pd/C bei Raumtemperatur, unter normalem Druck während 24 Stunden hydriert. Der Katalysator wird abfiltriert und mit Methanol gewaschen. Nach Eindampfen erhält man 2,8 g N-[(R)-2-Amino-1-hydroxymethyl)-äthyl]-2-naphthylsulfonamid. MS: 281 $(M+H)^+$ 251, 250, 221, 191, 128, 127, 60.

g) Eine Lösung von 1,3 g des Produkts von f) in 50 ml DMF wird mit 0,83 g Phenylglyoxalsäure und 0,9 ml Hünigbase versetzt. Nach Zugabe von 2,0 g Bentriazol-1-yloxy-tris(dimethylamino)phosphonium-hexafluorophosphat wird das Reaktionsgemisch während 5 Stunden gerührt. Das Lösungsmittel wird eingedampft und das Rohprodukt auf Kieselgel mit Hexan/Essigester 1/1 gereinigt. Man erhält 1,4 g N-[-(R)-3-Hydroxy-2-(2-naphthylsulfonamido)propyl]-2-phenylglyoxylsäureamid.

h) Eine Lösung von 0,42 g des Produkts von g) in 30 ml Aceton wird bei 0° mit 3,5 ml Jones-Reagenz (2,67 mg $CrO_3$ in Schwefelsäure) versetzt. Das Gemisch wird 1 Stunde gerührt, auf Eiswasser gegossen und mit Essigester extrahiert. Die organische Phase wird getrocknet und eingedampft. Man erhält 0,44 g (R)-2-(2-Naphthylsulfonamido)-3-(2-phenylglyoxylamido)propionsäure, FAB-MS: 427 $(M+1)^+$.

i) Analog Beispiel 5 erhält man aus dem Produkt von h) das (R)-N-[[(S)-1-Amidino-3-piperidinyl]methyl]-2-naphthylsulfonamido-3-(2-phenylglyoxylamido)propionamid-p-toluolsulfonat. FAB-MS: 565 $(M+H)^+$.

Beispiel 31

Analog Beispiel 5 und 9 erhält man

a) via N-[(6-Benzyloxy-2-naphthyl)sulfonyl]-D-tryptophan, FAB-MS: 394 $(M+H)^+$,

das (R)-N-[[(RS)-1-Amidino-3-piperidinyl]methyl]-α-(6-benzyloxy-2-naphthylsulfonamido)indol-3-propionamid-hydrochlorid, FAB-MS: 639 $(M^+)$,

b) via N-(1-Naphthylsulfonyl)-D-tryptophan, FAB-MS: 394 $(M+H)^+$,

das (R)-N-[[(RS)-1-Amidino-3-piperidinyl]methyl]-α-(1-naphthylsulfonamido)indol-3-propionamid-hydrochlorid, FAB-MS: 533 $(M+H)^+$,

c) via N-(2-Naphthylsulfonyl)-3-(m-nitrophenyl)-DL-alanin, MS: 400 $(M^+)$,

das (RS)-N-[[(RS)-1-Amidino-3-piperidinyl]methyl]-α-[2-naphthylsulfonamido]-m-nitrohydrocinnamamid-hydrochlorid, FAB-MS: 539 $(M+H)^+$.

Beispiel 32

a) Zu einer Lösung von 0,71 g D-Trypophan in 4,8 ml Wasser und 2,13 ml 1N NaOH werden 0,36 g Natriumhydrogencarbonat und eine Lösung von 0,50 g Methyl-p-(chlorsulfonyl)benzoat in 10 ml Aether zugegeben. Die entstandene Suspension wird 22 Stunden gerührt. Das Natriumsalz des N-[[p-(Methoxycarbonyl)phenyl]sulfonyl]-D-tryptophans wird abgesaugt und mit Wasser nachgespült (0,52 g), MS: 400 $(M-Na)^-$. Aus der Mutterlauge werden nach Ansäuren mit Zitronensäure 0,38 g Produkt als freie Säure isoliert.

b) Analog Beispiel 5 und 11 erhält man aus der Säure von a) das Methyl-p-[[(R)-1-[[[(RS)-1 Amidino-3-piperidinyl]methyl]carbamoyl]-2-indol-3-yläthyl]sulfamoyl]benzoat-hydrochlorid, FAB-MS: 541 $(M+H)^+$.

Beispiel 33

Analog Beispiel 31 erhält man das (RS)-N-[[(S)-1-Amidino-3-piperidinyl]methyl]-α-2-naphthylsulfonamido-m-nitrohydrocinnamamid-hydrochlorid, FAB-MS: 539 $(M+H)^+$.

Beispiel 34

Eine Lösung von 0,15 g (RS)-N-[[(S)-1-Amidino-3-piperidinyl]methyl]-α-2-naphthylsulfonamido-m-nitrohydrocinnamamid-hydrochlorid(Beispiel 33) in 50 ml Aethanol wird mit 0,12 g 10% Pd/C versetzt und bei Raumtemperatur unter Normaldruck hydriert. Die Reaktionslösung wird filtriert, abgedampft, der Rückstand in Methanol gelöst und mit Aether versetzt. Das ausgeschiedene (RS)-N-[[(S)-1-Amidino-3-piperidinyl]-methyl]-m-amino-α-2-naphthylsulfonamido-hydrocinnamamid-hydrochlorid wird abgesaugt und mit Aether nachgewaschen; Ausbeute 0,12 g, FAB-MS: 509 $(M+H)^+$.

Beispiel 35

a) Eine Lösung von 50 g N-(2-Naphthylsulfonyl)-3-p-nitrophenyl-D-alanin (Beispiel 9b) in 100 ml DMF mit 45 ml Raney-Nickel versetzt und bei Raumtemperatur und Normaldruck hydriert. Die Reaktionslösung wird filtriert, dann konzentriert und in Wasser gegossen. Ausgeschiedenes 3-(p-Aminophenyl)-N-(2-naphthylsulfonyl)-D-alanin wird abgesaugt, mit Wasser gewaschen und getrocknet. Ausbeute 37,6 g, Smp. 211-212°. MS: 370 $)M^+)$.

b) Eine Suspension von 1,0 g des Produkts von a) in 15 ml Methylenchlorid wird mit 1,13 ml Triäthylamin versetzt. Die entstandene Lösung wird im Eisbad abgekühlt und mit einer Lösung von 0,3 ml Oxalsäurechloridmonoäthylester in 5 ml Methylenchlorid zugegeben. Nach weiteren 30 Minuten im Eisbad wird die Lösung über Nacht bei Raumtemperatur gerührt. Das Reaktionsgemisch wird eingeengt, der Rückstand in Wasser gelöst, mit 10%iger Zitronensäure angesäuert und das ausgeschiedene Produkt abgenutscht. Dies wird in Essigester gelöst, mit Wasser gewaschen und getrocknet. Nach Abdampfen resultieren 0,92 g Oxalsäureesteramid, welches analog Beispiel 5 0,87 g Aethyl-4'-[(R)-2-[[[-(RS)-1-amidino-3-piperidinyl]methyl]carbamoyl]-2-[(2-naphthylsulfonamido)äthyl]oxanilat-hydrochloridergibt. FAB-MS: 609 $(M+H)^+$.

Beispiel 36

Analog Beispiel 35 werden folgende Verbindungen hergestellt:

a) Methyl-4'-[(R)-[[[(RS)-1-amino-3-piperidinyl]methyl]carbamoyl]-2-[(2-naphthylsulfonamido)äthyl]-succinanilat-hydrochlorid. FAB-MS: 623 $(M+H)^+$,

b) Methyl-3'-[(RS)-2-[[[(RS)-1-amidino-3-piperidinyl]methyl]carbamoyl]-2-(2-naphthylsulfonamido)äthyl]-succinanilat-hydrochlorid. FAB-MS: 623 $(M+H)^+$,

c) Benzyl-4'-[(R)-2-[[[(RS)-1-amidino-3-piperidinyl]methyl]carbamoyl]-2-[(2-naphthylsulfonamido)äthyl]-succinanilat-hydrochlorid FAB-MS: 699 $(M+H)^+$,

d) Methyl-4'-[(R)-2-[[[(RS)-1-amidino-3-piperidinyl]methyl]carbamoyl]-2-[(p-jodobenzolsulfonamido)äthyl]-succinanilat-hydrochlorid.FAB-MS: 699 $(M+H)^+$,

e) Aethyl-4'-[(R)-2-[[[(RS)-1-amidino-3-piperidinyl]methyl]carbamoyl]-2-(2-naphthylsulfonamido]äthyl]-carbanilat-hydrochlorid. FAB-MS: 581 $(M+H)^+$.

Beispiel 37

Analog Beispiel 35) erhält man das N-(2-Naphthylsulfonyl)-3-[p-(2-phenylglyoxylamido)phenyl]-D-alanin, MS: 502 $(M^+)$, welches analog zu Beispiel 6e)-g) zu (R)-N-[[(S)-1-Amidino-3-piperidinyl]methyl]-α-(2-naphthylsulfonyl)-p-(2-phenylglyoxylamido)hydrocinnamamid-hydrochlorid umgesetzt wird. FAB-MS: 641 $(M+H)^+$.

Beispiel 38

Analog Beispiel 35 und 42 erhält man via Methyl-p-[p-[(R)-2-[[(RS)-1-amidino-3-piperidinyl]methyl]-carbamoyl]-2-(2-naphthylsulfonamido)äthyl]benzolamido]benzoat-hydrochlorid, FAB-MS: 671 $(M+H)^+$, das p-[p-[(R)-2-[[[(RS)-1-Amidino-3-piperidinyl]methyl]carbamoyl]-2-(2-naphthylsulfonamido)äthyl]benzolamido]-benzoesäure-hydrochlorid. FAB-MS: 657 $(M+H)^+$.

Beispiel 39

Analog Beispiel 35 erhält man das (R)-N-[[(RS)-1-Amidino-3-piperidinyl]methyl]-p-[2-(2-methoxyäthoxy)-acetamido]-α-2-naphthylsulfonamidohydrocinnamamid-hydrochlorid. FAB-MS: 625 $(M+H)^+$.

Beispiel 40

Analog Beispiel 35 erhält man das 4'-[(R)-2-[[[(S)-1-Amidino-3-piperidinyl]methyl]carbamoyl]-2-(2-naphthylsulfonamido)äthyl]oxanilsäure-hydrochlorid. FAB-MS: 581 $(M+H)^+$.

Beispiel 41

Eine Lösung von 0,7 g 3-(p-Aminophenyl)-N-(2-naphthylsulfonyl)-D-alanin (Beispiel 35a) in 5 ml Pyridin wird mit 1 ml Essigsäureanhydrid versetzt und 20 Stunden bei Raumtemperatur stehen gelassen. Nach Zugabe von Wasser und konz. HCl (Eiskühlung) wird das ausgeschiedene Produkt abgesaugt, in Essigester gelöst, mit Wasser gewaschen, getrocknet und abgedampft. Das Produkt wird in 20 ml methanolischer 1N NaOH gelöst und stehen gelassen. Die Lösung wird abgedamptt, der Rückstand in Wasser gelöst, mit konz. HCl angesäuert, das ausgefallene Produkt abgesaugt, in Essigester gelöst und mit Wasser gewaschen. Der Rückstand wird nach Abdampfen analog Beispiel 5 umgesetzt und ergibt 146 mg (R)-p-Acetamido-N-[[(RS)-1-amidino-3-piperidinyl]methyl]-α-(2-naphthylsulfonamido)hydrocinnamamid-hydrochlorid. FAB-MS: 551 $(M+H)^+$.

Beispiel 42

Eine Suspension von 150 mg Aethyl-4'-[(R)-2-[[[(RS)-1-amidino-3-piperidinyl]methyl]carbamoyl]-2-(2-naphthylsulfonamido)äthyl]oxanilat-hydrochlorid (Beispiel 35) in 10 ml äthanolischer o,1N NaOH-Lösung wird bei Raumtemperatur unter Rühren gelöst. Die Reaktionslösung wird mit einer äthanolischen 2,78N HCl angesäuert, das ausgeschiedene NaCl abgesaugt und das Filtrat mit Aether versetzt. Das ausgeschiedene Hydrochlorid wird abdekantiert und mit Aether aufgerührt. Das Produkt wird abgesaugt. Ausbeute 60 mg 4'-[(R)-2-[[[(RS)-1-Amidino-3-piperidinyl]methyl]carbamoyl]-2-(2-naphthylsulfonamid)äthyl]oxanilsäure-hydrochlorid.FAB-MS: 581 $(M+H)^+$.

Beispiel 43

Eine Lösung von 0,5 g Benzyl-4'-[(R)-2-[[[(RS)-1-Amidino-3-piperidinyl]methyl]carbamoyl]-2-(2-naphthylsulfonamido)äthyl]succinanilat-hydrochlorid (Beispiel 36c) in 20 ml Aethanol wird mit 0,5 g 10% Pd/C versetzt und hydriert. Der Katalysator wird abfiltriert, das Filtrat konzentriert und mit Aether versetzt. Das ausgeschiedene Produkt wird abdekantiert, mit Aether aufgerührt und schliesslich abgenutscht. Ausbeute: 0,35 g 4'-[(R)-2-[[[(RS)-1-Amidino-3-piperidinyl]methyl]carbamoyl]-2-(2-naphthylsulfonamido)äthyl]-succinanilidsäure-hydrochlorid. FAB-MS: 609 $(M+H)^+$.

Beispiel 44

Eine Lösung von 0,5 g 3-(p-Aminophenyl)-N-(2-naphthylsulfonyl)-D-alanin (Beispiel 35a) in 1,35 ml 1N NaOH und 7 ml Wasser wird mit 6 ml Dioxan und 0,34 g Natriumhydrogencarbonat versetzt. Unter Rühren werden 0,26 g Tosylchlorid zugegeben. Nach 24 Stunden Rühren bei Raumtemperatur wird die Lösung konzentriert, mit Wasser verdünnt und mit Aether extrahiert. Die wässrige Phase wird unter Eiskühlung mit 6 ml 2N HCl versetzt und das ausgeschiedene Produkt abgenutscht. Letzteres wird ohne Reinigung analog Beispiel 5 in 0,39 g (R)-N-[[(RS)-1-Amidino-3-piperidinyl]methyl]-α-2-naphthylsulfonamido-p-(p-toluolsulfonamido)hydrocinnamamid-hydrochlorid übergeführt. FAB-MS: 663 $(M+H)^+$.

Beispiel 45

Analog Beispiel 44 erhält man das (R)-N-[[(S)-1-Amidino-3-piperidinyl]methyl]-p-(p-jodobenzolsulfonamido)-α-(2-naphthylsulfonamido)hydrocinnamamid-hydrochlorid. FAB-MS: 775 $(M+H)^+$.

Beispiel 46

Analog Beispiel 44 erhält man via Methyl-p-[[p-[(R)-2-[[[(RS)-1-Amidino-3-piperidinyl]methyl]carbamoyl]-2-(2-naphthylsulfonamido)äthyl]phenyl]sulfamoyl]benzoat-hydrochlorid. FAB-MS: 707 $(M+H)^+$, das p-[[p-[(R)-2-[[[(RS)-1-Amidino-3-piperidinyl]methyl]carbamoyl]-2-(2-naphthylsulfonamido)äthyl]phenyl]sulfamoyl]-benzoesäure-hydrochlorid FAB-MS: 693 $(M+H)^+$.

Beispiel 47

Einer Suspension von 0,37 g 3-(p-Aminophenyl)-N-(2-naphthylsulfonyl)-D-alanin (Beispiel 35a) in 20 ml Dioxan werden 0,45 ml einer 50%igen Lösung von Aethylglyoxylat in Toluol zugegeben. Die entstandene Lösung wird mit 0,3 g 10% Pd/C versetzt und 6 Stunden bei Raumtemperatur und Normaldruck hydriert. Der Katalysator wird abfiltriert, das Filtrat abgedampft und der Rückstand wie in Beispiel 5 umgesetzt. Ausbeute: 89 mg N-[p-[(R)-2-[[[(RS)-1-Amidino-3-piperidinyl]methyl]carbamoyl]-2-(2-naphthylsulfonamido)-äthyl]phenyl]glycinäthylester-hydrochlorid. FAB-MS: 595 $(M+H)^+$.

Beispiel 48

Analog Beispiel 26 erhält man das (R)-N-[[(RS)-1-Amidino-3-piperidinyl]methyl]-1,2,3,4-tetrahydro-2-(2-naphthylsulfonyl)-3-isochinolincarboxamid-hydrochlorid (Epimere 1:1),

$$[\alpha]^{25}_{589} = +76{,}8°$$

(MeOH, c = 0,5%), FAB-MS: 506 $(M+H)^+$.

Beispiel 49

Analog Beispiel 5 erhält man das (RS)-N-[[(RS)-1-Amidino-3-piperidinyl]methyl]-3-(o-aminobenzoyl)-2-(2-naphthylsulfonamido)propionamid-hemisulfit. FAB-MS: 537 $(M+H)^+$.

Beispiel 50

Analog Beispiel 23 stellt man folgende Verbindungen her:

a) (S)-N-[[(RS)-1-Amidino-3-piperidinyl]methyl]-β-2-naphthylsulfonamido-γ-oxo-4-morpholinbutyramid-trifluoracetat. FAB-MS: 531 $(M+H)^+$.

b) (R)-N-[[(RS)-1-Amidino-3-piperidinyl]methyl]hexahydro-β-2-naphthylsulfonamido-γ-oxo-1H-azepinbutyramid-trifluoracetat. FAB-MS: 543 $(M+H)^+$.

c) (S)-N-[[(RS)-1-Amidino-3-piperidinyl]methyl]hexahydro-β-2-naphthylsulfonamido-γ-oxo-1(2H)-azocinbutyramid-hydrochlorid. FAB-MS: 557 $(M+H)^+$.

Beispiel 51

a) 5 g Diphenylsulfon-3-sulfonsäure-kaliumsalz werden in 50 ml Thionylchlorid über Nacht unter Rückfluss gekocht. Das ausgefallene Material wird abfiltriert und man erhält nach Trocknen 3,2 g Diphenylsulfon-3-sulfonsäurechlorid.

b) Zu einer Lösung von 1,52 g p-Nitro-D-phenylalanin in 37 ml Dioxan werden 6,7 ml 1N Natronlauge und 2,82 g Natriumbicarbonat gegeben. Dazu gibt man eine Lösung des Produkts von a) in 119 ml Dioxan und rührt über Nacht bei Raumtemperatur. Anschliessend wird das Reaktionsgemisch auf Eis gegossen und mit Essigester extrahiert. Die organische Phase wird mit Wasser gewaschen, getrocknet und eingeengt. Man erhält so 1,75 g 3-(p-Nitrophenyl)-N-[[(m-phenylsulfonyl)phenyl]sulfonyl]-D-alanin.

c) Aus obiger Säure erhält man analog Beispiel 6 e)-g) (R)-N-[[(S)-1-Amidino-3-piperidinyl]methyl]-p-nitro-α-[m-(phenylsulfonyl)benzolsulfonamido]hydrocinnamamid-hemisulfit, FAB-MA: 629 $(M+H)^+$.

Beispiel 52

a) 2,3 g p-Nitro-D-phenylalanin werden in 25 ml Dioxan suspendiert und mit 10 ml 1N Natronlauge und 1,7 g Natriumbicarbonat versetzt. Dazu tropft man eine Lösung von 2,6 g 2-Chlorsulfonylbenzoesäuremethylester in 22 ml Dioxan und rührt während 9 Stunden bei Raumtemperatur. Das Reaktionsgemisch wird auf eiskalte 2N Salzsäure gegossen und mit Essigester extrahiert. Die organische Phase wird mit Wasser gewaschen, getrocknet und eingedampft. Man erhält 3,2 g N-[[o-(Methoxycarbonyl)phenyl]-sulfonyl]-3-(p-nitrophenyl)-D-alanin.

b) Das aus a) erhaltene Material wird in 30 ml DMF gelöst, mit 5,3 ml Hexamethylenimin versetzt und 5 Stunden bei 80 °C gerührt. Das Reaktionsgemisch wird eingedampft, der Rückstand in Essigester aufgenommen und einmal gegen 2N Salzsäure und zweimal gegen Wasser geschüttelt. Nach Trocknen der organischen Phase, Eindampfen und Chromatographie des Rückstands an Kieselgel erhält man 0,9 g N-[[o-[(Hexahydro-1H-azepin-1-yl)carbonyl]phenyl]sulfonyl]-3-(p-nitrophenyl)-D-alanin.

c) Analog Beispiel 5 erhält man aus unter b) erhaltenem Material (R)-N-[[(RS)-1-Amidino-3-piperidinyl]-methyl]-α-[o-[(hexahydro-1H-azepin-1-yl)carbonyl]benzolsulfonamido]-p-nitrohydrocinnamamid-hydrochlorid. FAB-MS: 614 $(M+H)^+$.

Beispiel 53

Analog Beispiel 52 stellt man folgende Verbindungen her:

a) (R)-N-[[(RS)-1-Amidino-3-piperidinyl]methyl]-α-(p-t-butylbenzolsulfonamido)-p-nitrohydrocinnamamid-acetat. FAB-MS: 545 $(M+H)^+$.

b) (R)-N-[[(RS)-1-Amidino-3-piperidinyl]methyl]p-nitro-α-(benzo[b]thiophensulfonamido)hydrocinnamid-hydrochlorid. FAB-MS: 545 $(M+H)^+$.

Beispiel 54

Analog Beispiel 19a) und 23 erhält man das (βS,2RS)-N-[[(RS)-1-Amidino-3-piperidinyl]methyl]-2-benzyloxymethyl-β-2-naphthylsulfonamido-γ-oxo-4-morpholinbutyramid-acetat. FAB-MS: 651 $(M+H)^+$.

Beispiel 55

Eine Lösung des Produkts von Beispiel 54 in Aethanol/1N Salzsäure wird in Gegenwart von 10% Pd/C während 30 Stunden unter Normalbedingungen hydriert. Man erhält (βS,2RS)-N-[[(RS)-1-Amidino-3-piperidinyl]methyl]-2-hydroxymethyl-β-2-naphthylsulfonamido-γ-oxo-4-morpholinbutyramid-hydrochlorid. FAB-MS:

561 $(M+H)^+$.

Beispiel 56

A)a) Eine Lösung von 23,3 g rac-2-(Aminomethyl)-4-benzylmorpholin in 250 ml Dioxan wird mit 27,1 g Di-t-butyldicarbonat in 250 ml Dioxan versetzt und 17 Stunden bei Raumtemperatur gerührt. Dann wird das Lösungsmittel abgedampft und der Rückstand mit Methylenchlorid-Essigester 3:1 an Kieselgel chromatographiert. Das Produkt wird aus Methylenchlorid-Hexan umkristallisiert und man erhält 25,6 g t-Butyl-rac-[(4-benzyl-2-morpholinyl)methyl]carbamat.

A)b) Eine Lösung des Produkts von a) in 500 ml Essigester und 50 ml Essigsäure wird mit 2,6 g Pd/C versetzt und während 5 Stunden bei Raumtemperatur unter Nomalbedingungen hydriert. Nach Filtration und Eindampfen wird der Rückstand in 230 ml DMF gelöst, mit 46 ml Triäthylamin und 10,8 g Formamidinsulfonsäure versetzt und 20 Stunden bei Raumtemperatur gerührt. Anschliessend wird das Reaktionsgemisch eingedampft und der Rückstand zwischen Essigester und Wasser verteilt. Nach Trocknen der organischen Phase und Eindampfen erhält man das t-Butyl-rac-[(4-amidino-2-morpholinyl)-methyl]carbamat-hemisulfit.

A)c) 6,5 g des unter b) erhaltenen Materials werden in 50 ml Methylenchlorid suspendiert und bei 0° mit 20 ml TFA versetzt. Nach 7 Stunden bei 0° wird das Reaktionsgemisch eingedampft und mit Aethylenchlorid und Toluol azeotropiert. Man isoliert rac-2-(Aminomethyl)-4-morpholincarboxamidintrifluoracetat.

B) Eine Lösung von 0,8 g analog Beispiel 23 a)-c) erhaltener (S)-β-2-Naphthylsulfonamido-γ-oxo-4-morpholinbuttersäure in 16 ml DME werden mit 0,76 ml 4-Aethylmorpholin, 0,89 g Benzotriazol-1-yloxy-tris(dimethylamino)phosphonium-hexafluorphosphat und 1,16 g des unter A)c) erhaltenen Materials versetzt und während 17 Stunden bei Raumtemperatur gerührt. Anschliessend wird das Reaktionsgemisch mit 1N Salzsäure versetzt und eingedampft. Nach Chromatographie an einer RP-18 Säule mit Wasser-Acetonitril erhält man 0,5 g (S)-N-[[(RS)-4-Amidino-2-morpholinyl]methyl]-β-2-naphthylsulfonamido-γ-oxo-morpholinbutyramid-hydrochlorid FAB-MS: 533 $(M+H)^+$.

Beispiel 57

Analog Beispiel 56 erhält man:

a) (S)-N-4-[[(RS)-4-Amidino-2-morpholinyl]methyl]-β-2-naphthylsulfonamidohexahydro-γ-oxo-1H-azepin-1-butyramid-hydrochlorid FAB-MS: 545 $(M+H)^+$.

b) (S)-N-4-[[(RS)-4-Amidino-2-morpholinyl]methyl]hexahydro-β-2-naphthylsulfonamido-γ-oxo-1(2H)-azocin-1-butyramid-hydrochlorid. FAB-MS: 559 $(M+H)^+$.

Beispiel 58

Analog Beispiel 8 erhält man das (R)-N-[[(RS)-1-Amidino-3-piperidinyl]methyl]-α-(2-naphthylsulfonamido)-2-oxo-3-benzoxazolinpropionamid-triacetat. FAB-MS: 551 $(M+H)^+$.

Beispiel 59

Analog Beispiel 8 und 56 stellt man folgende Verbindungen her:

a) (R)-N-[[(RS)-4-Amidino-2-morpholinyl]methyl]-α-2-naphthylsulfonamido-2,3-dioxo-1-indolinpropionamid-hydrochlorid. FAB-MS: 565 $(M+H)^+$.

b) (R)-N-[[(RS)-4-Amidino-2-morpholinyl]methyl]-α-2-naphthylsulfonamido-2-oxo-3-benzoxazolinpropionamid-hydrochlorid. FAB-MS: 553 $(M+H)^+$.

Beispiel 60

A)a) Zu einer Lösung von 10,0 g (S)-3-Aminomethyl-1-piperidincarbonsäure-t-butylester (Beispiel 6d) in 400 ml Hexan und 100 ml Wasser gibt man 3,7 g Tetrabutylammoniumhydrogensulfat und 100 ml 1N Natronlauge. Zu dieser Mischung tropft man 9,3 ml Benzylchloroformiat und rührt während 3 Stunden bei Raumtemperatur. Anschliessend wird die organische Phase abgetrennt, mit Wasser, 10%iger Zitronensäure, Wasser und gesättigter Natriumbicarbonatlösung gewaschen, getrocknet und eingedampft. Man erhält t-Butyl-(S)-3-[(1-(benzyloxy)formamido]methyl]-1-piperidincarboxylat.

A)b) 11,3 g des unter a) erhaltenen Materials werden in 120 ml Essigester gelöst, bei 4° mit 120 ml einer 4 molaren Lösung von Salzsäure in Essigester versetzt und 5 Stunden bei Raumtemperatur gerührt. Anschliessend wird die Reaktionslösung eingeengt, der Rückstand in 265 ml DMF gelöst, mit 18 ml Triäthylamin und 4,3 g Formamidinsulfonsäure versetzt und 17 Stunden bei Raumtemperatur gerührt. Das Lösungsmittel wird eingedampft, der Rückstand mit 1N Salzsäure versetzt, wiederum eingeengt und mit Wasser-Acetonitril an einer RP-18 Säule chromatographiert. Man isoliert so 5,4 g Benzyl-[[(S)-1-amidino-3-piperidinyl]methyl]carbamat-hydrochlorid.

A)c) 6,6 g des unter b) erhaltenen Materials werden in 165 ml Aethanol und 165 ml 1N Salzsäure gelöst, mit 1 g Pd/C versetzt und während 2 Stunden unter Normalbedingungen hydriert. Nach Filtration, Eindampfen und Azeotropieren mit Aethanol erhält man 4,5 g (S)-1-Amidino-3-(aminomethyl)piperidin-dihydrochlorid, Smp. 252-254°C, FAB-MS: 156 ($M^+$), $[\alpha]_D$ -16,9° (c = 1,0, Wasser).

B) Eine Lösung von 1,9 g N-(2-Naphthylsulfonyl)-3-(2,3-dioxo-1-indolinyl)-D-alanin in 30 ml DMF wird mit 2,3 ml 4-Aethylmorpholin, 2,0 g Benzotriazol-1-yloxy-tris(dimethylamino)phosphonium-hexafluorphosphat und 1,0 g des unter A)c) erhaltenen Materials versetzt und während 20 Stunden bei Raumtemperatur gerührt. Das Reaktionsgemisch wird eingeengt, mit 1N Salzsäure versetzt, nochmals eingedampft und der Rückstand mit Essigester-Aceton-Eisessig-Wasser 16:2:1:1 an Kieselgel chromatographiert. Man isoliert 0,8 g (R)-N-[(S)-1-Amidino-3-piperidinylmethyl]-α-(2-naphthylsulfonamido)-2,3-dioxo-1-indolinpropionamid-acetat. FAB-MS: 563 $(M+H)^+$.

## Beispiel 61

Analog Beispiel 8 und 60 erhält man folgende Verbindungen:

a) (R)-N-[[(S)-1-Amidino-3-piperidinyl]methyl]-α-2-naphthylsulfonamido-2-oxo-3-benzoxazolidinpropionamid-hydrochlorid. FAB-MS: 551 $(M+H)^+$,

b) (R)-N-[[(S)-1-Amidino-3-piperidinyl]methyl]-5-brom-α-2-naphthylsulfonamido-2,3-dioxo-1-indolinpropionamid-tetraacetat. FAB-MS: 643 $(M+H)^+$,

c) (R)-N-[[(S)-1-Amidino-3-piperidinyl]methyl]-5-methyl-α-2-naphthylsulfonamido-2,3-dioxo-1-indolinpropionamid-acetat. FAB-MS: 577 $(M+H)^+$,

d) (R)-N-[[(S)-1-Amidino-3-piperidinyl]methyl]-α-[(p-jodphenyl)sulfonyl]-2,3-dioxo-1-indolinpropionamid-hydrochlorid. FAB-MS: 639 $(M+H)^+$,

e) (R)-N-[[(S)-1-Amidino-3-piperidinyl]methyl]-α-(o-nitrobenzolsulfonamido)-2,3-dioxo-1-indolinpropionamid-hydrochlorid. FAB-MS: 558 $(M+H)^+$.

## Beispiel 62

a) Einer Lösung von 15 g N-α-Z-L-2,3-Diaminopropionsäure in 189 ml 1N Natronlauge wird unter Rühren eine Lösung von 21,4 g 2-Naphthylsulfochlorid in 200 ml Aether zugetropft. Man lässt weitere 6 Stunden rühren. Anschliessend wird das Reaktionsgemisch auf eiskalte 2N Salzsäure gegossen und mit Essigester extrahiert. Die organische Phase wird mit Wasser gewaschen, getrocknet und eingedampft. Nach Chromatographie des Rückstands mit Methylenchlorid-Methanol-Essigsäure 94:5:1 an Kieselgel isoliert man 17,9 g N-[(Benzyloxy)carbonyl]-3-(2-naphthylsulfonamido)-1-alanin.

b) Aus unter a) erhaltenem Material wird analog Beispiel 5 Benzyl-[(S)-1-[[[(RS)-1-amidino-3-piperidinyl]-methyl]carbamoyl-2-(2-naphthylsulfonamido)äthylcarbamat hergestellt, FAB-MS: 567 $(M+H)^+$.

## Beispiel 63

a) Eine Lösung von 1,1 g des Produkts von Beispiel 62b) in 22 ml 1N Salzsäure und 11 ml Aethanol wird mit 0,2 g Pd/C versetzt und während 5 Stunden unter Normalbedingungen hydriert. Nach Filtration und Eindampfen erhält man 0,96 g (S)-[[(RS)-1-Amidino-3-piperidinyl]methyl]-2-amino-3-(2-naphthylsulfonamido)propionamid-dihydrochlorid, FAB-MS: 433 $(M+H)^+$.

b) Eine Lösung von 0,95 g des unter a) erhaltenen Materials in 20 ml DMF wird mit 0,24 ml 4-Aethylmorpholin und 0,3 g Phthalsäureanhydrid versetzt und während 6 Stunden bei 50° gerührt. Das Reaktionsgemisch wird eingedampft und mit Wasser-Acetonitril über eine RP-18 Säule chromatographiert. Man erhält 0,3 g o-[[(S)-1-[[[(RS)-1-Amidino-3-piperidinyl]methyl]carbamoyl]-2-(2-naphthylsulfonamido)äthyl]carbamoyl]-benzoesäure, FAB-MS: 581 $(M+H)^+$.

Beispiel 64

Eine Lösung von 1,4 g des Produkts von Beispiel 63a) in 28 ml DMF wird mit 1,05 ml 4-Aethylmorpholin, 1,23 g Benzotriazol-1-yloxy-tris(dimethylamino)phosphonium-hexafluorphosphat und 0,95 g o-(Benzyloxy)benzoesäure versetzt und über Nacht bei Raumtemperatur gerührt. Das Reaktionsgemisch wird eingedampft und der Rückstand zwischen Essigester und Wasser verteilt. Die organischen Phasen werden getrocknet, eingedampft und der Rückstand mit Essigester-Aceton-Eisessig-Wasser 16:2:1:1 an Kieselgel chromatographiert. Man isoliert 0,2 g (S)-2-N-[o-(Benzyloxy)benzamido]-3-(2-naphthylsulfonamido)-N-[[(RS)-1-amidino-3-piperidinyl]methyl]propionamid-acetat. FAB-MS: 643 $(M+H)^+$.

Beispiel 65

a) Analog Beispiel 64 erhält man mittels t-Butyl-(R)-2-piperidincarbonsäurean Stelle von o-(Benzyloxy)-benzoesäure: t-Butyl-(R)-2-[[(S)-1-[[[(RS)-1-amidino-3-piperidyl]methyl]carbamoyl]-2-(2-naphthylsulfonamido)äthyl]carbamoyl]-1-piperidincarboxylat-trifluoracetat, FAB-MS: 644 $(M+H)^+$.

b) Eine Lösung von 300 mg des Produkts von a) in 5 ml Essigester wird mit 5 ml einer 4 molaren Salzsäurelösung in Essigester versetzt. Nach 3 Stunden Rühren bei Raumtemperatur wird die Suspension eingedampft und man isoliert (R)-N-(S)-1-[[(RS)-1-Amidino-3-piperidinyl]methyl]carbamoyl]-2-(2-naphthylsulfonamido)-2-piperidincarboxamid-dihydrochlorid, FAB-MS: 544 $(M+H)^+$.

Beispiel 66

Eine Lösung von 120 mg des Produkts von Beispiel 64 in 12 ml Essigsäure wird mit 50 mg Pd/C versetzt und während 6 Stunden unter Normalbedingungen hydriert. Nach Filtration und Eindampfen erhält man 80 mg (S)-N-[[(RS)-1-Amidino-3-piperidinyl]methyl]-2-(2-hydroxybenzamido)-3-(2-naphthylsulfonamido)-propionamid-acetat. FAB-MS: 553 $(M+H)^+$.

Beispiel 67

a) Das nach Beispiel 62a) erhaltene Material wird analog Beispiel 6a)-e) umgesetzt. Man erhält t-Butyl-(S)-3-[[[N-[(benzyloxy)carbonyl]-3-(2-naphthylsulfonamido)-1-alanyl]amino]methyl]-1-piperidincarboxylat, FAB-MS: 525 $(M+H)^+$Boc.

b) Eine Lösung von 3,0 g des nach a) erhaltenen Materials in 80 ml Essigester-Essigsäure (1:1) wird mit 0,8 g Pd/C versetzt und 48 Stunden unter Normalbedingungen hydriert. Nach Filtration und Eindampfen löst man den Rückstand in 25 ml DMF, gibt 1,6 ml 4-Aethylmorpholin und 0,81 g Isatosäureanhydrid zu und rührt während 16 Stunden bei 80°. Das Lösungsmittel wird abgedampft und der Rückstand zwischen Essigester und Wasser verteilt. Die organische Phase wird mit Wasser gewaschen, getrocknet, eingedampft und der Rückstand mit Methylenchlorid-Essigester 3:1 an Kieselgel chromatographiert. Man isoliert 1,3 g t-Butyl-(S)-3-[[N-anthraniloyl-3-(2-naphthylsulfonyl)-L-alanyl]amino]methyl]-1-piperidincarboxylat, FAB-MS: 610 $(M+H)^+$.

c) Aus nach b) isoliertem Material erhält man analog Beispiel 6f)-g) N-[(S)-1-[[[(S)-1-Amidino-3-piperidinyl]methyl]carbamoyl]-2-(2-naphthylsulfonamido)äthyl]-o-aminobenzamid-hemisulfit. FAB-MS: 552 $(M+H)^+$.

Beispiel 68

Analog Beispiel 23a)-c) und Beispiel 60B) erhält man mit Aethyl-rac-trans-4-methyl-2-piperidincarboxylat anstelle von Benzylamin das Aethyl-(2RS,4R)-1-[(S)-3-[[[(S)-1-amidino-3-piperidinyl]methyl]carbamoyl]-2-(2-naphthylsulfonamido)propionyl]-4-methyl-2-piperidincarboxylat-acetat (Epimere 1:1), FAB-MS: 615 $(M+H)^+$.

Beispiel 69

Durch Behandeln des Produkts von Beispiel 68 mit methanolischer Natronlauge erhält man die (2RS,4R)-1-[$N^4$-[[(S)-1-Amidino-3-piperidinyl]methyl]-$N^2$-(2-naphthylsulfonyl)-L-asparaginyl]-4-methyl-2-piperidincarbonsäure (Epimere 1:1), FAB-MS: 587 $(M+H)^+$.

Beispiel 70

a) Einer auf 10 °C abgekühlten Lösung von 5 g D-Asparaginsäure-β-benzylester und 5,07 g β-Naphthylsulfonylchlorid in 80 ml Dioxan werden bei 10 °C 22,4 ml 2N NaOH zugetropft. Das Reaktionsgemisch rührt man anschliessend 2 Stunden bei Raumtemperatur und versetzt es anschliessend mit 25 ml 1N Salzsäure. Nach Abdampfen des Dioxans nimmt man den Rückstand in Essigester auf und wäscht mit Wasser. Nach Trocknen und Eindampfen der Essigesterphase erhält man 9,1 g 4-Benzyl-1-hydrogen-N-(2-naphthylsulfonyl)-D-aspartat. Rf = 0,53 (Essigester/Eisessig 0,97:0,03).
b) Dem Produkt von a) in 100 ml DMF gibt man unter Rühren nacheinander 3,72 ml Hünigbase, 9,67 g Benzotriazol-1-yl-oxy-tris(dimethylamino)phosphonium-hexafluorphosphat und 4,68 g (S)-3-Aminomethyl-1-piperidincarbonsäure-t-butylester zu. Das Reaktionsgemisch rührt man 4 Stunden, nimmt es anschliessend in Aether auf und wäscht die Aetherphasen mit Wasser. Nach Trocknen und Eindampfen der Aetherphase wird der Rückstand über Kieselgel mit Aether/Hexan 9:1 chromatographiert. Man erhält 9,1 g Benzyl-(R)-3-[[[(S)-1-(t-butoxycarbonyl)-3-piperidinyl]methyl]carbamoyl]-3-(2-naphthylsulfonamido)-propionat. Rf = 0,35 (Aether/Hexan 9:1).
c) 3,0 g des Produkts von b) löst man in 60 ml Methanol und hydriert nach Zugabe von 10% Pd/C bei Raumtemperatur unter Normaldruck. Nach 8 Stunden filtriert man den Katalysator ab und engt die Methanollösung ein. Man erhält 2,38 g (R)-3-[[[(S)-1-(t-Butoxycarbonyl)-3-piperidinyl]methyl]carbamoyl]-3-(2-naphthylsulfonamido)propionsäure, Rf = 0,08 (Essigester).
d) 1 g Carbonsäure aus c) in 18 ml Methylenchlorid kühlt man auf -23 °C ab und gibt nacheinander 0,25 ml N-Methylmorpholin und 0,3 ml Chlorameisensäure-isobutylester zu. Dann rührt man das Reaktionsgemisch bei -23 °C und versetzt es mit 0,36 ml Piperidin-3-carbonsäure-äthylester. Das Reaktionsgemisch nimmt man anschliessend in 100 ml Aether auf. Die Aetherlösung wäscht man mit 1N Salzsäure und mit Wasser. Nach Trocknen und Eindampfen der Aetherphase wird der Rückstand über Kieselgel mit Essigester/Hexan 4:1 chromatographiert. Man erhält 917 mg Aethyl-(R,S)-1-[(R)-3-[[[(S)-1-(t-butoxycarbonyl)-3-piperidinyl]methyl]carbamoyl]-3-(2-naphthylsulfonamido)propionyl]-3-piperidincarboxylat. Rf = 0,4 (Essigester/Hexan 4:1).
e) Zu einer Lösung von 400 mg des Produkts aus d) in 9,5 ml Methylenchlorid gibt man unter Rühren 1,2 ml Trifluoressigsäure. Das Methylenchlorid und die Trifluoressigsäure werden anschliessend bei 50 °C abgedampft. Den Rückstand löst man in Methanol, versetzt die Methanollösung mit 0,42 ml Triäthylamin und 150 mg Formamidinsulfonsäure. Das Reaktionsgemisch wird anschliessend 8 Stunden bei Raumtemperatur gerührt. In Abständen von 2 Stunden gibt man noch dreimal je 0,09 ml Triäthylamin und 75 mg Formamidinsulfonsäure zu. Das Reaktionsgemisch engt man ein, schlämmt den Rückstand in 20 ml Takeda-Lösung/Essigester 1:1 auf (Takeda-Lösung = Essigester/Aceton/Wasser/Eisessig 6:2:1:1) und filtriert ab. Das Filtrat wird eingeengt und anschliessend über Kieselgel mit Takeda-Lösung/Essigester 1:1 chromatographiert. Aus der Chromatographie erhält man 114 mg Aethyl-(RS)-1-[(R)-[[(S)-1-amidino-3-piperidinyl]methyl]carbamoyl]-3-(2-naphthylsulfonamido)propionyl]-3-piperidincarboxylat-acetat. Rf = 0,34 MS (EI): 601 (M + H).

Beispiel 71

Analog Beispiel 70 werden folgende Verbindungen hergestellt:
a) [(R)-1-[(S)-3-[[(S)-1-Amidino-3-piperidinyl]methyl]carbamoyl]-3-(2-naphthylsulfonamido)propionyl]-3-piperidinyl]methyl-acetat, Rf = 0,61 (Takeda-Lösung), FAB-MS: 601 (M + 1).
b) [(S)-1-[(S)-3-[[(S)-1-Amidino-3-piperidinyl]methyl]carbamoyl]-3-(2-naphthylsulfonamido)propionyl]-3-piperidinyl]methyl-p-tosylat (1:1), Rf = 0,29 (Takeda-Lösung), FAB-MS: 601 (M + 1).
c) Methyl-(RS)-1-[(R)-3-[[[(S)-1-amidino-3-piperidinyl]methyl]carbamoyl]-3-(2-naphthylsulfonamido)-propionyl]-4-oxo-3-piperidincarboxylat-acetat (1:1), Rf = 0,27 (Takeda-Lösung), FAB-MS: 601 (M + 1).
d) [(S)-1-[(R)-3-[[(S)-1-Amidino-3-piperidinyl]methyl]carbamoyl]-(2-naphthylsulfonamido)acetyl]-3-piperidinyl]methylacetat-acetat, Rf = 0,29 (Takeda-Lösung), FAB-MS: 601 (M + 1).
e) [(R)-1-[(R)-3-[[(S)-1-Amidino-3-piperidinyl]methyl]carbamoyl]-(2-naphthylsulfonamido)acetyl]-3-piperidinyl]methylacetat-acetat, Rf = 0,35 (Takeda-Lösung), FAB-MS: 601 (M + 1).
f) Diäthyl-(RS)-1-[(R)-[[(S)-1-amidino-3-piperidinyl]methyl]carbamoyl]-3-(2-naphthylsulfonamido)-propionyl]-3-piperidincarboxamid-acetat, Rf = 0,3 (Takeda-Lösung), FAB-MS: 628 (M + 1).
g) [(S)-1-[(S)-3-[[[(S)-1-Amidino-3-piperidinyl]methyl]carbamoyl]-3-(2-naphthylsulfonamido)propionyl]-3-piperidinyl]methylisobutyrat-acetat, Rf = 0,43 (Takeda-Lösung), MS (EI): 629 (M + 1).
h) [(S)-1-[(S)-3-[[[(S)-1-Amidino-3-piperidinyl]methyl]carbamoyl]-3-(2-naphthylsulfonamido)propionyl]-3-piperidinyl]methylbutyrat-acetat, Rf = 0,47 (Takeda-Lösung), FAB-MS: 629 (M + 1).

i) Aethyl-(3R,4R)-4-acetoxy-1-[(R)-3-[[[(S)1-amidino-3-piperidinyl]methyl]carbamoyl]-3-(2-naphthylsulfonamido)propionyl]-3-piperidincarboxylat-acetat (1:1), Rf = 0,21 (Takeda-Lösung), FAB-MS: 659 (M + 1).

j) Aethyl-(3S,4S)-4-acetoxy-1-[(R)-3-[[[(S)-1-amidino-3-piperidinyl]methyl]carbamoyl]-3-(2-naphthylsulfonamido)propionyl]-3-piperidincarboxylat-acetat (1:1), Rf = 0,24 (Takeda-Lösung), FAB-MS: 659 (M + 1).

Für die Herstellung der Produkte a), b), g) und h) wird an Stelle von D-Asparaginsäure-β-benzylester (Beispiel 70a)) der S-Asparaginsäure-β-benzylester verwendet.

Die Zwischenprodukte für die Herstellung der Produkte a), b), d), e), i) und j) werden nach folgender Methode synthetisiert:

2,0 g (S)-3-Hydroxymethyl-1-piperidincarbonsäure-t-butylester bzw. (R)-3-Hydroxymethyl-1-piperidincarbonsäure-t-butylester werden zusammen mit 0,88 ml Essigsäureanhydrid, 26,3 mg Dimethylaminopyridin und 3,0 ml Pyridin 30 Minuten gerührt. Das Reaktionsgemisch nimmt man anschliessend in Aether auf, wäscht die Aetherphase mit 20%iger Zitronensäure, gesättigter Natriumbicarbonatlösung und Wasser. Nach Trocknen und Eindampfen der Aetherphase werden die 2,44 g Produkt Rf = 0,85 (Aether) in 50 ml Methylenchlorid gelöst und mit 12 ml Trifluoressigsäure versetzt. Man rührt die Reaktionslösung 30 Minuten und engt anschliessend zur Trockene ein. Das (S)- bzw. (R)-3-Acetoxymethyl-1-piperidin-trifluoressigsäuresalz Rf = 0,14 (Takeda-Lösung) wird mit äquivalenter Menge Triäthylamin in d) eingesetzt.

Die Zwischenprodukte für die Synthese der Produkte der Beispiele g) und h) werden wie folgt hergestellt:

Zu 3,0 g (S)-3-Hydroxymethyl-1-piperidincarbonsäure-t-butylester gibt man 170 mg Dimethylaminopyridin und 3,4 ml Pyridin. Dazu tropft man 1,66 ml Isobuttersäurechlorid. Das Reaktionsgemisch engt man dann ein. Den Rückstand nimmt man in Aether auf und wäscht die Aetherphase nacheinander mit 20%iger Zitronensäure, Wasser, Natriumbicarbonatlösung und nochmals mit Wasser. Nach Trocknen und Eindampfen der Aetherphase erhält man 3,9 g (S)-3-Isobutyroximethyl-1-piperidincarbonsäure-t-butylester. Rf = 0,87 (Aether). 3,09 g dieses Esters werden in 80 ml Methylenchlorid gelöst und mit 20 ml Trifluoressigsäure versetzt. Nach 30 Minuten Rühren engt man die Lösung ein. Zum Rückstand gibt man 30 ml Methylenchlorid und tropft gesättigte Natriumbicarbonatlösung zu. Die wässrige Phase extrahiert man mit Methylenchlorid, trocknet die Methylenchloridextrakte und engt sie ein. Man erhält 2,82 g (S)-3-Isobutyroxymethyl-1-piperidin-trifluoressigsäuresalz. Rf = 0,4 (Takeda-Lösung).

Das (S)-3-Butyroximethyl-1-piperidin-trifluoressigsäuresalz erhält man nach der gleichen Methode. Rf = 0,4 (Takeda-Lösung).

<u>Beispiel 72</u>

a) Zu 6,91 g Glycin-t-butylester-hydrochlorid und 12,1 g Naphthalin-2-sulfochlorid in 70 ml Methylenchlorid werden 14,4 ml Triäthylamin getropft. Das Reaktionsgemisch verdünnt man anschliessend mit 200 ml Aether. Die organische Phase wäscht man mit 1N Salzsäure und mit Wasser. Nach Trocknen und Eindampfen wird der Rückstand in Aether aufgeschlämmt und genutscht. Nach Trocknen der Kristalle erhält man 11,57 g N-(2-Naphthylsulfonyl)-glycin-t-butylester. Rf = 0,49 (Aether/Hexan 2:1).

b) 1 g des Produkts aus a), 923 mg Dansylchlorid, 0,48 ml Triäthylamin und 418 mg Dimethylaminopyridin werden zusammen in 10 ml Methylenchlorid gerührt. Das Reaktionsgemisch nimmt man in 100 ml Aether auf, wäscht mit 1N Salzsäure und mit Wasser. Nach Trocknen und Eindampfen werden 1,67 g N-[[5-(Dimethylamino)-1-naphthyl]sulfonyl]-N-(2-naphthylsulfonyl)glycin erhalten. Rf = 0,33 (Methylenchlorid/n-Hexan 9:1).

c) Einer Lösung des Produkts von b), in 17 ml Methylenchlorid, leitet man bei 0 bis 5 °C Salzsäuregas durch. Nach Einengen der Reaktionslösung wird 1,7g Carbonsäure erhalten. Rf = 0,72 (Essigester/Eisessig 97:3). Einer Lösung dieses Produkts in 17 ml Methylenchlorid werden bei Raumtemperatur 844 mg Dicyclohexylcarbodiimid (DCC) zugegeben. Dann gibt man 877 mg (S)-3-Aminomethyl-1-piperidin-carbonsäure-t-butylester, gelöst in 3 ml Methylenchlorid zu. Man filtriert das Reaktionsgemisch, dampft das Filtrat ein und chromatographiert den Rückstand über Kieselgel mit Methylenchlorid/Aether 9:1. Man erhält 0,82 g t-Butyl-(S)-3-[[N-[[5-(dimethylamino-1-naphthyl]sulfonyl]-N-(2-naphthylsulfonyl)-glycinyl]amino]methyl]-1-piperidincarboxylat. Rf = 0,2 (Methylenchlorid/Aether 9:1). Diese 0,82g werden analog Beispiel 70e) in 316 mg N-[[(S)-1-Amidino-3-piperidinyl]methyl]-2-[[[5-(dimethylamino)-1-naphthyl]sulfonyl]-(2-naphthylsulfonyl)amino]acetamid-acetat überführt. Rf = 0,46 (Takeda-Lösung). FAB-MS: 637 (M + 1).

Beispiel 73

Analog Beispiel 30 jedoch unter Verwendung von 2-Thienylglyoxalsäure, Benzoesäure bzw. Benzylchloroformiat an Stelle von Phenylglyoxalsäure (Beispiel 30g) erhält man:

a) (R)-N-[[(S)-1-Amidino-3-piperidinyl]methyl]-2-(2-naphthylsulfonamido)-3-(α-oxo-2-thiophenacetamido)-propionamid-p-toluolsulfonat (1:1), FAB-MS: 571 $(M+H)^+$,

b) N-[(R)-2-[[[(S)-1-Amidino-3-piperidinyl]methyl]carbamoyl]-2-(2-naphthylsulfonamido)äthyl]benzamid-p-toluolsulfonat (1:1), FAB-MS: 537 $(M+H)^+$, bzw.

c) Benzyl-[(R)-2-[[[(S)-1-amidino-3-piperidinyl]methyl]carbamoyl]-2-(2-naphthylsulfonamido]äthyl]-carbamat-acetat (1:1), FAB-MS: 567 $(M+H)^+$.

Beispiel 74

Aus dem Produkt von Beispiel 27 erhält man durch Behandeln mit wässriger Natronlauge die o-[[(R)-α-[[[(RS)-1Amidino-3-piperidinyl]methyl]carbamoyl]-p-nitrophenäthyl]sulfamoyl]benzoesäure, FAB-MS: 533 $(M+H)^+$.

Beispiel 75

Analog Beispiel 68 und 69 erhält man die (R)-4-[(S)-3-[[[(S)-1-Amidino-3-piperidinyl]methyl]carbamoyl]-2-(2-naphthylsulfonamido)propionyl]hexahydro-1,4-oxazepin-3-carbonsäure, FAB-MS: 589 $(M+H^+$.

Beispiel 76

Analog Beispiel 52 (aber mit 2-Naphthylsulfochlorid) und Beispiel 60 erhält man die p-[(RS)-2-[[[(S)-1-Amidino-3-piperidinyl]methyl]carbamoyl]-2-(2-naphthylsulfonamido)äthyl]benzoesäure, FAB-MS: 538 $(M+H)^+$.

Beispiel 77

Analog Beispiel 43 erhält man die 4'-[(R)-2-[[[(S)-1-Amidino-3-piperidinyl]methyl]carbamoyl]-2-(2-naphthylsulfonamido)äthyl]succinanilidsäure. FAB-MS: 609 $(M+H)^+$.

Eine Verbindung der Formel I, ein Solvat oder Salz davon kann man in an sich bekannter Weise als Wirkstoff zur Herstellung von pharmazeutischen Präparaten, z.B. von Tabletten und Kapseln folgender Zusammensetzung, verwenden:

| Beispiel A | |
|---|---|
| | pro Tablette |
| Wirkstoff | 200 mg |
| mikrokristalline Cellulose | 155 mg |
| Maisstärke | 25 mg |
| Talk | 25 mg |
| Hydroxypropylmethylcellulose | 20 mg |
| | 425 mg |

| Beispiel B | |
|---|---|
| | pro Kapsel |
| Wirkstoff | 100,0 mg |
| Maisstärke | 20,0 mg |
| Milchzucker | 95,0 mg |
| Talk | 4,5 mg |
| Magnesiumstearat | 0,5 mg |
| | 220,0 mg |

**Patentansprüche**

**Patentansprüche für folgende Vertragsstaaten : AT, BE, CH, DE, DK, FR, GB, IT, LI, LU, NL, SE**

**1.** Guanidine der Formel

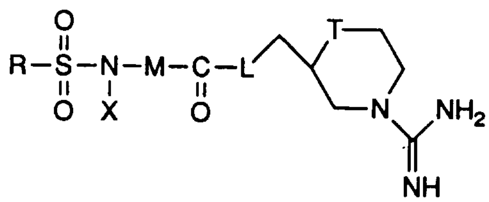

I

worin

| | |
|---|---|
| R | Aryl, Heteroaryl oder Heterocyclyl, |
| T | $CH_2$ oder O, |
| L | NH oder O und |
| -N(X)-M- | eine Gruppe $-N(SO_2-R^o)-CH_2-$ oder eine gegebenenfalls im Phenylring substituierte Isochinolylengruppe ist, und |
| $R^o$ | die gleichen Bedeutungen wie R hat, oder |
| X | H, $-CH_2COOH$, $-CH_2COO-C_{1-4}$-Alkyl, $-CH_2CO$-(Tetra- bis Heptamethylenimino) oder gegebenenfalls N-mono- oder N-di-$C_{1-4}$-alkyliertes $-CH_2CONH_2$, und |
| M | eine Gruppe $R'-(CH_2)_{1-2}CH=$, $R'-COCH_2CH=$, $R''-COCH_2CH=$, $R'-(CO)_{1-2}NHCH_2CH=$, Benzyl-$OCONHCH_2CH=$, $-CH_2[R'-(CO)_{1-2}NH]CH-$, $-CH_2$(Benzyl-OCONH)CH- oder $-CH(CO-Q)CH_2-$, |
| R' | Aryl, Heteroaryl, Cycloalkyl oder Heterocyclyl, |
| R'' | Tetra- bis Heptamethylenimino gegebenenfalls mit bis zu 2 Substituenten aus der Gruppe von Oxo, $-COO-C_{1-4}$-Alkyl, $-(CH_2)_{0-1}OH$, $-(CH_2)_{0-1}OCO-C_{1-4}$-Alkyl und gegebenenfalls mono- oder di-$C_{1-4}$-alkyliertes Carbamoyl, und |
| Q | Benzylamino oder eine gegebenenfalls durch ein O- oder S-Atom unterbrochene und gegebenenfalls durch bis zu 2 Substituenten aus der Gruppe von $C_{1-4}$-Alkyl, COOH, $-COO-C_{1-4}$-Alkyl, $-CH_2OH$ und $-CH_2O$-Benzyl substituierte Tetra- bis Heptamethyleniminogruppe ist, |

sowie Hydrate oder Solvate und physiologisch verwendbare Salze davon.

**2.** Guanidine nach Anspruch 1 und der Formel

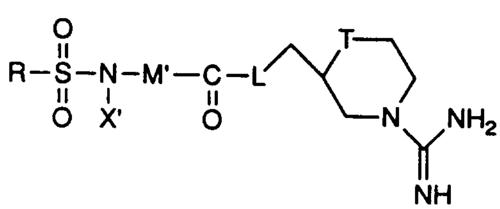

IA

worin

| | |
|---|---|
| -N(X')-M- | eine gegebenenfalls im Phenylring substituierte Isochinolylengruppe, oder |
| X' | H, $-CH_2COOH$, $-CH_2COO-C_{1-4}$-Alkyl, $-CH_2CO-$ (Tetra- bis Heptamethylenimino) oder gegebenenfalls N-mono- oder N-di-$C_{1-4}$-alkyliertes $-CH_2CONH_2$, und |
| M' | eine Gruppe R'-$(CH_2)_{1-2}CH=$, R'-$COCH_2CH=$ oder -CH(CO-Q')$CH_2$-, |
| Q' | Benzylamino, Morpholino oder Tetra- bis Heptamethylenimino ist, und |
| R, R', L und T | die gleichen Bedeutungen wie in Anspruch 1 haben, |

sowie Hydrate oder Solvate und physiologisch verwendbare Salze davon.

**3.** Verbindungen nach Anspruch 2, worin X' H ist und R, M', L und T die gleichen Bedeutungen wie in Anspruch 2 haben.

**4.** Guanidine nach Anspruch 1 und der Formel

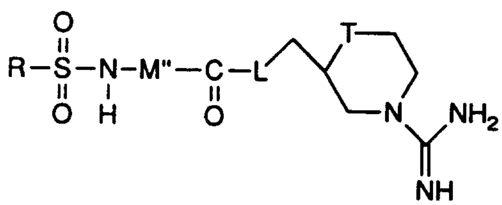

IB

worin

| | |
|---|---|
| M'' | eine Gruppe R''-$COCH_2CH=$, R'-$(CO)_{1-2}NHCH_2CH=$, Benzyl-$OCONHCH_2CH=$, $-CH_2$[R'-$(CO)_{1-2}$NH]CH-, $-CH_2$(Benzyl-OCONH)CH- oder -CH(CO-Q'')$CH_2$-, |
| Q'' | eine gegebenenfalls durch ein O- oder S-Atom unterbrochene und gegebenenfalls durch bis zu 2 Substituenten aus der Gruppe von $C_{1-4}$-Alkyl, COOH, -COO-$C_{1-4}$-Alkyl, $-CH_2OH$ und $-CH_2O$-Benzyl substituierte Tetra- bis Heptamethyleniminogruppe ist, und |
| R, R', R'', L und T | die gleichen Bedeutungen wie in Anspruch 1 haben. |

**5.** Guanidine nach Anspruch 1, worin R, L, T, M bzw. -N(X)-M- und X die gleichen Bedeutungen wie in Anspruch 1 haben, X jedoch nicht H ist.

**6.** Guanidine nach Anspruch 1, worin R Aryl, insbesondere Naphthyl, Hydroxynaphthyl, 4-Biphenyl, 2-Anthryl, Jodphenyl, Nitrophenyl, Benzyloxyphenyl, Dimethoxyphenyl, 4-Methoxy-2,3,6-trimethylphenyl, 2,4,6-Triisopropylphenyl, Carboxyphenyl, Methoxycarbonylphenyl, Benzyloxynaphthyl, Phenylsulfonylphenyl, Hexahydroazepinoylphenyl oder t-Butylphenyl ist.

**7.** Guanidine nach Anspruch 1, worin R Heteroaryl ist, insbesondere 3-Methyl-8-chinolyl, 5-(1-Methyl-5-trifluormethylpyrazol-3-yl)-2-thienyl oder Benzothienyl ist.

**8.** Guanidine nach Anspruch 1 oder 2, worin R Heterocyclyl, insbesondere 3-Methyl-1,2,3,4-tetrahydro-8-chinoly, ist.

**9.** Guanidine nach Anspruch 1, worin -N(X)-M- Isochinolylen oder N-Dimethylaminonaphthylsulfonylaminomethylen ist.

**10.** Guanidine nach Anspruch 1 oder 2, worin X H oder $-CH_2COOH$ ist.

**11.** Guanidine nach Anspruch 1, worin M eine Gruppe R'-$(CH_2)_{1-2}CH=$ ist, insbesondere 3-Indolyläthyliden, 2,3-Dioxo-1-indolinyläthyliden, Phenäthyliden, 1,4-Dioxo-5H-2,5-benzodiazepin-5-yläthyliden, (Fluor, Chlor, Jod, Cyano, Nitro, Amino, Carboxy, $C_{1-4}$-Alkoxycarbonyl oder Hydroxy)-phenäthyliden, Cyclohexylpropyliden, Decalyläthyliden, Imidazolyläthyliden, Thienyläthyliden, (Methyl, Brom, Fluor oder Carboxymethyl)-3-indolyläthyliden, Naphthyläthyliden, (Aethoxycarbonylcarbonylamino, Methoxycarbonyläthylcarbonylamino, Benzyloxycarbonyläthylcarbonylamino, Aethoxycarbonylamino, Benzoylcarbonylamino, Carboxybenzoylamino, Methoxyäthoxyacetamido, Acetamido, Carboxycarbonylamino, Carboxypropionylamino, Tolylsulfonamido, Jodophenylsulfonamido, Carboxyphenylsulfonamido oder

Aethoxycarbonylmethylamino)phenäthyliden, Oxobenzoxazolinäthyliden oder 5-Bromo- oder 5-Methyl-2,3-dioxo-1-indolinyläthyliden.

**12.** Guanidine nach Anspruch 1, worin M eine Gruppe (R' oder R") $COCH_2CH=$ ist, insbesondere Hexahydroazepinoyläthyliden, (Methoxycarbonyl oder Carboxy)pyrrolidinoyläthyliden, 3,4-Dihydro-2(1H)-isochinolinoyläthyliden, (Nitro, Amino, Jodo oder Formamido)benzoyl-äthyliden, Morpholinoäthyliden, Heptahydroazocinoyläthyliden, (Aethoxycarbonyl, Acetoxymethyl, Dimethylcarbamoyl, Isobutyryloxymethyl oder Butyryloxymethyl)piperidinoyläthyliden, 3-Methoxycarbonyl-4-oxopiperidinoyläthyliden oder 4-Acetoxy-3-äthoxycarbonylpiperidinoyläthyliden.

**13.** Guanidine nach Anspruch 1, Worin M eine Gruppe $R'-(CO)_{1-2}NHCH_2CH=$ ist, insbesondere Benzoylcarboxamidoäthyliden, Thienoylcarboxamidoäthyliden, Benzoylamidoäthyliden oder Benzyloxycarboxamidoäthyliden.

**14.** Guanidine nach Anspruch 1, worin M eine Gruppe $-CH_2[R'-(CO)_{1-2}NH]CH-$ ist, insbesondere 2-(Carboxybenzylamido)äthylen, 2-(Benzyloxybenzoylamido)äthylen, 2-(2-Piperidincarboxamido)äthylen, 2-(Hydroxybenzoylamido)äthylen oder 2-(Aminobenzoylamido)äthylen.

**15.** Guanidine nach Anspruch 1, worin M eine Gruppe $-CH(CO-Q)CH_2-$ist,insbesondere 1-(Benzylaminocarbonyl)äthylen, 1-(Hexahydroazepinoyl)äthylen,1-(Morpholinoyl)äthylen, 1-(Heptahydroazocinoyl)äthylen, 1-[2-(Benzyloxymethylmorpholinoyl)]äthylen, 1-[2-(Hydroxymethylmorpholinoyl)]äthylen, 1-(2-Aethoxycarbonyl-4-methylpiperidinoyl)äthylen, 1-(2-Carboxy-4-methylpiperidinoyl)äthylen oder 1-(3-Carboxyhexahydro-1,4-oxazepinoyl)äthylen ist.

**16.** Guanidine nach Anspruch 1,2 oder 6, worin R Aryl, besonders Naphthyl oder Nitro- oder Jodphenyl, L NH ist und worin das asymmetrische C-Atom im Piperidin- oder Morpholinring die S-Konfiguration hat.

**17.** Guanidine nach Anspruch 1, 11 oder 12, und der Formel

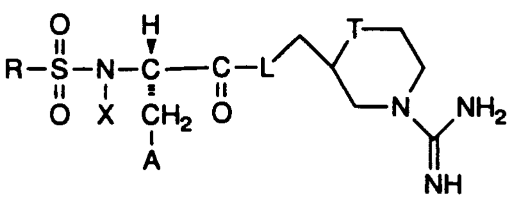

IC

worin A Aryl, Aroyl oder Heterocyclyl ist, insbesondere Phenyl, Nitrophenyl, Indolyl, 2,3-Dioxo-1-indolinyl oder Aminobenzoyl.

**18.** Guanidine nach Anspruch 1 oder 2 aus der Gruppe der folgenden:

(R)-N-[(RS)-1-Amidino-3-piperidinylmethyl]-α-(2-naphthylsulfonamido)-2,3-dioxo-1-indolinpropionamid,

(R)-N-[(RS)-1-Amidino-3-piperidinylmethyl]-α-(2-naphthylsulfonamido)-o-nitrohydrocinnamamid,

(R)-N-[(RS)-1-Amidino-3-piperidinylmethyl]-α-(o-nitrobenzolsulfonamido)indol-3-propionamid,

(R)-N-[(S)-1-Amidino-3-piperidinylmethyl]-α-(p-jodbenzolsulfonamido)indol-3-propionamid,

(R)-N-[(S)-1-Amidino-3-piperidinylmethyl]-α-(p-jodbenzolsulfonamido)-p-nitrohydrocinnamamid,

(R)-N-[(RS)-1-Amidino-3-piperidinylmethyl]-3-(o-aminobenzoyl)-(2-naphthylsulfonamido)-propionamid,

N-[(R)-α-[[(S)-1-Amidino-3-piperidinyl]methylcarbamoyl]phenäthyl]-N-(2-naphthylsulfonyl)glycin,

(R)-N-[(S)-1-Amidino-3-piperidinylmethyl]-1,2,3,4-tetrahydro-2-(2-naphthylsulfonyl)-3-isochinolincarboxamid,

(S)-N-[(RS)-1-Amidino-3-piperidinylmethyl]hexahydro-β-(2-naphthylsulfonamido)-γ-oxo-1H-1-azepinbutyramid,

(R)-N-[(S)-1-Amidino-3-piperidinylmethyl]-α-(2-naphthylsulfonamido)-2,3-dioxo-1-indolinpropionamid,

4'-[(R)-2-[[[(S)-1-Amidino-3-piperidinyl]methyl]carbamoyl]-2-(2-naphthylsulfonamido)äthyl]-oxanilsäure,

(S)-N-[[(RS)-1-Amidino-3-piperidinyl]methyl]hexahydro-β-2-naphthylsulfonamido-γ-oxo-1(2H)-azocinbutyramid,

(2RS,4R)-1-[$N^4$-[[(S)-1-Amidino-3-piperidinyl]methyl]-$N^2$-(2-naphthylsulfonyl)-L-asparaginyl]-4-methyl-2-piperidincarbonsäure,

4'-[(R)-2-[[[(S)-1-Amidino-3-piperidinyl]methyl]carbamoyl]-2-(2-naphthylsulfonamido)äthyl]-succinanilidsäure.

**19.** Verbindungen nach einem der Ansprüche 1-18 zur Verwendung als Heilmittel, insbesondere als Hemmer der durch Thrombin induzierten Plättchenaggregation und Gerinnung von Fibrinogen im Blutplasma.

**20.** Verfahren zur Herstellung der Verbindungen nach einem der Ansprüche 1-18, dadurch gekennzeichnet, dass man

a) eine Säure der Formel

R-$SO_2$N(X)-M-COOH II

unter intermediärem Schutz eines in der Gruppe X, R oder M enthaltenen Carboxygruppe mit einem Amin oder Alkohol der Formel

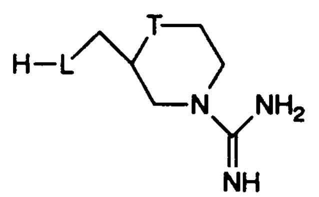

III

oder einem Salz davon umsetzt oder

b) eine Verbindung der Formel

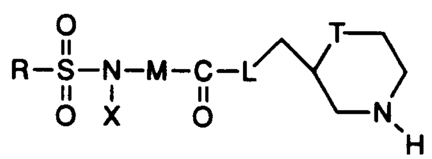

IV

mit einem Amidinierungsreagenz umsetzt oder

c) ein Amin der Formel

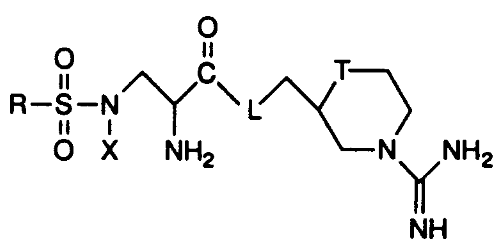

V

mit einer Säure der Formel R'-COOH oder einem funktionellen Derivat davon umsetzt, und

d) gewünschtenfalls eine in der Gruppe M einer Verbindung der Formel I enthaltene reaktionsfähige Gruppe funktionell abwandelt, und

e) gewünschtenfalls eine Verbindung der Formel I in ein physiologisch verträgliches Salz überführt oder ein Salz einer Verbindung der Formel I in die freie Säure oder Base überführt.

**21.** Pharmazeutische Präparate enthaltend eine Verbindung nach einem der Ansprüche 1-18 als Wirkstoff.

**22.** Verwendung einer Verbindung nach einem der Ansprüche 1-18 zur Herstellung von Arzneimitteln zur Behandlung oder Prophylaxe von Krankheiten, die durch Thrombin induzierter Plättchenaggregation

oder Gerinnung von Fibrinogen im Blutplasma verursacht sind.

**23.** Die Verbindungen der Formeln III, IV und V gemäss Anspruch 20.

**Patentansprüche für folgenden Vertragsstaat : ES**

**1.** Verfahren zur Herstellung der Guanidine der Formel

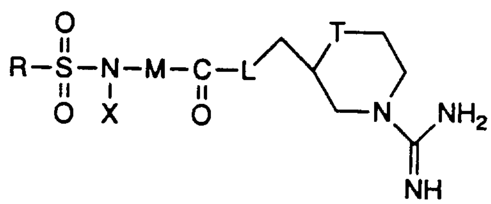

I

worin

| | |
|---|---|
| R | Aryl, Heteroaryl oder Heteroryclyl, |
| T | $CH_2$ oder O, |
| L | NH oder O und |
| -N(X)-M- | eine Gruppe $-N(SO_2-R^o)-CH_2-$ oder eine gegebenenfalls im Phenylring substituierte Isochinolylengruppe ist, und |
| $R^o$ | die gleichen Bedeutungen wie R hat, oder |
| X | H, $-CH_2COOH$, $-CH_2COO-C_{1-4}$-Alkyl, $-CH_2CO$-(Tetra- bis Heptamethylenimino) oder gegebenenfalls N-mono- oder N-di-$C_{1-4}$-alkyliertes $-CH_2CONH_2$, und |
| M | eine Gruppe $R'-(CH_2)_{1-2}CH=$, $R'-COCH_2CH=$, $R''-COCH_2CH=$, $R'-(CO)_{1-2}NHCH_2CH=$, Benzyl-$OCONHCH_2CH=$, $-CH_2[R'-(CO)_{1-2}NH]CH-$, $-CH_2$(Benzyl-OCONH)CH- oder $-CH(CO-Q)CH_2-$, |
| R' | Aryl, Heteroaryl, Cycloalkyl oder Heterocyclyl, |
| R'' | Tetra- bis Heptamethylenimino gegebenenfalls mit bis zu 2 Substituenten aus der Gruppe von Oxo, $-COO-C_{1-4}$-Alkyl, $-(CH_2)_{0-1}OH$, $-(CH_2)_{0-1}OCO-C_{1-4}$-Alkyl und gegebenenfalls mono- oder di-$C_{1-4}$-alkyliertes Carbamoyl, und |
| Q | Benzylamino oder eine gegebenenfalls durch ein O- oder S-Atom unterbrochene und gegebenenfalls durch bis zu 2 Substituenten aus der Gruppe von $C_{1-4}$-Alkyl, COOH, $-COO-C_{1-4}$-Alkyl, $-CH_2OH$ und $-CH_2O$-Benzyl substituierte Tetra- bis Heptamethyleniminogruppe ist, |

sowie Hydrate oder Solvate und physiologisch verwendbare Salze davon, dadurch gekennzeichnet, dass man

a) eine Säure der Formel

$R-SO_2N(X)-M-COOH$ II

unter intermediärem Schutz eines in der Gruppe X, R oder M enthaltenen Carboxygruppe mit einem Amin oder Alkohol der Formel

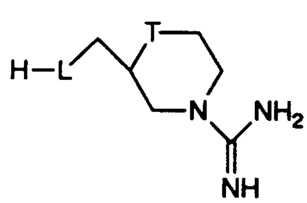

III

oder einem Salz davon umsetzt oder

b) eine Verbindung der Formel

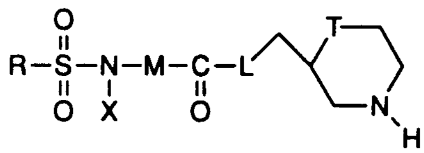

IV

mit einem Amidinierungsreagenz umsetzt oder

c) ein Amin der Formel

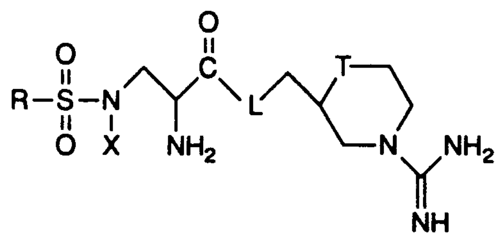

V

mit einer Säure der Formel R'-COOH oder einem funktionellen Derivat davon umsetzt, und

d) gewünschtenfalls eine in der Gruppe M einer Verbindung der Formel I enthaltene reaktionsfähige Gruppe funktionell abwandelt, und

e) gewünschtenfalls eine Verbindung der Formel I in ein physiologisch verträgliches Salz überführt oder ein Salz einer Verbindung der Formel I in die freie Säure oder Base überführt.

**2.** Verfahren nach Anspruch 1 zur Herstellung der Guanidine der Formel

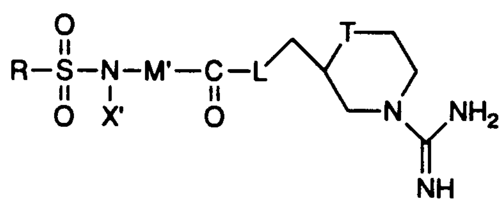

IA

worin

| | |
|---|---|
| -N(X')-M- | eine gegebenenfalls im Phenylring substituierte Isochinolylengruppe, oder |
| X' | H, $-CH_2COOH$, $-CH_2COO-C_{1-4}$-Alkyl, $-CH_2CO-$ (Tetra- bis Heptamethylenimino) oder gegebenenfalls N-mono- oder N-di-$C_{1-4}$-alkyliertes $-CH_2CONH_2$, und |
| M' | eine Gruppe $R'-(CH_2)_{1-2}CH=$, $R'-COCH_2CH=$ oder $-CH(CO-Q')CH_2-$, |
| Q' | Benzylamino, Morpholino oder Tetra- bis Heptamethylenimino ist, und |
| R, R', L und T | die gleichen Bedeutungen wie in Anspruch 1 haben, |

sowie Hydrate oder Solvate und physiologisch verwendbare Salze davon.

**3.** Verfahren nach Anspruch 2, worin X' H ist und R, M', L und T die gleichen Bedeutungen wie in Anspruch 2 haben.

**4.** Verfahren nach Anspruch 1 zur Herstellung der Guanidine der Formel

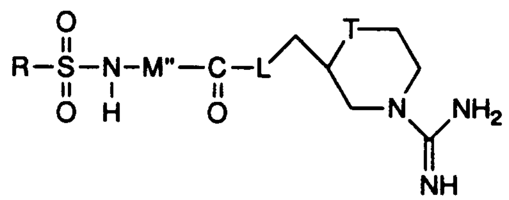

IB

worin

| | |
|---|---|
| M" | eine Gruppe R"-$COCH_2CH$=, R'-$(CO)_{1-2}NHCH_2CH$=, Benzyl-$OCONHCH_2CH$=, -$CH_2$[R'-$(CO)_{1-2}$NH]CH-, -$CH_2$(Benzyl-OCONH)CH- oder -CH(CO-Q")$CH_2$-, |
| Q" | eine gegebenenfalls durch ein O- oder S-Atom unterbrochene und gegebenenfalls durch bis zu 2 Substituenten aus der Gruppe von $C_{1-4}$-Alkyl, COOH, -COO-$C_{1-4}$-Alkyl, -$CH_2$OH und -$CH_2$O-Benzyl substituierte Tetra- bis Heptamethyleniminogruppe ist, und |
| R, R', R", L und T | die gleichen Bedeutungen wie in Anspruch 1 haben. |

**5.** Verfahren nach Anspruch 1, worin R, L, T, M bzw. -N(X)-M- und X die gleichen Bedeutungen wie in Anspruch 1 haben, X jedoch nicht H ist.

**6.** Verfahren nach Anspruch 1, worin R Aryl, insbesondere Naphthyl, Hydroxynaphthyl, 4-Biphenyl, 2-Anthryl, Jodphenyl, Nitrophenyl, Benzyloxyphenyl, Dimethoxyphenyl, 4-Methoxy-2,3,6-trimethylphenyl, 2,4,6-Triisopropylphenyl, Carboxyphenyl, Methoxycarbonylphenyl, Benzyloxynaphthyl, Phenylsulfonylphenyl, Hexahydroazepinoylphenyl oder t-Butylphenyl ist.

**7.** Verfahren nach Anspruch 1, worin R Heteroaryl ist, insbesondere 3-Methyl-8-chinolyl, 5-(1-Methyl-5-trifluormethylpyrazol-3-yl)-2-thienyl oder Benzothienyl ist.

**8.** Verfahren nach Anspruch 1 oder 2, worin R Heterocyclyl, insbesondere 3-Methyl-1,2,3,4-tetrahydro-8-chinolyl, ist.

**9.** Verfahren nach Anspruch 1, worin -N(X)-M- Isochinolylen oder N-Dimethylaminonaphthylsulfonylaminomethylen ist.

**10.** Verfahren nach Anspruch 1 oder 2, worin X H oder -$CH_2$COOH ist.

**11.** Verfahren nach Anspruch 1, worin M eine Gruppe R'-$(CH_2)_{1-2}CH$= ist, insbesondere 3-Indolyläthyliden, 2,3-Dioxo-1-indolinyläthyliden, Phenäthyliden, 1,4-Dioxo-5H-2,5-benzodiazepin-5-yläthyliden, (Fluor, Chlor, Jod, Cyano, Nitro, Amino, Carboxy, $C_{1-4}$-Alkoxycarbonyl oder Hydroxy)-phenäthyliden, Cyclohexylpropyliden, Decalyläthyliden, Imidazolyläthyliden, Thienyläthyliden, (Methyl, Brom, Fluor oder Carboxymethyl)-3-indolyläthyliden, Naphthyläthyliden, (Aethoxycarbonylcarbonylamino, Methoxycarbonyläthylcarbonylamino, Benzyloxycarbonyläthylcarbonylamino, Aethoxycarbonylamino, Benzoylcarbonylamino, Carboxybenzoylamino, Methoxyäthoxyacetamido, Acetamido, Carboxycarbonylamino, Carboxypropionylamino, Tolylsulfonamido, Jodophenylsulfonamido, Carboxyphenylsulfonamido oder Aethoxycarbonylmethylamino)phenäthyliden, Oxobenzoxazolinäthyliden oder 5-Bromo- oder 5-Methyl-2,3-dioxo-1-indolinyläthyliden.

**12.** Verfahren nach Anspruch 1, worin M eine Gruppe (R' oder R") $COCH_2CH$= ist, insbesondere Hexahydroazepinoyläthyliden, (Methoxycarbonyl oder Carboxy)pyrrolidinoyläthyliden, 3,4-Dihydro-2(1H)-isochinolinoyläthyliden, (Nitro, Amino, Jodo oder Formamido)benzoyläthyliden, Morpholinoäthyliden, Heptahydroazocinoyläthyliden, (Aethoxycarbonyl, Acetoxymethyl, Dimethylcarbamoyl, Isobutyryloxymethyl oder Butyryloxymethyl)piperidinoyläthyliden, 3-Methoxycarbonyl-4-oxopiperidinoyläthyliden oder 4-Acetoxy-3-äthoxycarbonylpiperidinoyläthyliden.

**13.** Verfahren nach Anspruch 1, worin M eine Gruppe R'-(CO)$_{1-2}$NHCH$_2$CH= ist, insbesondere Benzoylcarboxamidoäthyliden, Thienoylcarboxamidoäthyliden, Benzoylamidoäthyliden oder Benzyloxycarboxamidoäthyliden.

**14.** Verfahren nach Anspruch 1, worin M eine Gruppe -CH$_2$[R'-(CO)$_{1-2}$NH]CH- ist, insbesondere 2-(Carboxybenzoylamido)äthylen, 2-(Benzyloxybenzoylamido)äthylen, 2-(2-Piperidincarboxamido)äthylen, 2-(Hydroxybenzoylamido)äthylen oder 2-(Aminobenzoylamido)äthylen.

**15.** Verfahren nach Anspruch 1, worin M eine Gruppe -CH(CO-Q)CH$_2$- ist, insbesondere 1-(Benzylaminocarbonyl)äthylen, 1-(Hexahydroazepinoyl)äthylen,1-(Morpholinoyl)äthylen, 1-(Heptahydroazocinoyl)-äthylen, 1-[2-(Benzyloxymethylmorpholinoyl)]äthylen, 1-[2-(Hydroxymethylmorpholinoyl)]äthylen, 1-(2-Aethoxycarbonyl-4-methylpiperidinoyl)äthylen, 1-(2-Carboxy-4-methylpiperidinoyl)äthylen oder 1-(3-Carboxyhexahydro-1,4-oxazepinoyl)äthylen ist.

**16.** Verfahren nach Anspruch 1,2 oder 6, worin R Aryl, besonders Naphthyl oder Nitro- oder Jodphenyl, L NH ist und worin das asymmetrische C-Atom im Piperidin- oder Morpholinring die S-Konfiguration hat.

**17.** Verfahren nach Anspruch 1, 11 oder 12, zur Herstellung der Guanidine der Formel

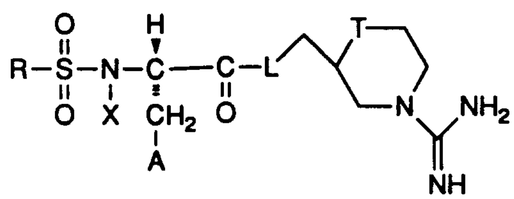

IC

worin A Aryl, Aroyl oder Heterocyclyl ist, insbesondere Phenyl, Nitrophenyl, Indolyl, 2,3-Dioxo-1-indolinyl oder Aminobenzoyl.

**18.** Verfahren nach Anspruch 1 oder 2 zur Herstellung der Guanidine aus der Gruppe der folgenden:
(R)-N-[(RS)-1-Amidino-3-piperidinylmethyl]-α-(2-naphthylsulfonamido)-2,3-dioxo-1-indolinpropionamid,
(R)-N-[(RS)-1-Amidino-3-piperidinylmethyl]-α-(2-naphthylsulfonamido)-o-nitrohydrocinnamamid,
(R)-N-[(RS)-1-Amidino-3-piperidinylmethyl]-α-(o-nitrobenzolsulfonamido)indol-3-propionamid,
(R)-N-[(S)-1-Amidino-3-piperidinylmethyl]-α-(p-jodbenzolsulfonamido)indol-3-propionamid,
(R)-N-[(S)-1 Amidino-3-piperidinylmethyl]-α-(p-jodbenzolsulfonamido)-p-nitrohydrocinnamamid,
(R)-N-[(RS)-1-Amidino-3-piperidinylmethyl]-3-(o-aminobenzoyl)-(2-naphthylsulfonamido)-propionamid,
N[(R)-α-[[(S)-1-Amidino-3-piperidinyl]methylcarbamoyl]phenäthyl]-N-(2-naphthylsulfonyl)glycin,
(R)-N-[(S)-1-Amidino-3-piperidinylmethyl]-1,2,3,4-tetrahydro-2-(2-naphthylsulfonyl)-3-isochinolincarboxamid,
(S)-N-[(RS)-1-Amidino-3-piperidinylmethyl]hexahydro-β-(2-naphthylsulfonamido)-γ-oxo-1H-1-azepinbutyramid,
(R)-N-[(S)-1-Amidino-3-piperidinylmethyl]-α-(2-naphthylsulfonamido)-2,3-dioxo-1-indolinpropionamid,
4'-[(R)-2-[[[(S)-1-Amidino-3-piperidinyl]methyl]carbamoyl]-2-(2-naphthylsulfonamido)äthyl]-oxanilsäure,
(S)-N-[[(RS)-1-Amidino-3-piperidinyl]methyl]hexahydro-β-2-naphthylsulfonamido-γ-oxo-1(2H)-azocinbutyramid,
(2RS,4R)-1-[N$^4$-[[(S)-1-Amidino-3-piperidinyl]methyl]-N$^2$-(2-naphthylsulfonyl)-L-asparaginyl]-4-methyl-2-piperidincarbonsäure,
4'-[(R)-2-[[[(S)-1-Amidino-3-piperidinyl]methyl]carbamoyl]-2-(2-naphthylsulfonamido)äthyl]-succinanilidsäure.

**19.** Verwendung einer Verbindung nach einem der Ansprüche 1-18 zur Herstellung von Arzneimitteln zur Behandlung oder Prophylaxe von Krankheiten, die durch Thrombin induzierter Plättchenaggregation oder Gerinnung von Fibrinogen im Blutplasma verursacht sind.

**Patentansprüche für folgenden Vertragsstaat : GR**

**1.** Verfahren zur Herstellung der Guanidine der Formel

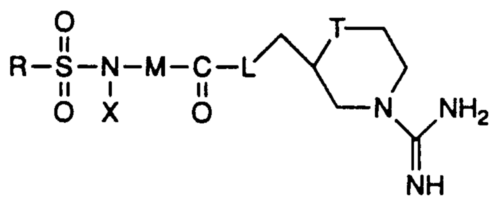

I

worin

| | |
|---|---|
| R | Aryl, Heteroaryl oder Heterocyclyl, |
| T | $CH_2$ oder O, |
| L | NH oder O und |
| -N(X)-M- | eine Gruppe $-N(SO_2R^o)-CH_2-$ oder eine gegebenenfalls im Phenylring substituierte Isochinolylengruppe ist, und |
| $R^o$ | die gleichen Bedeutungen wie R hat, oder |
| X | H, $-CH_2COOH$, $-CH_2COO-C_{1-4}$-Alkyl, $-CH_2CO$-(Tetra- bis Heptamethylenimino) oder gegebenenfalls N-mono- oder N-di-$C_{1-4}$-alkyliertes $-CH_2CONH_2$, und |
| M | eine Gruppe $R'-(CH_2)_{1-2}CH=$, $R'-COCH_2CH=$, $R''-COCH_2CH=$, $R'-(CO)_{1-2}NHCH_2CH=$, Benzyl-$OCONHCH_2CH=$, $-CH_2[R'-(CO)_{1-2}NH]CH-$, $-CH_2$(Benzyl-OCONH)CH- oder -CH(CO-Q)$CH_2$-, |
| R' | Aryl, Heteroaryl, Cycloalkyl oder Heterocyclyl, |
| R'' | Tetra- bis Heptamethylenimino gegebenenfalls mit bis zu 2 Substituenten aus der Gruppe von Oxo, $-COO-C_{1-4}$-Alkyl, $-(CH_2)_{0-1}OH$, $-(CH_2)_{0-1}OCO-C_{1-4}$-Alkyl und gegebenenfalls mono- oder di-$C_{1-4}$-alkyliertes Carbamoyl, und |
| Q | Benzylamino oder eine gegebenenfalls durch ein O- oder S-Atom unterbrochene und gegebenenfalls durch bis zu 2 Substituenten aus der Gruppe von $C_{1-4}$-Alkyl, COOH, $-COO-C_{1-4}$-Alkyl, $-CH_2OH$ und $-CH_2O$-Benzyl substituierte Tetra- bis Heptamethyleniminogruppe ist, |

sowie Hydrate oder Solvate und physiologisch verwendbare Salze davon,
dadurch gekennzeichnet, dass man

a) eine Säure der Formel

$R-SO_2N(X)-M-COOH$ II

unter intermediärem Schutz eines in der Gruppe X, R oder M enthaltenen Carboxygruppe mit einem Amin oder Alkohol der Formel

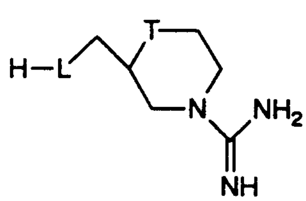

III

oder einem Salz davon umsetzt oder

b) eine Verbindung der Formel

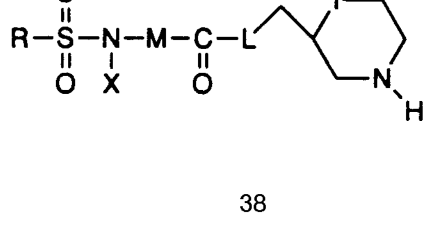

IV

mit einem Amidinierungsreagenz umsetzt oder

c) ein Amin der Formel

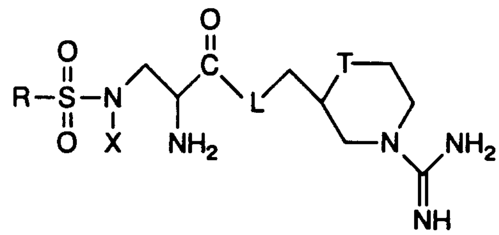

V

mit einer Säure der Formel R'-COOH oder einem funktionellen Derivat davon umsetzt, und

d) gewünschtenfalls eine in der Gruppe M einer Verbindung der Formel I enthaltene reaktionsfähige Gruppe funktionell abwandelt, und

e) gewünschtenfalls eine Verbindung der Formel I in ein physiologisch verträgliches Salz überführt oder ein Salz einer Verbindung der Formel I in die freie Säure oder Base überführt.

**2.** Verfahren nach Anspruch 1 zur Herstellung der Guanidine der Formel

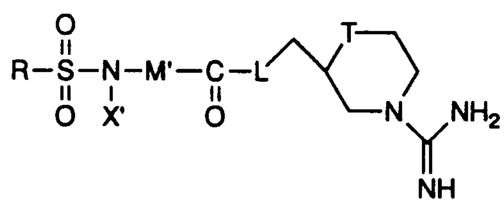

IA

worin

| | |
|---|---|
| -N(X')-M- | eine gegebenenfalls im Phenylring substituierte Isochinolylengruppe, oder |
| X' | H, $-CH_2COOH$, $-CH_2COO-C_{1-4}$-Alkyl, $-CH_2CO-$ (Tetra- bis Heptamethylenimino) oder gegebenenfalls N-mono- oder N-di-$C_{1-4}$-alkyliertes $-CH_2CONH_2$, und |
| M' | eine Gruppe $R'-(CH_2)_{1-2}CH=$, $R'-COCH_2CH=$ oder $-CH(CO-Q')CH_2-$, |
| Q' | Benzylamino, Morpholino oder Tetra- bis Heptamethylenimino ist, und |
| R, R', L und T | die gleichen Bedeutungen wie in Anspruch 1 haben, |

sowie Hydrate oder Solvate und physiologisch verwendbare Salze davon.

**3.** Verfahren nach Anspruch 2, worin X' H ist und R, M', L und T die gleichen Bedeutungen wie in Anspruch 2 haben.

**4.** Verfahren nach Anspruch 1 zur Herstellung der Guanidine der Formel

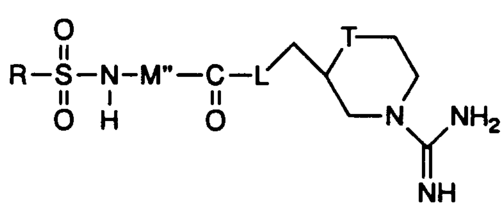

IB

worin

| | |
|---|---|
| M'' | eine Gruppe $R''-COCH_2CH=$, $R'-(CO)_{1-2}NHCH_2CH=$, Benzyl-$OCONHCH_2CH=$, $-CH_2[R'-(CO)_{1-2}NH]CH-$, $-CH_2$(Benzyl-OCONH)CH- oder $-CH(CO-Q'')CH_2-$, |
| Q'' | eine gegebenenfalls durch ein O- oder S-Atom unterbrochene und gegebenenfalls durch bis zu 2 Substituenten aus der Gruppe von $C_{1-4}$-Alkyl, COOH, $-COO-C_{1-4}$-Alkyl, $-CH_2OH$ und $-CH_2O$-Benzyl substituierte Tetra- bis Heptamethyleniminogruppe ist, und |

R, R', R'', L und T die gleichen Bedeutungen wie in Anspruch 1 haben.

5. Verfahren nach Anspruch 1, worin R, L, T, M bzw. -N(X)-M- und X die gleichen Bedeutungen wie in Anspruch 1 haben, X jedoch nicht H ist.

6. Verfahren nach Anspruch 1, worin R Aryl, insbesondere Naphthyl, Hydroxynaphthyl, 4-Biphenyl, 2-Anthryl, Jodphenyl, Nitrophenyl, Benzyloxyphenyl, Dimethoxyphenyl, 4-Methoxy-2,3,6-trimethylphenyl, 2,4,6-Triisopropylphenyl, Carboxyphenyl, Methoxycarbonylphenyl, Benzyloxynaphthyl, Phenylsulfonylphenyl, Hexahydroazepinoylphenyl oder t-Butylphenyl ist.

7. Verfahren nach Anspruch 1, worin R Heteroaryl ist, insbesondere 3-Methyl-8-chinolyl, 5-(1-Methyl-5-trifluormethylpyrazol-3-yl)-2-thienyl oder Benzothienyl ist.

8. Verfahren nach Anspruch 1 oder 2, worin R Heterocyclyl, insbesondere 3-Methyl-1,2,3,4-tetrahydro-8-chinolyl, ist.

9. Verfahren nach Anspruch 1, worin -N(X)-M- Isochinolylen oder N-Dimethylaminonaphthylsulfonylaminomethylen ist.

10. Verfahren nach Anspruch 1 oder 2, worin X H oder $-CH_2COOH$ ist.

11. Verfahren nach Anspruch 1, worin M eine Gruppe $R'-(CH_2)_{1-2}CH=$ ist, insbesondere 3-Indolyläthyliden, 2,3-Dioxo-1-indolinyläthyliden, Phenäthyliden, 1,4-Dioxo-5H-2,5-benzodiazepin-5-yläthyliden, (Fluor, Chlor, Jod, Cyano, Nitro, Amino, Carboxy, $C_{1-4}$-Alkoxycarbonyl oder Hydroxy)-phenäthyliden, Cyclohexylpropyliden, Decalyläthyliden, Imidazolyläthyliden, Thienyläthyliden, (Methyl, Brom, Fluor oder Carboxymethyl)-3-indolyläthyliden, Naphthyläthyliden, (Aethoxycarbonylcarbonylamino, Methoxycarbonyläthylcarbonylamino, Benzyloxycarbonyläthylcarbonylamino, Aethoxycarbonylamino, Benzoylcarbonylamino, Carboxybenzoylamino, Methoxyäthoxyacetamido, Acetamido, Carboxycarbonylamino, Carboxypropionylamino, Tolylsulfonamido, Jodophenylsulfonamido, Carboxyphenylsulfonamido oder Aethoxycarbonylmethylamino)phenäthyliden, Oxobenzoxazolinäthyliden oder 5-Bromo- oder 5-Methyl-2,3-dioxo-1-indolinyläthyliden.

12. Verfahren nach Anspruch 1, worin M eine Gruppe
(R' oder R'') $COCH_2CH=$ ist, insbesondere Hexahydroazepinoyläthyliden, (Methoxycarbonyl oder Carboxy)pyrrolidinoyläthyliden, 3,4-Dihydro-2(1H)-isochinolinoyläthyliden, (Nitro, Amino, Jodo oder Formamido)benzoyläthyliden, Morpholinoäthyliden, Heptahydroazocinoyläthyliden, (Aethoxycarbonyl, Acetoxymethyl, Dimethylcarbamoyl, Isobutyryloxymethyl oder Butyryloxymethyl)piperidinoyläthyliden, 3-Methoxycarbonyl-4-oxopiperidinoyläthyliden oder 4-Acetoxy-3-äthoxycarbonylpiperidinoyläthyliden.

13. Verfahren nach Anspruch 1, worin M eine Gruppe $R'-(CO)_{1-2}NHCH_2CH=$ ist, insbesondere Benzoylcarboxamidoäthyliden, Thienoylcarboxamidoäthyliden, Benzoylamidoäthyliden oder Benzyloxycarboxamidoäthyliden.

14. Verfahren nach Anspruch 1, worin M eine Gruppe $-CH_2[R'-(CO)_{1-2}NH]CH-$ ist, insbesondere 2-(Carboxybenzoylamido)äthylen, 2-(Benzyloxybenzoylamido)äthylen, 2-(2-Piperidincarboxamido)äthylen, 2-(Hydroxybenzoylamido)äthylen oder 2-(Aminobenzoylamido)äthylen.

15. Verfahren nach Anspruch 1, worin M eine Gruppe $-CH(CO-Q)CH_2-$ ist, insbesondere 1-(Benzylaminocarbonyl)äthylen, 1-(Hexahydroazepinoyl)äthylen,1-(Morpholinoyl)äthylen, 1-(Heptahydroazocinoyl)-äthylen, 1-[2-(Benzyloxymethylmorpholinoyl)]äthylen, 1-[2-(Hydroxymethylmorpholinoyl)]äthylen, 1-(2-Aethoxycarbonyl-4-methylpiperidinoyl)äthylen, 1-(2-Carboxy-4-methylpiperidinoyl)äthylen oder 1-(3-Carboxyhexahydro-1,4-oxazepinoyl)äthylen ist.

16. Verfahren nach Anspruch 1, 2 oder 6, worin R Aryl, besonders Naphthyl oder Nitro- oder Jodphenyl, L NH ist und worin das asymmetrische C-Atom im Piperidin- oder Morpholinring die S-Konfiguration hat.

**17.** Verfahren nach Anspruch 1, 11 oder 12, zur Herstellung der Guanidine der Formel

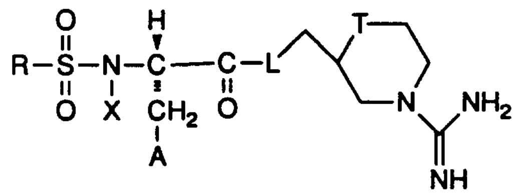

IC

worin A Aryl, Aroyl oder Heterocyclyl ist, insbesondere Phenyl, Nitrophenyl, Indolyl, 2,3-Dioxo-1-indolinyl oder Aminobenzoyl.

**18.** Verfahren nach Anspruch 1 oder 2 zur Herstellung der Guanidine aus der Gruppe der folgenden:
(R)-N-[(RS)-1-Amidino-3-piperidinylmethyl]-α-(2-naphthylsulfonamido)-2,3-dioxo-1-indolinpropionamid,
(R)-N-[(RS)-1-Amidino-3-piperidinylmethyl]-α-(2-naphthylsulfonamido)-o-nitrohydrocinnamamid,
(R)-N-[(RS)-1-Amidino-3-piperidinylmethyl]-α-(o-nitrobenzolsulfonamido)indol-3-propionamid,
(R)-N-[(S)-1-Amidino-3-piperidinylmethyl]-α-(p-jodbenzolsulfonamido)indol-3-propionamid,
(R)-N-[(S)-1 Amidino-3-piperidinylmethyl]-α-(p-jodbenzolsulfonamido)-p-nitrohydrocinnamamid,
(R)-N-[(RS)-1-Amidino-3-piperidinylmethyl]-3-(o-aminobenzoyl)-(2-naphthylsulfonamido)-propionamid,
N-[(R)-α-[[(S)-1-Amidino-3-piperidinyl]methylcarbamoyl]phenäthyl]-N-(2-naphthylsulfonyl)glycin,
(R)-N-[(S)-1-Amidino-3-piperidinylmethyl]-1,2,3,4-tetrahydro-2-(2-naphthylsulfonyl)-3-isochinolincarboxamid,
(S)-N-[(RS)-1-Amidino-3-piperidinylmethyl]hexahydro-β-(2-naphthylsulfonamido)-γ-oxo-1H-1-azepinbutyramid,
(R)-N-[(S)-1 Amidino-3-piperidinylmethyl]-α-(2-naphthylsulfonamido)-2,3-dioxo-1-indolinpropionamid,
4'-[(R)-2-[[[(S)-1-Amidino-3-piperidinyl]methyl]carbamoyl]-2-(2-naphthylsulfonamido)äthyl]-oxanilsäure,
(S)-N-[[(RS)-1-Amidino-3-piperidinyl]methyl]hexahydro-β-2-naphthylsulfonamido-γ-oxo-1(2H)-azocinbutyramid,
(2RS,4R)-1-[$N^4$-[[(S)-1-Amidino-3-piperidinyl]methyl]-$N^2$-(2-naphthylsulfonyl)-L-asparaginyl]-4-methyl-2-piperidincarbonsäure,
4'-[(R)-2-[[[(S)-1-Amidino-3-piperidinyl]methyl]carbamoyl]-2-(2-naphthylsulfonamido)äthyl]-succinanilidsäure.

**19.** Verfahren zur Herstellung von pharmazeutischen Präparaten, speziell zur Verwendung als Heilmittel, insbesondere als Hemmer der durch Thrombin induzierten Plättchenaggregation und Gerinnung von Fibrinogen im Blutplasma, dadurch gekennzeichnet, dass man ein Guanidin nach einem der Ansprüche 1-18 oder ein Salz davon in eine galenische Form bringt.

**20.** Verwendung einer Verbindung nach einem der Ansprüche 1-18 zur Herstellung von Arzneimitteln zur Behandlung oder Prophylaxe von Krankheiten, die durch Thrombin induzierter Plättchenaggregation oder Gerinnung von Fibrinogen im Blutplasma verursacht sind.

**21.** Die Verbindungen der Formeln III, IV und V gemäss Anspruch 1.

## Claims

**Claims for the following Contracting States : AT, BE, CH, DE, DK, FR, GB, IT, LI, LU, NL, SE**

**1.** Guanidines of the formula

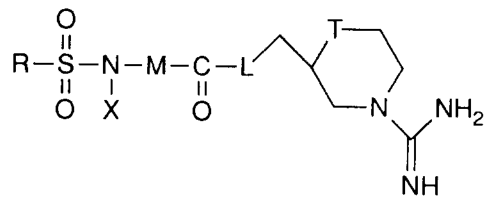

I

wherein

| | |
|---|---|
| R | is aryl, heterorary l or heterocyclyl, |
| T | is $CH_2$ or O, |
| L | is NH or O and |
| -N(X)-M- | is a $-N(SO_2R^o)-CH_2-$ group or an isoquinolylene group optionally substituted in the phenyl ring and |
| $R^o$ | has the same significance as R or |
| X | is H, $-CH_2COOH$, $-CH_2COO-C_{1-4}$-alkyl, $-CH_2CO$-(tetra- to heptamethyleneimino) or optionally N-mono- or N-di-$C_{1-4}$-alkylated $-CH_2CONH_2$ and |
| M | is a $R'-(CH_2)_{1-2}CH=$, $R'-COCH_2CH=$, $R''-COCH_2CH=$, $R'-(CO)_{1-2}NHCH_2CH=$, benzyl-$OCONHCH_2CH=$, $-CH_2[R'-(CO)_{1-2}NH]CH-$, $-CH_2$(benzyl-OCONH)CH- or -CH(CO-Q)$CH_2$- group, |
| R' | is aryl, heteroaryl, cycloalkyl or heterocyclyl, |
| R'' | is tetra- to heptamethyleneimino optionally carrying up to 2 substituents from the group of oxo, $-COO-C_{1-4}$-alkyl, $-(CH_2)_{0-1}OH$, $-(CH_2)_{0-1}OCO-C_{1-4}$-alkyl and optionally mono- or di-$C_{1-4}$-alkylated carbamoyl and |
| Q | is benzylamino or a tetra- to heptamethyleneimino group optionally interrupted by an O or S atom and optionally substituted by up to 2 substituents from the group of $C_{1-4}$-alkyl, COOH, $-COO-C_{1-4}$-alkyl, $-CH_2OH$ and $-CH_2O$-benzyl, |

as well as hydrates or solvates and physiologically usable salts thereof.

**2.** Guanidines according to claim 1 and of the formula

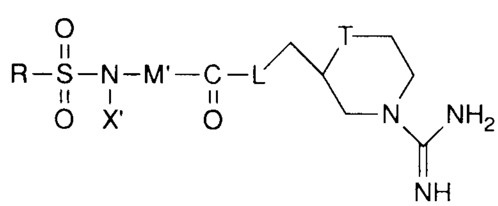

IA

wherein

| | |
|---|---|
| -N(X')-M'- | is an isoquinolylene group optionally substituted in the phenyl ring or |
| X' | is H, $-CH_2COOH$, $-CH_2COO-C_{1-4}$-alkyl, $-CH_2CO$-(tetra- to heptamethyleneimino) or optionally N-mono- or N-di-$C_{1-4}$-alkylated $-CH_2CONH_2$ and |
| M' | is a $R'-(CH_2)_{1-2}CH=$, $R'-COCH_2CH=$ or -CH(CO-Q')$CH_2$- group, |
| Q' | is benzylamino, morpholino or tetra- to heptamethyleneimino and |
| R, R', L and T | have the same significances as in claim 1, |

as well as hydrates or solvents and physiological usable salts thereof.

**3.** Compounds according to claim 2, wherein X' is H and R, M', L and T have the same significances as in claim 2.

**4.** Guanidines according to claim 1 and of the formula

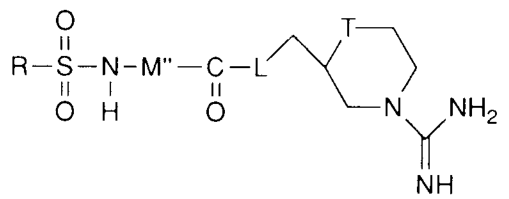

IB

wherein

| | |
|---|---|
| M" | is a R"-$COCH_2CH=$, R'-$(CO)_{1-2}NHCH_2CH=$, benzyl-$OCONHCH_2CH=$, -$CH_2$[R'-$(CO)_{1-2}$NH]CH-, -$CH_2$(benzyl-OCONH)CH- or -CH(CO-Q")$CH_2$-group, |
| Q" | is a tetra- to heptamethyleneimino group optionally interrupted by an O or S atom and optionally substituted by up to 2 substituents from the group of $C_{1-4}$-alkyl, COOH, -COO-$C_{1-4}$-alkyl, -$CH_2$OH and -$CH_2$O-benzyl and |
| R, R', R", L and T | have the same significances as given in claim 1. |

**5.** Guanidines according to claim 1, wherein R, L, T, M or -N(X)-M- and X have the same significances as in claim 1, but X is not H.

**6.** Guanidines according to claim 1 , wherein R is aryl, especially naphthyl, hydroxynaphthyl, 4-biphenyl, 2-anthryl, iodophenyl, nitrophenyl, benzyloxyphenyl, dimethoxyphenyl, 4-methoxy-2,3,6-trimethylphenyl, 2,4,6-triisopropylphenyl, carboxyphenyl, methoxycarbonylphenyl, benzyloxynaphthyl, phenylsulphonylphenyl, hexahydroazepinoylphenyl or t-butylphenyl.

**7.** Guanidines according to claim 1, wherein R is heteroaryl, especially 3-methyl-8-quinolyl, 5-(1-methyl-5-trifluoromethylpyrazol-3-yl)-2-thienyl or benzothienyl.

**8.** Guanidines according to claim 1 or 2, wherein R is heterocyclyl, especially 3-methyl-1,2,3,4-tetrahydro-8-quinolyl.

**9.** Guanidines according to claim 1, wherein -N(X)-M- is isoquinolylene or N-dimethylaminonaphthylsulphonylaminomethylene.

**10.** Guanidines according to claim 1 or 2, wherein X is H or -$CH_2COOH$.

**11.** Guanidines according to claim 1, wherein M is a R'-$(CH_2)_{1-2}CH=$ group, especially 3-indolylethylidene, 2,3-dioxo-1-indolinylethylidene, phenethylidene, 1 ,4-dioxo-5H-2,5-benzodiazepin-5-ylethylidene, (fluoro, chloro, iodo, cyano, nitro, amino, carboxy, $C_{1-4}$-alkoxycarbonyl or hydroxy)-phenethylidene, cyclohexylpropylidene, decalylethylidene, imidazolylethylidene, thienylethylidene, (methyl, bromo, fluoro or carboxymethyl)-3-indolylethylidene, naphthylethylidene, (ethoxycarbonylcarbonylamino, methoxycarbonylethylcarbonylamino, benzyloxycarbonylethylcarbonylamino, ethoxycarbonylamino, benzoylcarbonylamino, carboxybenzoylamino, methoxyethoxyacetamido, acetamido, carboxycarbonylamino, carboxypropionylamino, tolylsulphonamido, iodophenylsulphonamido, carboxyphenylsulphonamido or ethoxycarbonylmethylamino)phenethylidene, oxobenzoxazolinethylidene or 5-bromo- or 5-methyl-2,3-dioxo-1-indolinylethylidene.

**12.** Guanidines according to claim 1, wherein M is a (R' or R")$COCH_2CH=$ group, especially hexahydroazepinoylethylidene, (methoxycarbonyl or carboxy)-pyrrolidinoylethylidene, 3,4-dihydro-2(1H)-isoquinolinoylethylidene, (nitro, amino, iodo or formamido)benzoylethylidene, morpholinoethylidene, heptahydroazocinoylethylidene, (ethoxycarbonyl, acetoxymethyl, dimethylcarbamoyl, isobutyryloxymethyl or butyryloxymethyl)piperidinoylethylidene, 3-methoxycarbonyl-4-oxopiperidinoylethylidene or 4-acetoxy-3-ethoxycarbonylpiperidinoylethylidene.

**13.** Guanidines according to claim 1, wherein M is a $R'-(CO)_{1-2}NHCH_2CH=$ group, especially benzoylcarboxamidoethylidene, thienoylcarboxamidoethylidene, benzoylamidoethylidene or benzyloxycarboxamidoethylidene.

**14.** Guanidines according to claim 1, wherein M is a $-CH_2[R'-(CO)_{1-2}NH]CH-$ group, especially 2-(carboxybenzoylamido)ethylene, 2-(benzyloxybenzoylamido)ethylene, 2-(2-piperidinecarboxamido)-ethylene, 2-(hydroxybenzoylamido)ethylene or 2-(aminobenzoylamido)ethylene.

**15.** Guanidines according to claim 1, wherein M is a $-CH(CO-Q)CH_2-$ group, especially 1-(benzylaminocarbonyl)ethylene, 1-(hexahydroazepinoyl)ethylene, 1-(morpholinoyl)ethylene, 1 -(heptahydroazocinoyl)-ethylene, 1-[2-(benzyloxymethylmorpholinoyl)]ethylene, 1-[2-(hydroxymethylmorpholinoyl)]ethylene, 1-(2-ethoxycarbonyl-4-methylpiperidinoyl)ethylene, 1-(2-carboxy-4-methylpiperidinoyl)ethylene or 1-(3-carboxyhexahydro-1,4-oxazepinoyl)ethylene.

**16.** Guanidines according to claim 1, 2 or 6, wherein R is aryl, especially naphthyl or nitro- or iodophenyl, L is NH and the asymmetric C atom in the piperidine or morpholine ring has the S-configuration.

**17.** Guanidines according to claim 1, 11 or 12 and of the formula

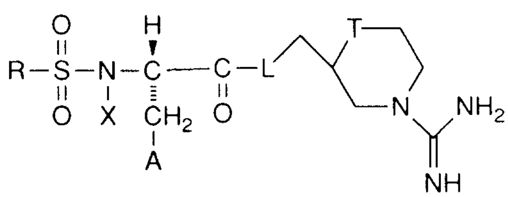

IC

wherein A is aryl, aroyl or heterocyclyl, especially phenyl, nitrophenyl, indolyl, 2,3-dioxo-1-indolinyl or aminobenzoyl.

**18.** Guanidines according to claim 1 or 2 from the following group:

(R)-N-[(RS)-1-Amidino-3-piperidinylmethyl]-α-(2-naphthylsulphonamido)-2,3-dioxo-1-indolinepropionamide,

(R)-N-[(RS)-1-amidino-3-piperidinylmethyl]-α-(2-naphthylsulphonamido)-o-nitrohydrocinnamamide,

(R)-N-[(RS)-1-amidino-3-piperidinylmethyl]-α-(o-nitrobenzenesulphonamido)indole-3-propionamide,

(R)-N-[(S)-1-amidino-3-piperidinylmethyl]-α-(p-iodobenzenesulphonamido)indole-3-propionamide,

(R)-N-[(S)-1-amidino-3-piperidinylmethyl]-α-(p-iodobenzenesulphonamido)-p-nitrohydrocinnamamide,

(R)-N-[(RS)-1-amidino-3-piperidinylmethyl]-3-(o-aminobenzoyl)-(2-naphthylsulphonamido)-propionamide,

N-[(R)-α-[[(S)-1-amidino-3-piperidinyl]methylcarbamoyl]phenethyl]-N-(2-naphthylsulphonyl)glycine,

(R)-N-[(S)-1-amidino-3-piperidinylmethyl]-1,2,3,4-tetrahydro-2-(2-naphthylsulphonyl)-3-isoquinolinecarboxamide,

(S)-N-[(RS)-1-amidino-3-piperidinylmethyl]hexahydro-β-(2-naphthylsulphonamido)-γ-oxo-1H-1-azepinebutyramide,

(R)-N-[(S)-1-amidino-3-piperidinylmethyl]-α-(2-naphthylsulphonamido)-2,3-dioxo-1-indolinepropionamide,

4'-[(R)-2-[[[(S)-1-amidino-3-piperidinyl]methyl]carbamoyl]-2-(2-naphthylsulphonamido)ethyl]oxanilic acid,

(S)-N-[[(RS)-1-amidino-3-piperidinyl]methyl]hexahydro-β-2-naphthylsulphonamido-γ-oxo-1(2H)-azocinebutyramide,

(2RS,4R)-1-[$N^4$-[[(S)-1-amidino-3-piperidinyl]methyl]-$N^2$-(2-naphthylsulphonyl)-L-asparaginyl]-4-methyl-2-piperidinecarboxylic acid,

4'-[(R)-2-[[[(S)-1-amidino-3-piperidinyl]methyl]carbamoyl]-2-(2-naphthylsulphonamido)ethyl]-succinanilidic acid.

**19.** Compounds according to any one of claims 1-18 for use as medicaments, especially as inhibitors of platelet aggregation induced by thrombin and of the clotting of fibrinogen in blood plasma.

**20.** A process for the manufacture of the compounds according to any one of claims 1-18, characterized by
a) reacting an acid of the formula

$R\text{-}SO_2N(X)\text{-}M\text{-}COOH$ II

with intermediary protection of a carboxy group present in the group X, R or M with an amine or alcohol of the formula

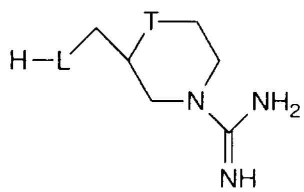

III

or a salt thereof, or
b) reacting a compound of the formula

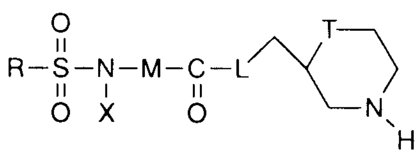

IV

with an amidinating reagent, or
c) reacting an amine of the formula

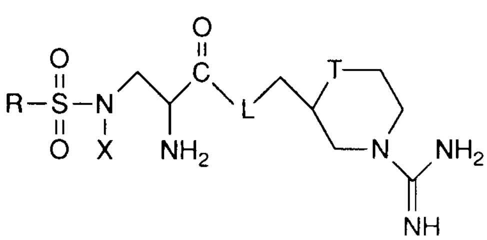

V

with an acid of the formula R'-COOH or a functional derivative thereof, and
d) if desired, functionally modifying a reactive group present in the group M in a compound of formula I, and
e) if desired, converting a compound of formula I into a physiologically acceptable salt or converting a salt of a compound of formula I into the free acid or base.

**21.** A pharmaceutical preparation containing a compound according to any one of claims 1-18 as the active ingredient.

**22.** The use of a compound according to any one of claims 1-18 for the manufacture of medicaments for the treatment or prophylaxis of illnesses which are caused by thrombin-induced platelet aggregation or the clotting of fibrinogen in blood plasma.

**23.** The compounds of formulae III, IV and V in accordance with claim 20.

**Claims for the following Contracting State : ES**

**1.** A process for the manufacture of guanidines of the formula

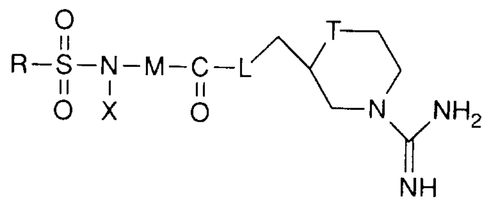

I

wherein

| | |
|---|---|
| R | is aryl, heterorary1 or heterocyclyl, |
| T | is $CH_2$ or O, |
| L | is NH or O and |
| -N(X)-M- | is a $-N(SO_2-R^o)-CH_2-$ group or an isoquinolylene group optionally substituted in the phenyl ring and |
| $R^o$ | has the same significance as R or |
| X | is H, $-CH_2COOH$, $-CH_2COO-C_{1-4}$-alkyl, $-CH_2CO$-(tetra- to heptamethyleneimino) or optionally N-mono- or N-di-$C_{1-4}$-alkylated $-CH_2CONH_2$ and |
| M | is a $R'-(CH_2)_{1-2}CH=$, $R'-COCH_2CH=$, $R''-COCH_2CH=$, $R'-(CO)_{1-2}NHCH_2CH=$, benzyl-$OCONHCH_2CH=$, $-CH_2[R'-(CO)_{1-2}NH]CH-$, $-CH_2$(benzyl-OCONH)CH- or -CH(CO-Q)$CH_2$- group, |
| R' | is aryl, heteroaryl, cycloalkyl or heterocyclyl, |
| R'' | is tetra- to heptamethyleneimino optionally carrying up to 2 substituents from the group of oxo, $-COO-C_{1-4}$-alkyl, $-(CH_2)_{0-1}OH$, $-(CH_2)_{0-1}OCO-C_{1-4}$-alkyl and optionally mono- or di-$C_{1-4}$-alkylated carbamoyl and |
| Q | is benzylamino or a tetra- to heptamethyleneimino group optionally interrupted by an O or S atom and optionally substituted by up to 2 substituents from the group of $C_{1-4}$-alkyl, COOH, $-COO-C_{1-4}$-alkyl, $-CH_2OH$ and $-CH_2O$-benzyl, |

as well as hydrates or solvates and physiologically usable salts thereof, characterized by

a) reacting an acid of the formula

$R-SO_2N(X)-M-COOH$  II

with intermediary protection of a carboxy group present in the group X, R or M with an amine or alcohol of the formula

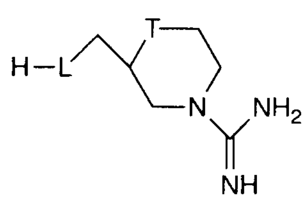

III

or a salt thereof, or

b) reacting a compound of the formula

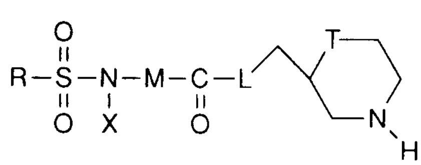

IV

with an amidinating reagent, or

c) reacting an amine of the formula

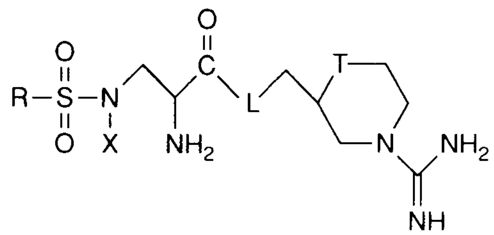

V

with an acid of the formula R'-COOH or a functional derivative thereof, and

d) if desired, functionally modifying a reactive group present in the group M in a compound of formula I, and

e) if desired, converting a compound of formula I into a physiologically acceptable salt or converting a salt of a compound of formula I into the free acid or base.

**2.** A process according to claim 1 for the manufacture of guanidines of the formula

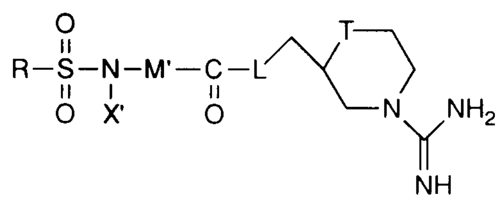

IA

wherein

-N(X')-M'- is an isoquinolylene group optionally substituted in the phenyl ring or

X' is H, $-CH_2COOH$, $-CH_2COO-C_{1-4}$-alkyl, $-CH_2CO$-(tetra- to heptamethyleneimino) or optionally N-mono- or N-di-$C_{1-4}$-alkylated $-CH_2CONH_2$ and

M' is a $R'-(CH_2)_{1-2}CH=$, $R'-COCH_2CH=$ or $-CH(CO-Q')CH_2-$ group,

Q' is benzylamino, morpholino or tetra- to heptamethyleneimino and

R, R', L and T have the same significances as in claim 1,

as well as hydrates or solvents and physiological usable salts thereof.

**3.** A process according to claim 2, wherein X' is H and R, M', L and T have the same significances as in claim 2.

**4.** A process according to claim 1 for the manufacture of guanidines of the formula

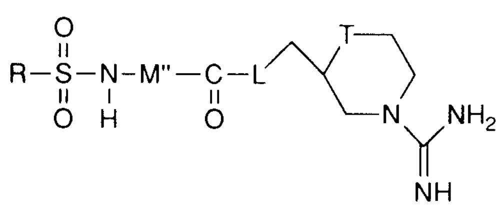

IB

wherein

M'' is a $R''-COCH_2CH=$, $R'-(CO)_{1-2}NHCH_2CH=$, benzyl-$OCONHCH_2CH=$, $-CH_2[R'-(CO)_{1-2}NH]CH-$, $-CH_2$(benzyl-OCONH)CH- or $-CH(CO-Q'')CH_2-$ group,

Q'' is a tetra- to heptamethyleneimino group optionally interrupted by an O or S atom and optionally substituted by up to 2 substituents from the group of $C_{1-4}$-alkyl, COOH, $-COO-C_{1-4}$-alkyl, $-CH_2OH$ and $-CH_2O$-benzyl and

R, R', R'', L and T have the same significances as given in claim 1.

5. A process according to claim 1, wherein R, L, T, M or -N(X)-M- and X have the same significances as in claim 1, but X is not H.

6. A process according to claim 1, wherein R is aryl, especially naphthyl, hydroxynaphthyl, 4-biphenyl, 2-anthryl, iodophenyl, nitrophenyl, benzyloxyphenyl, dimethoxyphenyl, 4-methoxy-2,3,6-trimethylpheny, 2,4,6-triisopropylphenyl, carboxyphenyl, methoxycarbonylphenyl, benzyloxynaphthyl, phenylsulphonylphenyl, hexahydroazepinoylphenyl or t-butylphenyl.

7. A process according to claim 1, wherein R is heteroaryl, especially 3-methyl-8-quinolyl, 5-(1-methyl-5-trifluoromethylpyrazol-3-yl)-2-thienyl or benzothienyl.

8. A process according to claim 1 or 2, wherein R is heterocyclyl, especially 3-methyl-1,2,3,4-tetrahydro-8-quinolyl.

9. A process according to claim 1, wherein -N(X)-M- is isoquinolylene or N-dimethylaminonaphthylsulphonylaminomethylene.

10. A process according to claim 1 or 2, wherein X is H or $-CH_2COOH$.

11. A process according to claim 1, wherein M is a $R'-(CH_2)_{1-2}CH=$ group, especially 3-indolylethylidene, 2,3-dioxo-1-indolinylethylidene, phenethylidene. 1,4-dioxo-5H-2,5-benzodiazepin-5-ylethylidene, (fluoro, chloro, iodo, cyano, nitro, amino, carboxy, $C_{1-4}$-alkoxycarbonyl or hydroxy)-phenethylidene, cyclohexylpropylidene, decalylethylidene, imidazolylethylidene, thienylethylidene, (methyl, bromo, fluoro or carboxymethyl)-3-indolylethylidene, naphthylethylidene, (ethoxycarbonylcarbonylamino, methoxycarbonylethylcarbonylamino, benzyloxycarbonylethylcarbonylamino, ethoxycarbonylamino, benzoylcarbonylamino, carboxybenzoylamino, methoxyethoxyacetamido, acetamido, carboxycarbonylamino, carboxypropionylamino, tolylsulphonamido, iodophenylsulphonamido, carboxyphenylsulphonamido or ethoxycarbonylmethylamino)phenethylidene, oxobenzoxazolinethylidene or 5-bromo- or 5-methyl-2,3-dioxo-1-indolinylethylidene.

12. A process according to claim 1, wherein M is a (R' or R'')$COCH_2CH=$ group, especially hexahydroazepinoylethylidene, (methoxycarbonyl or carboxy)-pyrrolidinoylethylidene, 3,4-dihydro-2(1H)-isoquinolinoylethylidene, (nitro, amino, iodo or formamido)benzoylethylidene, morpholinoethylidene, heptahydroazocinoylethylidene, (ethoxycarbonyl, acetoxymethyl, dimethylcarbamoyl, isobutyryloxymethyl or butyryloxymethyl)piperidinoylethylidene, 3-methoxycarbonyl-4-oxopiperidinoylethylidene or 4-acetoxy-3-ethoxycarbonylpiperidinoylethylidene.

13. A process according to claim 1, wherein M is a $R'-(CO)_{1-2}NHCH_2CH=$ group, especially benzoylcarboxamidoethylidene, thienoylcarboxamidoethylidene, benzoylamidoethylidene or benzyloxycarboxamidoethylidene.

14. A process according to claim 1, wherein M is a $-CH_2[R'-(CO)_{1-2}NH]CH-$ group, especially 2-(carboxybenzoylamido)ethylene, 2-(benzyloxybenzoylamido)ethylene, 2-(2-piperidinecarboxamido)-ethylene, 2-(hydroxybenzoylamido)ethylene or 2-(aminobenzoylamido)ethylene.

15. A process according to claim 1, wherein M is a $-CH(CO-Q)CH_2-$ group, especially 1-(benzylaminocarbonyl)ethylene, 1-(hexahydroazepinoyl)ethylene, 1-(morpholinoyl)ethylene, 1-(heptahydroazocinoyl)-ethylene, 1-[2-(benzyloxymethylmorpholinoyl)]ethylene, 1-[2-(hydroxymethylmorpholinoyl)]ethylene, 1-(2-ethoxycarbonyl-4-methylpiperidinoyl)ethylene, 1-(2-carboxy-4-methylpiperidinoyl)ethylene or 1-(3-carboxyhexahydro-1,4-oxazepinoyl)ethylene.

16. A process according to claim 1, 2 or 6, wherein R is aryl, especially naphthyl or nitro- or iodophenyl, L is NH and the asymmetric C atom in the piperidine or morpholine ring has the S-configuration.

**17.** A process according to claim 1, 11 or 12 for the manufacture of guanidines of the formula

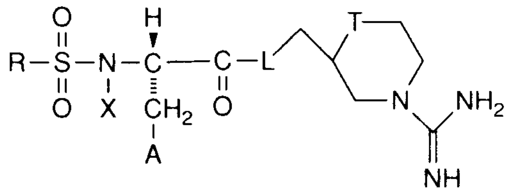

IC

wherein A is aryl, aroyl or heterocyclyl, especially phenyl, nitrophenyl, indolyl, 2,3-dioxo-1-indolinyl or aminobenzoyl.

**18.** A process according to claim 1 or 2 for the manufacture of guanidines from the following group:
(R)-N-[(RS)-1-Amidino-3-piperidinylmethyl]-α-(2-naphthylsulphonamido)-2,3-dioxo-1-indolinepropionamide,
(R)-N-[(RS)-1-amidino-3-piperidinylmethyl]-α-(2-naphthylsulphonamido)-o-nitrohydrocinnamamide,
(R)-N-[(RS)-1-amidino-3-piperidinylmethyl]-α-(o-nitrobenzenesulphonamido)indole-3-propionamide,
(R)-N-[(S)-1-amidino-3-piperidinylmethyl)-α-(p-iodobenzenesulphonamido)indole-3-propionamide,
(R)-N-[(S)-1-amidino-3-piperidinylmethyl]-α-(p-iodobenzenesulphonamido)-p-nitrohydrocinnamamide,
(R)-N-[(RS)-1-amidino-3-piperidinylmethyl]-3-(o-aminobenzoyl)-(2-naphthylsulphonamido)-propionamide,
N-[(R)-α-[[(S)-1-amidino-3-piperidinyl]methylcarbamoyl]phenethyl]-N-(2-naphthylsulphonyl)glycine,
(R)-N-[(S)-1-amidino-3-piperidinylmethyl]-1,2,3,4-tetrahydro-2-(2-naphthylsulphonyl)-3-isoquinolinecarboxamide,
(S)-N-[(RS)-1-amidino-3-piperidinylmethyl]hexahydro-β-(2-naphthylsulphonamido)-γ-oxo-1H-1-azepinebutyramide,
(R)-N-[(S)-1-amidino-3-piperidinylmethyl]-α-(2-naphthylsulphonamido)-2,3-dioxo-1-indolinepropionamide,
4'-[(R)-2-[[[(S)-1-amidino-3-piperidinyl]methyl]carbamoyl]-2-(2-naphthylsulphonamido)ethyl]oxanilic acid,
(S)-N-[[(RS)-1-amidino-3-piperidinyl]methyl]hexahydro-β-2-naphthylsulphonamido-γ-oxo-1(2H)-azocinebutyramide,
(2RS,4R)-1-[$N^4$-[[(S)-1-amidino-3-piperidinyl]methyl]-$N^2$-(2-naphthylsulphonyl)-L-asparaginyl]-4-methyl-2-piperidinecarboxylic acid,
4'-[(R)-2-[[[(S)-1-amidino-3-piperidinyl]methyl]carbamoyl]-2-(2-naphthylsulphonamido)ethyl]-succinanilidic acid.

**19.** The use of a compound according to any one of claims 1-18 for the manufacture of medicaments for the treatment or prophylaxis of illnesses which are caused by thrombin-induced platelet aggregation or the clotting of fibrinogen in blood plasma.

**Claims for the following Contracting State : GR**

**1.** A process for the manufacture of guanidines of the formula

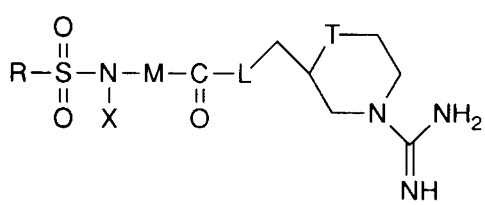

I

wherein
R is aryl, heteroraryl or heterocyclyl,

| | |
|---|---|
| T | is $CH_2$ or O, |
| L | is NH or O and |
| -N(X)-M- | is a $-N(SO_2-R^o)-CH_2-$ group or an isoquinolylene group optionally substituted in the phenyl ring and |
| $R^o$ | has the same significance as R or |
| X | is H, $-CH_2COOH$, $-CH_2COO-C_{1-4}$-alkyl, $-CH_2CO$-(tetra- to heptamethyleneimino) or optionally N-mono- or N-di-$C_{1-4}$-alkylated $-CH_2CONH_2$ and |
| M | is a $R'-(CH_2)_{1-2}CH=$, $R'-COCH_2CH=$, $R''-COCH_2CH=$, $R'-(CO)_{1-2}NHCH_2CH=$, benzyl-$OCONHCH_2CH=$, $-CH_2[R'-(CO)_{1-2}NH]CH-$, $-CH_2$(benzyl-OCONH)CH- or -CH(CO-Q)$CH_2$- group, |
| R' | is aryl, heteroaryl, cycloalkyl or heterocyclyl, |
| R'' | is tetra- to heptamethyleneimino optionally carrying up to 2 substituents from the group of oxo, $-COO-C_{1-4}$-alkyl, $-(CH_2)_{0-1}OH$, $-(CH_2)_{0-1}OCO-C_{1-4}$-alkyl and optionally mono- or di-$C_{1-4}$-alkylated carbamoyl and |
| Q | is benzylamino or a tetra- to heptamethyleneimino group optionally interrupted by an O or S atom and optionally substituted by up to 2 substituents from the group of $C_{1-4}$-alkyl, COOH, $-COO-C_{1-4}$-alkyl, $-CH_2OH$ and $-CH_2O$-benzyl, |

as well as hydrates or solvates and physiologically usable salts thereof, characterized by

a) reacting an acid of the formula

$R-SO_2N(X)-M-COOH$ II

with intermediary protection of a carboxy group present in the group X, R or M with an amine or alcohol of the formula

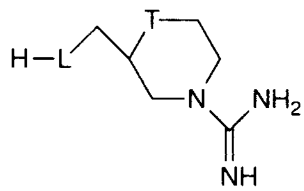

III

or a salt thereof, or

b) reacting a compound of the formula

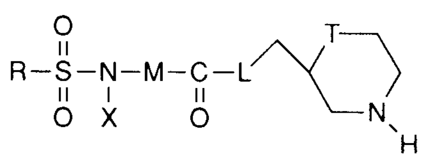

IV

with an amidinating reagent, or

c) reacting an amine of the formula

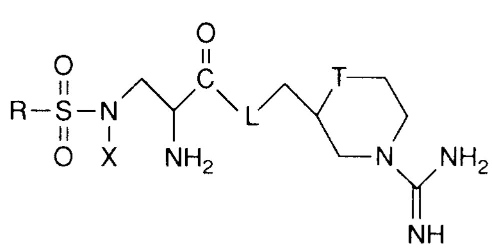

V

with an acid of the formula R'-COOH or a functional derivative thereof, and

d) if desired, functionally modifying a reactive group present in the group M in a compound of formula I, and

e) if desired, converting a compound of formula I into a physiologically acceptable salt or converting a salt of a compound of formula I into the free acid or base.

**2.** A process according to claim 1 for the manufacture of guanidines of the formula

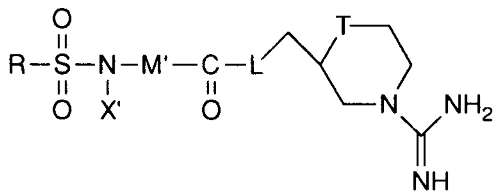

IA

wherein

| | |
|---|---|
| -N(X')-M'- | is an isoquinolylene group optionally substituted in the phenyl ring or |
| X' | is H, $-CH_2COOH$, $-CH_2COO-C_{1-4}$-alkyl, $-CH_2CO$-(tetra- to heptamethyleneimino) or optionally N-mono- or N-di-$C_{1-4}$-alkylated $-CH_2CONH_2$ and |
| M' | is a $R'-(CH_2)_{1-2}CH=$, $R'-COCH_2CH=$ or $-CH(CO-Q')CH_2-$ group, |
| Q' | is benzylamino, morpholino or tetra- to heptamethyleneimino and |
| R, R', L and T | have the same significances as in claim 1, |

as well as hydrates or solvents and physiological usable salts thereof.

**3.** A process according to claim 2, wherein X' is H and R, M', L and T have the same significances as in claim 2.

**4.** A process according to claim 1 for the manufacture of guanidines of the formula

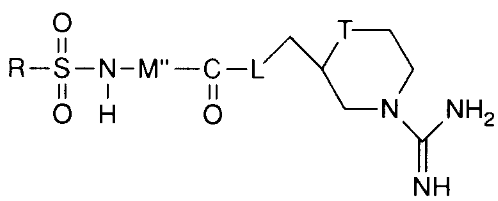

IB

wherein

| | |
|---|---|
| M'' | is a $R''-COCH_2CH=$, $R'-(CO)_{1-2}NHCH_2CH=$, benzyl-$OCONHCH_2CH=$, $-CH_2[R'-(CO)_{1-2}NH]CH-$, $-CH_2$(benzyl-OCONH)CH- or $-CH(CO-Q'')CH_2-$ group, |
| Q'' | is a tetra- to heptamethyleneimino group optionally interrupted by an O or S atom and optionally substituted by up to 2 substituents from the group of $C_{1-4}$-alkyl, COOH, $-COO-C_{1-4}$-alkyl, $-CH_2OH$ and $-CH_2O$-benzyl and |
| R, R', R'', L and T | have the same significances as given in claim 1. |

**5.** A process according to claim 1, wherein R, L, T, M or -N(X)-M- and X have the same significances as in claim 1, but X is not H.

**6.** A process according to claim 1, wherein R is aryl, especially naphthyl, hydroxynaphthyl, 4-biphenyl, 2-anthryl, iodophenyl, nitrophenyl, benzyloxyphenyl, dimethoxyphenyl, 4-methoxy-2,3,6-trimethylphenyl, 2,4,6-triisopropylphenyl, carboxyphenyl, methoxycarbonylphenyl, benzyloxynaphthyl, phenylsulphonylphenyl, hexahydroazepinoylphenyl or t-butylphenyl.

**7.** A process according to claim 1, wherein R is heteroaryl, especially 3-methyl-8-quinolyl, 5-(1-methyl-5-trifluoromethylpyrazol-3-yl)-2-thienyl or benzothienyl.

**8.** A process according to claim 1 or 2, wherein R is heterocyclyl, especially 3-methyl-1,2,3,4-tetrahydro-8-quinolyl.

**9.** A process according to claim 1, wherein -N(X)-M- is isoquinolylene or N-dimethylaminonaphthylsulphonylaminomethylene.

**10.** A process according to claim 1 or 2, wherein X is H or $-CH_2COOH$.

**11.** A process according to claim 1, wherein M is a $R'-(CH_2)_{1-2}CH=$ group, especially 3-indolylethylidene, 2,3-dioxo-1-indolinylethylidene, phenethylidene, 1,4-dioxo-5H-2,5-benzodiazepin-5-ylethylidene, (fluoro, chloro, iodo, cyano, nitro, amino, carboxy, $C_{1-4}$-alkoxycarbonyl or hydroxy)-phenethylidene, cyclohexylpropylidene, decalylethylidene, imidazolylethylidene, thienylethylidene, (methyl, bromo, fluoro or carboxymethyl)-3-indolylethylidene, naphthylethylidene, (ethoxycarbonylcarbonylamino, methoxycarbonylethylcarbonylamino, benzyloxycarbonylethylcarbonylamino, ethoxycarbonylamino, benzoylcarbonylamino, carboxybenzoylamino, methoxyethoxyacetamido, acetamido, carboxycarbonylamino, carboxypropionylamino, tolylsulphonamido, iodophenylsulphonamido, carboxyphenylsulphonamido or ethoxycarbonylmethylamino)phenethylidene, oxobenzoxazolinethylidene or 5-bromo- or 5-methyl-2,3-dioxo-1-indolinylethylidene.

**12.** A process according to claim 1, wherein M is a (R' or $R''(COCH_2CH=$ group, especially hexahydroazepinoylethylidene, (methoxycarbonyl or carboxy)-pyrrolidinoylethylidene, 3,4-dihydro-2(1H)-isoquinolinoylethylidene, (nitro, amino, iodo or formamido)benzoylethylidene, morpholinoethylidene, heptahydroazocinoylethylidene, (ethoxycarbonyl, acetoxymethyl, dimethylcarbamoyl, isobutyryloxymethyl or butyryloxymethyl)piperidinoylethylidene, 3-methoxycarbonyl-4-oxopiperidinoylethylidene or 4-acetoxy-3-ethoxycarbonylpiperidinoylethylidene.

**13.** A process according to claim 1, wherein M is a $R'-(CO)_{1-2}NHCH_2CH=$ group, especially benzoylcarboxamidoethylidene, thienoylcarboxamidoethylidene, benzoylamidoethylidene or benzyloxycarboxamidoethylidene.

**14.** A process according to claim 1, wherein M is a $-CH_2[R'-(CO)_{1-2}NH]CH-$ group, especially 2-(carboxybenzoylamido)ethylene, 2-(benzyloxybenzoylamido)ethylene, 2-(2-piperidinecarboxamido)-ethylene, 2-(hydroxybenzoylamido)ethylene or 2-(aminobenzoylamido)ethylene.

**15.** A process according to claim 1, wherein M is a $-CH(CO-Q)CH_2-$ group, especially 1-(benzylaminocarbonyl)ethylene, 1-(hexahydroazepinoyl)ethylene, 1-(morpholinoyl)ethylene, 1-(heptahydroazocinoyl)-ethylene, 1-[2-(benzyloxymethylmorpholinoyl)]ethylene, 1-[2-(hydroxymethylmorpholinoyl)]ethylene, 1-(2-ethoxycarbonyl-4-methylpiperidinoyl)ethylene, 1-(2-carboxy-4-methylpiperidinoyl)ethylene or 1-(3-carboxyhexahydro-1,4-oxazepinoyl)ethylene.

**16.** A process according to claim 1, 2 or 6, wherein R is aryl, especially naphthyl or nitro- or iodophenyl, L is NH and the asymmetric C atom in the piperidine or morpholine ring has the S-configuration.

**17.** A process according to claim 1, 11 or 12 for the manufacture of guanidines of the formula

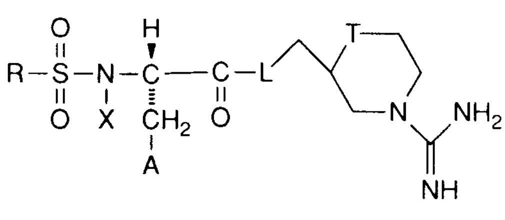

IC

wherein A is aryl, aroyl or heterocyclyl, especially phenyl, nitrophenyl, indolyl, 2,3-dioxo-1-indolinyl or aminobenzoyl.

**18.** A process according to claim 1 or 2 for the manufacture of guanidines from the following group:

(R)-N-[(RS)-1-Amidino-3-piperidinylmethyl]-α-(2-naphthylsulphonamido)-2,3-dioxo-1-indolinepropionamide,

(R)-N-[(RS)-1-amidino-3-piperidinylmethyl]-α-(2-naphthylsulphonamido)-o-nitrohydrocinnamamide,

(R)-N-[(RS)-1-amidino-3-piperidinylmethyl]-α-(o-nitrobenzenesulphonamido)indole-3-propionamide,

(R)-N-[(S)-1-amidino-3-piperidinylmethyl]-α-(p-iodobenzenesulphonamido)indole-3-propionamide,
(R)-N-[(S)-1-amidino-3-piperidinylmethyl]-α-(p-iodobenzenesulphonamido)-p-nitrohydrocinnamamide,
(R)-N-[(RS)-1-amidino-3-piperidinylmethyl]-3-(o-aminobenzoyl)-(2-naphthylsulphonamido)-propionamide,
N-[(R)-α-[[(S)-1-amidino-3-piperidinyl]methylcarbamoyl]phenethyl]-N-(2-naphthylsulphonyl)glycine,
(R)-N-[(S)-1-amidino-3-piperidinylmethyl]-1,2,3,4-tetrahydro-2-(2-naphthylsulphonyl)-3-isoquinolinecarboxamide,
(S)-N-[(RS)-1-amidino-3-piperidinylmethyl]hexahydro-β-(2-naphthylsulphonamido)-γ-oxo-1H-1-azepinebutyramide,
(R)-N-[(S)-1-amidino-3-piperidinylmethyl]-α-(2-naphthylsulphonamido)-2,3-dioxo-1-indolinepropionamide,
4'-[(R)-2-[[[(S)-1-amidino-3-piperidinyl]methyl]carbamoyol]-2-(2-naphthylsulphonamido)ethyl]oxanilic acid,
(S)-N-[[(RS)-1-amidino-3-piperidinyl]methyl]hexahydro-β-2-naphthylsulphonamido-γ-oxo-1(2H)-azocinebutyramide,
(2RS,4R)-1-[$N^4$-[[(S)-1-amidino-3-piperidinyl]methyl]-$N^2$-(2-naphthylsulphonyl)-L-asparaginyl]-4-methyl-2-piperidinecarboxylic acid,
4'-[(R)-2-[[[(S)-1-amidino-3-piperidinyl]methyl]carbamoyl]-2-(2-naphthylsulphonamido)ethyl]-succinanilidic acid.

**19.** A process for the manufacture of pharmaceutical preparations, especially for use as medicaments, particularly as inhibitors of platelet aggregation induced by thrombin and of the clotting of fibrinogen in blood plasma, characterized by bringing a guanidine according to claims 1-18 or a salt thereof into a galenical form.

**20.** The use of a compound according to any one of claims 1-18 for the manufacture of medicaments for the treatment or prophylaxis of illnesses which are caused by thrombin-induced platelet aggregation or the clotting of fibrinogen in blood plasma.

**21.** The compounds of formulae III, IV and V in accordance with claim 20.

**Revendications**

**Revendications pour les Etats contractants suivants : AT, BE, CH, DE, DK, FR, GB, IT, LI, LU, NL, SE**

**1.** Guanidines de formule

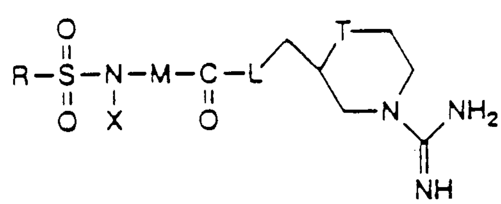

I

où

| | |
|---|---|
| R | représente un aryle, un hétéroaryle ou un hétérocyclyle, |
| T | représente $CH_2$ ou O, |
| L | représente NH ou O, et |
| -N(X)-M- | représente un groupe $-N(SO_2-R^o)-CH_2-$ ou un groupe isoquinolylène éventuellement substitué dans le cycle du phényle, et |
| $R^o$ | a la même signification que R, ou |
| X | représente H, $-CH_2COOH$, $-CH_2COO$-alkyle en $C_{1-4}$-, $-CH_2CO$-(de tétra-à heptaméthylèneimino) ou $-CH_2CONH_2$, éventuellement alkylé sur N par un ou deux alkyles en $C_{1-4}$, et |
| M | représente un groupe $R'-(CH_2)_{1-2}CH=$, $R'-COCH_2CH=$, $R''-COCH_2CH=$, $R'-(CO)_{1-2}NHCH_2CH=$, benzyl-$OCONHCH_2CH=$, $-CH_2[R'-(CO)_{1-2}NH]CH-$, $-CH_2$(benzyl-OCONH)CH- ou $-CH(CO-Q)CH_2-$, |

| | |
|---|---|
| R' | représente un aryle, un hétéroaryle, le cycloalkyle ou un hétérocyclyle, |
| R'' | représente le tétra- jusqu'à heptaméthylèneimino ayant éventuellement jusqu'à 2 substituants choisis dans le groupe formé par l'oxo, -COO-alkyle en $C_{1-4}$, $-(CH_2)_{0-1}OH$, $-(CH_2)_{0-1}OCO$-alkyle en $C_{1-4}$ et le carbamoyle éventuellement alkylé par un ou deux alkyles en $C_{1-4}$, et |
| Q | représente le benzylamino ou un groupe de tetra à heptaméthylèneimino éventuellement interrompu par un atome de O ou de S et éventuellement substitué par jusqu'à 2 substituants choisis dans le groupe constitué par les alkyles en $C_{1-4}$, COOH, -COO-alkyle en $C_{1-4}$, $-CH_2OH$ et $-CH_2O$-benzyle, |

ainsi que leurs hydrates ou leurs solvates et leurs sels physiologiquement utilisables.

**2.** Guanidines selon la revendication 1 et selon la formule

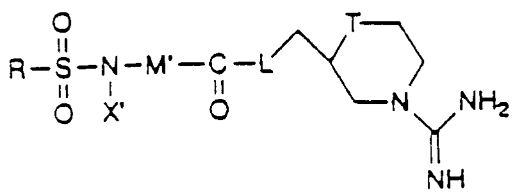

IA

dans laquelle

| | |
|---|---|
| -N(X')-M- | est un groupe isoquinolylène éventuellement substitué dans le cycle du phényle, ou |
| X' | représente H, $-CH_2COOH$, $-CH_2COO$-alkyle en $C_{1-4}$, $-CH_2CO$-(de tétra- à heptaméthylèneimino) ou $-CH_2CONH_2$ éventuellement alkylé sur N par un ou deux alkyles en $C_{1-4}$, et |
| M' | représente un groupe $R'-(CH_2)_{1-2}CH=$, $R'-COCH_2CH=$ ou $-CH(CO-Q')CH_2-$, |
| Q' | représente le benzylamino, le morpholino ou le tétra- jusqu'à heptaméthylèneimino, et |
| R, R' L et T | ont les mêmes significations que dans la revendication 1, |

ainsi que leurs hydrates ou leurs solvates et leurs sels physiologiquement utilisables.

**3.** Composés selon la revendication 2, où X' est H, et R, M', L et T ont les mêmes significations que dans la revendication 2.

**4.** Guanidines selon la revendication 1 et selon la formule

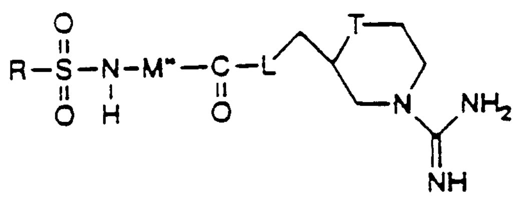

IB

où

| | |
|---|---|
| M'' | représente un groupe $R''-COCH_2CH=$, $R'-(CO)_{1-2}NHCH_2CH=$, benzyl-$OCONHCH_2CH=$,$-CH_2[R'-(CO)_{1-2}NH]CH-$,$-CH_2$(benzyl-OCONH)CH-ou-CH(CO-Q'')$CH_2$-, |
| Q'' | représente un groupe de tétra- à heptaméthylèneimino éventuellement interrompu par un atome de O ou de S et éventuellement substitué par jusqu'à 2 substituants choisis dans le groupe constitué par les alkyles en $C_{1-4}$, COOH, -COO-alkyle en $C_{1-4}$, $-CH_2OH$ et $-CH_2O$-benzyle, et |
| R, R', R'', L et T | ont les mêmes significations que dans la revendication 1. |

**5.** Guanidines selon la revendication 1, où R, L, T, M ou -N(X)-M- et X ont les mêmes significations que dans la revendication 1, mais X n'est pas H.

**6.** Guanidines selon la revendication 1, où R est un aryle, en particulier le naphtyle, l'hydroxynaphtyle, le 4-biphényle, le 2-anthryle, le iodophényle, le nitrophényle, le berryloxyphényle, le diméthoxyphényle, le 4-méthoxy,-2,3,6-triméthylphényle, le 2,4,6-triisopropylphényle, le carboxyphényle, le méthoxycarbonylphényle, le benzyloxynaphtyle, le phénylsulfonylphényle, l'hexahydroazépinoylphényle ou le t-butylphényle.

**7.** Guanidines selon la revendication 1, où R est un hétéroaryle, en particulier le 3-méthyl-8-quinolyle, le 5-(1-méthyl-5-trifluorométhylpyrazole-3-yl)-2-thiényle ou le benzothiényle.

**8.** Guanidines selon la revendication 1 ou 2, où R est un hétérocyclyle, en particulier le 3-méthyl-1,2,3,4-tétrahydro-8-quinolyle.

**9.** Guanidines selon la revendication 1, où -N(X)-M est l'isoquinolylène ou le N-diméthylaminonaphtylsulfonyl-aminométhylène.

**10.** Guanidines selon la revendication 1 ou 2, où X est H ou $-CH_2COOH$.

**11.** Guanidines selon la revendication 1, où M est un groupe $R'-(CH_2)_{1-2}CH=$, en particulier le 3-indolyléthylidène, le 2,3-dioxo-1-indolinyléthylidène, le phénéthylidène, le 1,4-dioxo-5H-2,5-benzodiazépine-5-yléthylidène, le (fluoro, chloro, iodo, cyano, nitro, amino, carboxy, alcoxy en $C_{1-4}$-carbonyle ou hydroxy)phénéthylidène, le cyclohexylpropylidène, le décalyléthylidène, l'imidazolyléthylidène, le thiényléthylidène, le (méthyl, bromo, fluoro ou carboxyméthyl)-3-indolyléthylidène le naphtyléthylidène, le (éthoxycarbonylcarbonylamino, méthoxycarbonyléthylcarbonylamino, benzyloxycarbonyléthylcarbonylamino, éthoxycarbonylamino, benzoylcarbonylamino, carboxybenzoylamino, méthoxyéthoxyacétamido, acétamido, carboxycarbonylamino, carboxypropionylamino, tolylsulfonamido, iodophénylsulfonamido, carboxyphénylsulfonamido ou éthoxycarbonylméthylamino)phénéthylidène, l'oxobenzoxazolinéthylidène ou le 5-bromo- ou 5-méthyl-2,3-dioxo-1-indolinyléthylidène.

**12.** Guanidines selon la revendication 1, où M est un groupe (R' ou R'')$COCH_2CH=$, en particulier l'hexahydroazépinoyléthylidène, le (méthoxycarbonyl ou carboxy)pyrrolidinoyléthylidène, le 3,4-dihydro-2(1H)-isoquinoléinoyléthylidène, le (nitro, amino, iodo ou formamido)benzoyléthylidène, le morpholinoéthylidène, l'heptahydroazocinoyléthylidène, le (éthoxycarbonyl, acétoxyméthyl, diméthylcarbamoyl, isobutyryloxyméthyl ou butyryloxyméthyl)pipéridinoyléthylidène, le 3-méthoxycarbonyl-4-oxopipéridinoyléthylidène ou le 4-acétoxy-3-éthoxycarbonylpipéridinoyléthylidène.

**13.** Guanidines selon la revendication 1, où M est un groupe $R'-(CO)_{1-2}NHCH_2CH=$, en particulier le benzoylcarboxamidoéthylidène, le thiénoylcarboxamidoéthylidène, le benzoylamidoéthylidène ou le benzyloxycarboxamidoéthylidène.

**14.** Guanidines selon la revendication 1, où M est un groupe $-CH_2[R'-(CO)_{1-2}NH]CH-$, en particulier le 2-(carboxybenzoylamido)éthylène, le 2-(benzyloxybenzoylamido)éthylène, le 2-(2-pipéridinecarboxamido)-éthylène, le 2-(hydroxybenzoylamido)éthylène ou le 2-(aminobenzoylamido)éthylène.

**15.** Guanidines selon la revendication 1, où M est un groupe$-CH(CO-Q)CH_2-$, en particulier le 1-(benzylaminocarbonyl)éthylène, le 1-(hexahydroazépinoyl)-éthylène, le 1-(morpholinoyl)éthylène, le 1-(heptahydroazocinoyl)éthylène, le 1-[2-(benzyloxyméthylmorpholinoyl)]éthylène, le 1-[2-(hydroxyméthylmorpholinoyl)éthylène, le 1-(2-éthoxycarbonyl-4-méthylpipéridinoyl)éthylène, le 1-(2-carboxy-4-méthylpipéridinoyl)éthylène ou le 1-(3-carboxyhexahydro-1,4-oxazépinoyl )éthylène.

**16.** Guanidines selon la revendication 1, 2 ou 6, où R est un aryle, en particulier le naphtyle ou le nitro- ou iodophényle, L est NH, et où l'atome de C asymétrique dans le cycle de la pipéridine ou de la morpholine a la configuration S.

**17.** Guanidines selon la revendication 1, 11 ou 12 et selon la formule

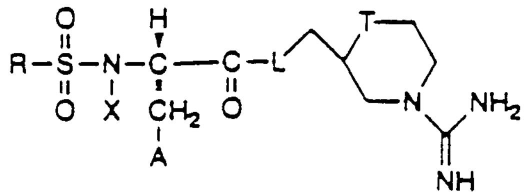

IC

où A est un aryle, un aroyle ou un hétérocyclyle, en particulier le phényle, le nitrophényle, l'indolyle, le 2,3-dioxo-1-indolinyle ou l'aminobenzoyle.

**18.** Guanidines selon la revendication 1 ou 2, choisies à partir du groupe des composés suivants :
le (R)-N-[(RS)-1-amidino-3-pipéridinylméthyl]-α-(2-naphtylsulfonamido)-2,3-dioxo-1-indolinepropionamide,
le (R)-N-[(RS)-1-amidino-3-pipéridinylméthyl]-α-(2-naphtylsulfonamido)-o-nitrohydrocinnamamide,
le (R)-N-[(RS)-1-amidino-3-pipéridinylméthyl]-α-(o-nitrobenzènesulfonamido)indole-3-propionamide,
le (R)-N-[(S)-1-amidino-3-pipéridinylméthyl]-α-(p-iodobenzènesulfonamido)-indole-3-propionamide,
le (R)-N-[(S)-1-amidino-3-pipéridinylméthyl]-α-(p-iodobenzènesulfonamido)-p-nitrohydrocinnamamide,
le (R)-N-[(RS)-1-amidino-3-pipéridinylméthyl]-3-(o-aminobenzoyl)-(2-naphtylsulfonamido)propionamide,
la N-[(R)-α-[[(S)-1-amidino-3-pipéridinyl]méthylcarbamoyl]phénéthyl]-N-(2-naphtylsulfonyl)glycine,
le (R)-N-[(S)-1-amidino-3-pipéridinylméthyl]-1,2,3,4-tétrahydro-2-(2-naphtylsulfonyl)-3-isoquinoléinecarboxamide,
le (S)-N-[(RS)-1-amidino-3-pipéridinylméthyl]hexahydro-β-( 2-naphtylsulfonamido)-γ-oxo-1H-1-azépinebutyramide,
le (R)-N-[(S)-1-amidino-3-pipéridinylméthyl]-α-(2-naphtylsulfonamido)-2,3-dioxo-1-indolinepropionamide.
l'acide 4'-[(R)-2-[[[(S)-1-amidino-3-pipéridinyl]méthyl]carbamoyl]-2-(2-naphtylsulfonamido)éthyl]-oxanilique,
le (S)-N-[[(RS)-1-amidino-3-pipéridinyl]méthyl]hexahydro-β-2-naphtylsulfonamido-γ-oxo-1(2H)-azocinbutyramide,
l'acide (2RS,4R)-1-[$N^4$-[[(S)-1-amidino-3-pipéridinyl]méthyl]-$N^2$-(2 naphtylsulfonyl)-L-asparaginyl]-4-méthyl-2-pipéridinecarboxylique,
l'acide 4'-[(R)-2-[[[(S)-1-amidino-3-pipéridinyl]méthyl]carbamoyl]-2-(2-naphtylsulfonamido)éthyl]-succinanilique.

**19.** Composés selon une des revendications 1 à 18 pour utilisation comme médicament, en particulier pour inhiber l'agrégation des plaquettes induite par la thrombine et la coagulation du fibrinogène dans le plasma du sang.

**20.** Procédé pour préparer les composés selon une des revendications 1 à 18, caractérisé par les points suivants :

a) on fait réagir un acide de formule

$R-SO_2N(X)-M-COOH$ II

en protégeant de façon intermédiaire un groupe carboxy contenu dans le groupe X, R ou M, avec une amine ou un alcool de formule

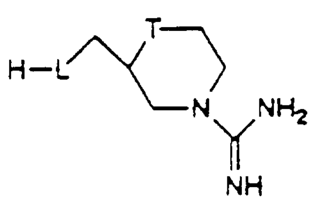

III

ou un sel de ceux-ci, ou

b) on fait réagir un composé de formule

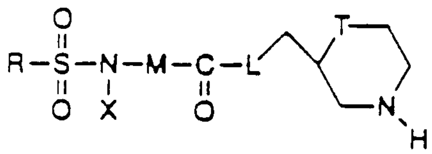

IV

avec un réactif pour former un amide, ou

c) on fait réagir une amine de formule

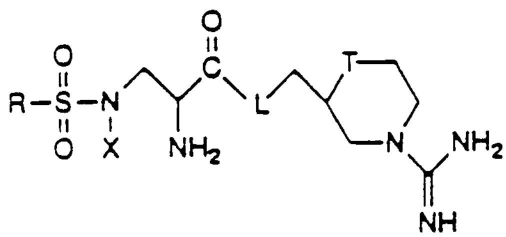

V

avec un acide de formule R'-COOH ou un dérivé fonctionnel de celui-ci, et

d) si on le désire, on modifie de façon fonctionnelle un groupe réactif contenu dans le groupe M d'un composé de formule I, et

e) si on le désire, on transforme un composé de formule I en un sel acceptable physiologiquement ou on transforme un sel d'un composé de formule I en acide libre ou en base libre.

**21.** Préparation pharmaceutique comportant un composé selon une des revendications 1 à 18 comme matière active.

**22.** Utilisation d'un composé selon une des revendications 1 à 18 pour préparer des médicaments pour le traitement ou la prophylaxie de maladies qui sont provoquées par l'agrégation des plaquettes induite par la thrombine ou la coagulation du fibrinogène dans le plasma du sang.

**23.** Les composés des formules III, IV et V selon la revendication 20.

**Revendications pour l'Etat contractant suivant : ES**

**1.** Procédé pour préparer les guanidines de formule

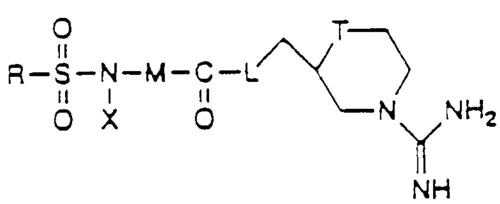

I

où

| | |
|---|---|
| R | représente un aryle, un hétéroaryle ou un hétérocyclyle, |
| T | représente $CH_2$ ou O, |
| L | représente NH ou O, et |
| -N(X)-M- | représente un groupe -N( $SO_2$-R°)-$CH_2$- ou un groupe isoquinolylène éventuellement substitué dans le cycle du phényle, et |
| R° | a la même signification que R, ou |
| X | représente H, -$CH_2$COOH, -$CH_2$COO-alkyle en $C_{1-4}$-, -$CH_2$CO-(de tétra-à heptaméthylèneimino) ou -$CH_2$CONH$_2$, éventuellement alkylé sur N par un ou deux alkyles en $C_{1-4}$, et |

M représente un groupe $R'-(CH_2)_{1-2}CH=$, $R'-COCH_2CH=$, $R''-COCH_2CH=$, $R'-(CO)_{1-2}NHCH_2CH=$, benzyl-$OCONHCH_2CH=$, $-CH_2[R'-(CO)_{1-2}NH]CH-$, $-CH_2$(benzyl-OCONH)CH- ou $-CH(CO-Q)CH_2-$,

R' représente un aryle, un hétéroaryle, le cycloalkyle ou un hétérocyclyle,

R'' représente le tétra-jusqu'à heptaméthylèneimino ayant éventuellement jusqu'à 2 substituants choisis dans le groupe formé par l'oxo, -COO-alkyle en $C_{1-4}$, $-(CH_2)_{0-1}OH$, $-(CH_2)_{0-1}OCO$-alkyle en $C_{1-4}$ et le carbamoyle éventuellement alkylé par un ou deux alkyles en $C_{1-4}$, et

Q représente le benzylamino ou un groupe de tétra à heptaméthylèneimino éventuellement interrompu par un atome de O ou de S et éventuellement substitué par jusqu'à 2 substituants choisis dans le groupe constitué par les alkyles en $C_{1-4}$, COOH, -COO-alkyle en $C_{1-4}$, $-CH_2OH$ et $-CH_2O$-benzyle,

ainsi que leurs hydrates ou leurs solvates et leurs sels physiologiquement utilisables, caractérisé par les points suivants :

a) on fait réagir un acide de formule

$R-SO_2N(X)-M-COOH$ II

en protégeant de façon intermédiaire un groupe carboxy contenu dans le groupe X, R ou M, avec une amine ou un alcool de formule

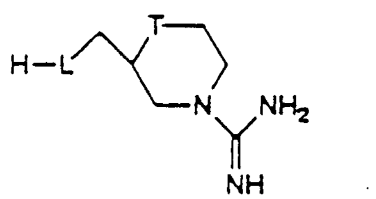

III

ou un sel de ceux-ci, ou

b) on fait réagir un composé de formule

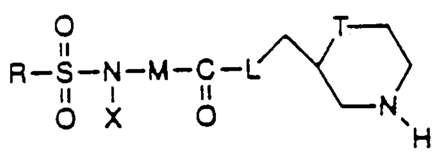

IV

avec un réactif pour former un amide, ou

c) on fait réagir une amine de formule

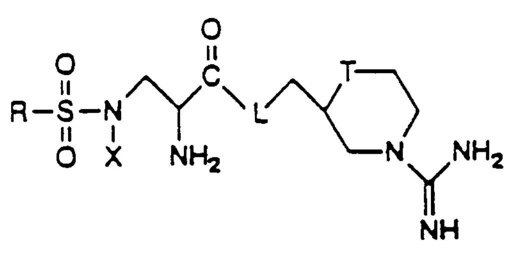

V

avec un acide de formule R'-COOH ou un dérivé fonctionnel de celui-ci, et

d) si on le désire, on modifie de façon fonctionnelle un groupe réactif contenu dans le groupe M d'un composé de formule I, et

e) si on le désire, on transforme un composé de formule I en un sel acceptable physiologiquement ou on transforme un sel d'un composé de formule I en acide libre ou en base libre.

2. Procédé selon la revendication 1 pour préparer les guanidines de formule

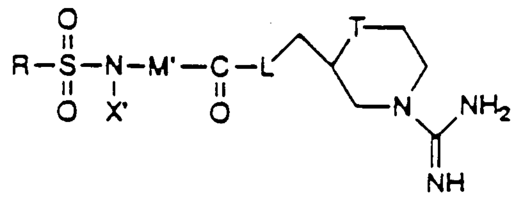

IA

dans laquelle

| | |
|---|---|
| -N(X')-M- | est un groupe isoquinolylène éventuellement substitué dans le cycle du phényle, ou |
| X' | représente H, $-CH_2COOH$, $-CH_2COO$-alkyle en $C_{1-4}$ $-CH_2CO$-(de tétra- à heptaméthylèneimino) ou $-CH_2CONH_2$ éventuellement alkylé sur N par un ou deux alkyles en $C_{1-4}$, et |
| M' | représente un groupe $R'-(CH_2)_{1-2}CH=$, $R'-COCH_2CH=$ ou -CH(CO-Q')CH2-, |
| Q' | représente le benzylamino, le morpholino ou le tétra-jusqu'à heptaméthylèneimino, et |
| R, R', L et T | ont les mêmes significations que dans la revendication 1, |

ainsi que leurs hydrates ou leurs solvates et leurs sels physiologiquement utilisables.

3. Procédé selon la revendication 2, où X' est H, et R, M', L et T ont les mêmes significations que dans la revendication 2.

4. Procédé selon la revendication 1 pour préparer les guanidines de formule

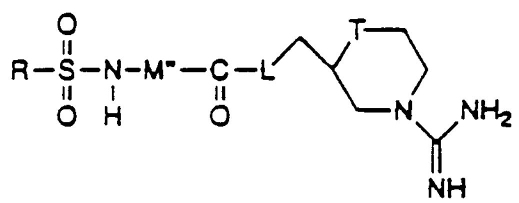

IB

où

| | |
|---|---|
| M'' | représente un groupe $R''-COCH_2CH=$, $R'-(CO)_{1-2}NHCH_2CH=$, benzyl-$OCONHCH_2CH=$, $-CH_2[R'-(CO)_{1-2}NH]CH-$, $-CH_2$(benzyl-OCONH)CH- ou -CH-(CO-Q'')$CH_2$-, |
| Q'' | représente un groupe de tétra- à heptaméthylèneimino éventuellement interrompu par un atome de O ou de S et éventuellement substitué par jusqu'à 2 substituants choisis dans le groupe constitué par les alkyles en $C_{1-4}$, COOH, -COO-alkyle en $C_{1-4}$,$-CH_2OH$ et $-CH_2O$-benzyle, et |
| R, R', R'' L et T | ont les mêmes significations que dans la revendication 1. |

5. Procédé selon la revendication 1, où R, L, T, M ou -N(X)-M- et X ont les mêmes significations que dans la revendication 1, mais X n'est pas H.

6. Procédé selon la revendication 1, où R est un aryle, en particulier le naphtyle, l'hydroxynaphtyle, le 4-biphényle, le 2-anthryle, le iodophényle, le nitrophényle, le benzyloxyphényle, le diméthoxyphényle, le 4-méthoxy-2,3,6-triméthylphényle, le 2,4,6-triisopropylphényle, le carboxyphényle, le méthoxycarbonylphényle, le benzyloxynaphtyle, le phénylsulfonylphényle, l'hexahydroazépinoylphényle ou le t-butylphényle.

7. Procédé selon la revendication 1, où R est un hétéroaryle, en particulier le 3-méthyl-8-quinolyle, le 5-(1-méthyl-5-trifluorométhylpyrazole-3-yl)-2-thiényle ou le benzothiényle.

8. Procédé selon la revendication 1 ou 2, où R est un hétérocyclyle, en particulier le 3-méthyl-1,2,3,4-tétrahydro-8-quinolyle.

**9.** Procédé selon la revendication 1, où -N(X)-M- est l'isoquinolylène ou le N-diméthylaminonaphtylsulfonyl-aminométhylène.

**10.** Procédé selon la revendication 1 ou 2, où X est H ou $-CH_2COOH$.

**11.** Procédé selon la revendication 1, où M est un groupe $R'-(CH_2)_{1-2}CH=$, en particulier le 3-indolyléthylidène, le 2,3-dioxo-1-indolinyléthylidène, le phénéthylidène, le 1,4-dioxo-5H-2,5-benzodiazépine-5-yléthylidène, le (fluoro, chloro, iodo, cyano, nitro, amino, carboxy, alcoxy en $C_{1-4}$-carbonyle ou hydroxy)-phénéthylidène, le cyclohexylpropylidène, le décalyléthylidène, l'imidazolyléthylidène, le thiényléthylidène, le (méthyl, bromo, fluoro ou carboxyméthyl)-3-indolyléthylidène, le naphtyléthylidène, le (éthoxycarbonylcarbonylamino, méthoxycarbonyléthylcarbonylamino, benzyloxycarbonyléthylcarbonylamino, éthoxycarbonylamino, benzoylcarbonylamino, carboxybenzoylamino, méthoxyéthoxyacétamido, acétamido, carboxycarbonylamino, carboxypropionylamino, tolylsulfonamido, iodophénylsulfonamido, carboxyphénylsulfonamido ou éthoxycarbonylméthylamino)phénéthylidène, l'oxobenzoxazolinéthylidène ou le 5-bromo- ou 5-méthyl-2,3-dioxo-1-indolinyléthylidène.

**12.** Procédé selon la revendication 1, où M est un groupe (R' ou R")$COCH_2CH=$, en particulier l'hexahydroazépinoyléthylidène, le (méthoxycarbonyl ou carboxy)pyrrolidinoyléthylidène, le 3,4-dihydro-2(1H)-isoquinoléinoyléthylidène, le (nitro, amino, iodo ou formamido)benzoyléthylidène, le morpholinoéthylidène, l'heptahydroazocinoyléthylidène, le (éthoxycarbonyl, acétoxyméthyl, diméthylcarbamoyl, isobutyryloxyméthyl ou butyryloxyméthyl)pipéridinoyléthylidène, le 3-méthoxycarbonyl-4-oxopipéridinoyléthylidène ou le 4-acétoxy-3-éthoxycarbonylpipéridinoyléthylidène.

**13.** Procédé selon la revendication 1, où M est un groupe $R'-(CO)_{1-2}NHCH_2CH=$, en particulier le benzoylcarboxamidoéthylidène, le thiénoylcarboxamidoéthylidène, le benzoylamidoéthylidène ou le benzyloxycarboxamidoéthylidène.

**14.** Procédé selon la revendication 1, où M est un groupe $-CH_2[R'-(CO)_{1-2}NH[CH-$, en particulier le 2-(carboxybenzoylamido)éthylène, le 2-(benzyloxybenzoylamido)éthylène, le 2-(2-pipéridinecarboxamido)-éthylène, le 2-(hydroxybenzoylamido)éthylène ou le 2-(aminobenzoylamido)éthylène.

**15.** Procédé selon la revendication 1, où M est un groupe-CH(CO-Q)$CH_2$-, en particulier le 1-(benzylaminocarbonyl)éthylène, le 1-(hexahydroazépinoyl)-éthylène, le 1-(morpholinoyl)éthylène, le 1-(heptahydroazocinoyl)éthylène, le 1-[2-(benzyloxyméthylmorpholinoyl)]éthylène, le 1-[2-(hydroxyméthylmorpholinoyl)éthylène, le 1-(2-éthoxycarbonyl-4-méthylpipéridinoyl)éthylène, le 1-(2-carboxy-4-méthylpipéridinoyl)éthylène ou le 1-(3-carboxyhexahydro-1,4-oxazépinoyl)éthylène.

**16.** Procédé selon la revendication 1, 2 ou 6, où R est un aryle, en particulier le naphtyle ou le nitro- ou iodophényle, L est NH, et où l'atome de C asymétrique dans le cycle de la pipéridine ou de la morpholine a la configuration S.

**17.** Procédé selon la revendication 1, 11 ou 12 pour préparer les guanidines de formule

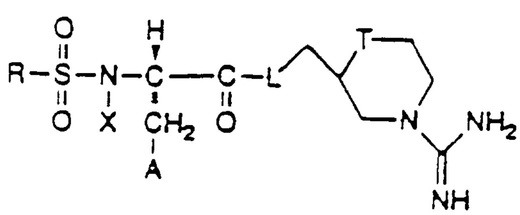

IC

où A est un aryle, un aroyle ou un hétérocyclyle, en particulier le phényle, le nitrophényle, l'indolyle, le 2,3-dioxo-1-indolinyle ou l'aminobenzoyle.

**18.** Procédé selon la revendication 1 ou 2, pour préparer les guanidines choisies à partir du groupe des composés suivants :

le (R)-N-[(RS)-1-amidino-3-pipéridinylméthyl]-α-(2-naphtylsulfonamido)-2,3-dioxo-1-indolinepropionamide,
le (R)-N-[(RS)-1-amidino-3-pipéridinylméthyl]-α-(2-naphtylsulfonamido)-o-nitrohydrocinnamamide,
le (R)-N-[(RS)-1-amidino-3-pipéridinylméthyl]-α-(o-nitrobenzènesulfonamido)indole-3-propionamide,
le (R)-N-[(S)-1-amidino-3-pipéridinylméthyl]-α-(p-iodobenzènesulfonamido)-indole-3-propionamide,
le (R)-N-[(S)-1-amidino-3-pipéridinylméthyl]-α-(p-iodobenzènesulfonamido)-p-nitrohydrocinnamamide,
le (R)-N-[(RS)-1-amidino-3-pipéridinylméthyl]-3-(o-aminobenzoyl)-(2-naphtylsulfonamido)propionamide,
la N-[(R)-α-[[(S)-1-amidino-3-pipéridinyl]méthylcarbamoyl]phénéthyl]-N-(2-naphtylsulfonyl)glycine,
le (R)-N-[(S)-1-amidino-3-pipéridinylméthyl]-1,2,3,4-tétrahydro-2-(2-naphtylsulfonyl)-3-isoquinoléinecarboxamide,
le (S)-N-[(RS)-1-amidino-3-pipéridinylméthyl]hexahydro-β-(2-naphtylsulfonamido)-γ-oxo-1H-1-azépinebutyramide,
le (R)-N-[(S)-1-amidino-3-pipéridinylméthyl]-α-(2-naphtylsulfonamido)-2,3-dioxo-1-indolinepropionamide,
l'acide 4'-[(R)-2-[[[(S)-1-amidino-3-pipéridinyl]méthyl]carbamoyl]-2-(2-naphtylsulfonamido)éthyl]-oxanilique,
le (S)-N-[[(RS)-1-amidino-3-pipéridinyl]méthyl]hexahydro-β-2-naphtylsulfonamido-γ-oxo-1(2H)-azocinbutyramide,
l'acide (2RS,4R)-1-[$N^4$-[[(S)-1-amidino-3-pipéridinyl]méthyl]-$N^2$-(2-naphtylsulfonyl)-L-asparaginyl]-4-méthyl-2-pipéridinecarboxylique,
l'acide 4'-[(R)-2-[[[(S)-1-amidino-3-pipéridinyl]méthyl]carbamoyl]-2-(2-naphtylsulfonamido)éthyl]-succinanilique.

**19.** Utilisation d'un composé selon une des revendications 1 à 18 pour préparer des médicaments pour le traitement ou la prophylaxie de maladies qui sont provoquées par l'agrégation des plaquettes induite par la thrombine ou la coagulation du fibrinogène dans le plasma du sang.

**Revendications pour l'Etat contractant suivant : GR**

**1.** Procédé pour préparer les guanidines de formule

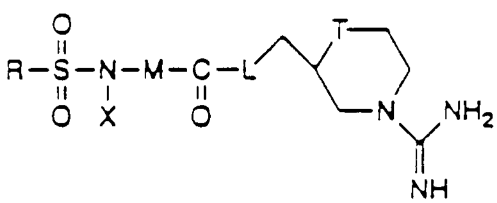

I

où

| | |
|---|---|
| R | représente un aryle, un hétéroaryle ou un hétérocyclyle, |
| T | représente $CH_2$ ou O, |
| L | représente NH ou O, et |
| -N(X)-M- | représente un groupe $-N(SO_2-R^o)-CH_2-$ ou un groupe isoquinolylène éventuellement substitué dans le cycle du phényle, et |
| $R^o$ | a la même signification que R, ou |
| X | représente H, $-CH_2COOH$, $-CH_2COO$-alkyle en $C_{1-4}$, $-CH_2CO$-(de tétra-à heptaméthylèneimino) ou $-CH_2CONH_2$ éventuellement alkylé sur N par un ou deux alkyles en $C_{1-4}$, et |
| M | représente un groupe $R'-(CH_2)_{1-2}CH=$, $R'-COCH_2CH=$, $R''-COCH_2CH=$, $R'-(CO)_{1-2}NHCH_2CH$, benzyl-$OCONHCH_2CH=$ $-CH_2[R'-(CO)_{1-2}NH]CH-$, $-CH_2$(benzyl-OCONH)CH- ou -CH(CO-Q)$CH_2$-, |
| R' | représente un aryle, un hétéroaryle, le cycloalkyle ou un hétérocyclyle, |
| R'' | représente le tétra- jusqu'à heptaméthylèneimino ayant éventuellement jusqu'à 2 substituants choisis dans le groupe formé par l'oxo, -COO-alkyle en $C_{1-4}$, $-(CH_2)_{0-1}OH$, $-(CH_2)_{0-1}OCO$-alkyle en $C_{1-4}$ et le carbamoyle éventuellement alkylé par un ou deux alkyles en $C_{1-4}$, et |
| Q | représente le benzylamino ou un groupe de tétra à heptaméthylèneimino éventuellement interrompu par un atome de O ou de S et éventuellement substitué par jusqu'à 2 substituants choisis dans le groupe constitué par les alkyles en $C_{1-4}$, COOH, -COO- |

alkyle en $C_{1-4}$, $-CH_2OH$ et $-CH_2O$-benzyle,

ainsi que leurs hydrates ou leurs solvates et leurs sels physiologiquement utilisables, caractérisé par les points suivants :

a) on fait réagir un acide de formule

$R-SO_2N(X)-M-COOH$ II

en protégeant de façon intermédiaire un groupe carboxy contenu dans le coupe X, R ou M avec une amine ou un alcool de formule

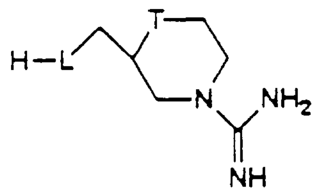

III

ou un sel de ceux-ci, ou

b) on fait réagir un composé de formule

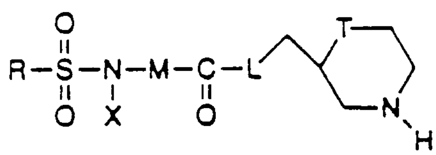

IV

avec un réactif pour former un amide, ou

c) on fait réagir une amine de formule

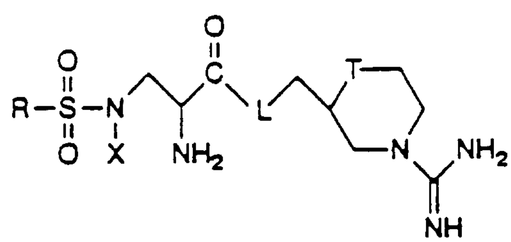

V

avec un acide de formule R'-COOH ou un dérivé fonctionnel de celui-ci, et

d) si on le désire, on modifie de façon fonctionnelle un groupe réactif contenu dans le groupe M d'un composé de formule I, et

e) si on le désire, on transforme un composé de formule I en un sel acceptable physiologiquement ou on transforme un sel d'un composé de formule I en acide libre ou en base libre.

**2.** Procédé selon la revendication 1 pour préparer les guanidines de formule

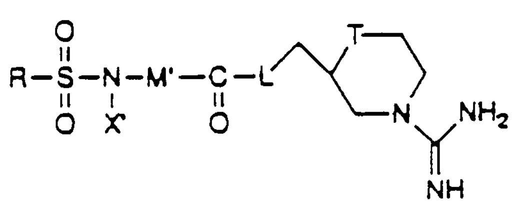

IA

dans laquelle

| | |
|---|---|
| -N(X')-M- | est un groupe isoquinolylène éventuellement substitué dans le cycle du phényle, ou |
| X' | représente H, $-CH_2COOH$, $-CH_2COO$-alkyle en $C_{1-4}$, $-CH_2CO$-(de tétra- à heptaméthylèneimino) ou $-CH_2CONH_2$ éventuellement alkylé sur N par un ou deux alkyles en $C_{1-4}$, et |
| M' | représente un groupe $R'-(CH_2)_{1-2}CH=$, $R'-COCH_2CH=$ ou $-CH(CO-Q')CH_2-$, |
| Q' | représente le benzylamino, le morpholino ou le tétra-jusqu'à heptaméthylèneimino, et |
| R, R', L et T | ont les mêmes significations que dans la revendication 1, |

ainsi que leurs hydrates ou leurs solvates et leurs sels physiologiquement utilisables.

**3.** Procédé selon la revendication 2, où X' est H, et R, M', L et T ont les mêmes significations que dans la revendication 2.

**4.** Procédé selon la revendication 1 pour préparer les guanidines de formule

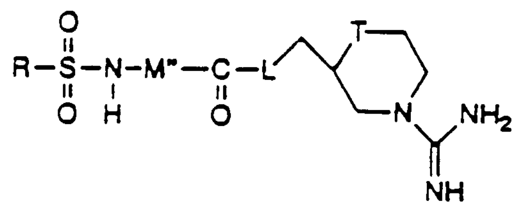

IB

où

| | |
|---|---|
| M" | représente un groupe $R''-COCH_2CH=$, $R'-(CO)_{1-2}NHCH_2CH=$, benzyl-$OCONHCH_2CH=$, $-CH_2[R'-(CO)_{1-2}NH]CH-$, $-CH_2$(benzyl-OCONH)CH- ou -CH-(CO-Q")$CH_2$-, |
| Q" | représente un groupe de tétra- à heptaméthylèneimino éventuellement interrompu par un atome de O ou de S et éventuellement substitué par jusqu'à 2 substituants choisis dans le groupe constitué par les alkyles en $C_{1-4}$, COOH, -COO-alkyle en $C_{1-4}$, $-CH_2OH$ et $-CH_2O$-benzyle, et |
| R, R', R", L et T | ont les mêmes significations que dans la revendication 1. |

**5.** Procédé selon la revendication 1, où R, L, T, M ou -N(X)-M- et X ont les mêmes significations que dans la revendication 1, mais X n'est pas H.

**6.** Procédé selon la revendication 1, où R est un aryle, en particulier le naphtyle, l'hydroxynaphtyle, le 4-biphényle, le 2-anthryle, le iodophényle, le nitrophényle, le bepzyloxyphényle, le diméthoxyphényle, le 4-méthoxy-2,3,6-triméthylphényle, le 2,4,6-triisopropylphényle, le carboxyphényle, le méthoxycarbonylphényle, le benzyloxynaphtyle, le phénylsulfonylphényle, l'hexahydroazépinoylphényle ou le t-butylphényle.

**7.** Procédé selon la revendication 1 où R est un hétéroaryle, en particulier le 3-méthyl-8-quinolyle, le 5-(1 méthyl-5-trifluorométhylpyrazole-3-yl)-2-thiényle ou le benzothiényle.

**8.** Procédé selon la revendication 1 ou 2, où R est un hétérocyclyle, en particulier le 3-méthyl-1,2,3,4-tétrahydro-8-quinolyle.

**9.** Procédé selon la revendication 1 où -N(X)-M- est l'isoquinolylène ou le N-diméthylaminonaphtylsulfonyl-aminométhylène.

**10.** Procédé selon la revendication 1 ou 2 où X est H ou $-CH_2COOH$.

**11.** Procédé selon la revendication 1, où M est un groupe $R'-(CH_2)_{1-2}CH=$, en particulier le 3-indolyléthylidène, le 2,3-dioxo-1-indolinyléthylidène, le phénéthylidène, le 1,4-dioxo-5H-2,5-benzodiazépine-5-yléthylidène, le (fluoro, chloro, iodo, cyano, nitro, amino, carboxy, alcoxy en $C_{1-4}$-carbonyle ou hydroxy)-phénéthylidène, le cyclohexylpropylidène le décalyléthylidène, l'imidazolyléthylidène, le thiényléthylidène, le (méthyl, bromo, fluoro ou carboxyméthyl)-3-indolyléthylidène, le naphtyléthylidène, le (éthoxycarbonylcarbonylamino, méthoxycarbonyléthylcarbonylamino, benzyloxycarbonyléthylcarbonylamino, éthoxycarbonylamino, benzoylcarbonylamino, carboxybenzoylamino, méthoxyéthoxyacétamido, acétamido, carboxycarbonylamino, carboxypropionylamino, tolylsulfonamido, iodophénylsulfonamido, carboxyphénylsulfonamido ou éthoxycarbonylméthylamino)phénéthylidène, l'oxobenzoxazolinéthylidène ou le 5-bromo- ou 5-méthyl-2,3-dioxo-1-indolinyléthylidène.

12. Procédé selon la revendication 1, où M est un groupe (R' ou R'')$COCH_2CH=$, en particulier l'hexahydroazépinoyléthylidène, le (méthoxycarbonyl ou carboxy)pyrrolidinoyléthylidène, le 3,4-dihydro-2(1H)-isoquinoléinoyléthylidène, le (nitro, amino, iodo ou formamido)benzoyléthylidène, le morpholinoéthylidène, l'heptahydroazocinoyléthylidène, le (éthoxycarbonyl, acétoxyméthyl, diméthylcarbamoyl, isobutyryloxyméthyl ou butyryloxyméthyl)pipéridinoyléthylidène, le 3-méthoxycarbonyl-4-oxopipéridinoyléthylidène ou le 4-acétoxy-3-éthoxycarbonylpipéridinoyléthylidène.

13. Procédé selon la revendication 1, où M est un groupe $R'\text{-}(CO)_{1-2}NHCH_2CH=$, en particulier le benzoylcarboxamidoéthylidène, le thiénoylcarboxamidoéthylidène, le benzoylamidoéthylidène ou le benzyloxycarboxamidoéthylidène.

14. Procédé selon la revendication 1, où M est un groupe $-CH_2[R'\text{-}(CO)_{1-2}NH]CH-$, en particulier le 2-(carboxybenzoylamido)éthylène, le 2-(benzyloxybenzoylamido)éthylène, le 2-(2-pipéridinecarboxamido)-éthylène, le 2-(hydroxybenzoylamido)éthylène ou le 2-(aminobenzoylamido)éthylène.

15. Procédé selon la revendication 1, où M est un groupe-$CH(CO\text{-}Q)CH_2$-, en particulier le 1-(benzylaminocarbonyl)éthylène, le 1-(hexahydroazépinoyl)-éthylène, le 1-(morpholinoyl)éthylène, le 1-(heptahydroazocinoyl)éthylène, le 1-[2-(benzyloxyméthylmorpholinoyl)]éthylène, le 1-[2-(hydroxyméthylmorpholinoyl)éthylène le 1-(2-éthoxycarbonyl-4-méthylpipéridinoyl)éthylène, le 1-(2-carboxy-4-méthylpipéridinoyl)éthylène ou le 1-(3-carboxyhexahydro-1,4-oxazépinoyl)éthylène.

16. Procédé selon la revendication 1,2 ou 6, où R est un aryle, en particulier le naphtyle ou le nitro- ou iodophényle, L est NH, et où l'atome de C asymétrique dans le cycle de la pipéridine ou de la morpholine a la configuration S.

17. Procédé selon la revendication 1, 11 ou 12 pour préparer les guanidines de formule

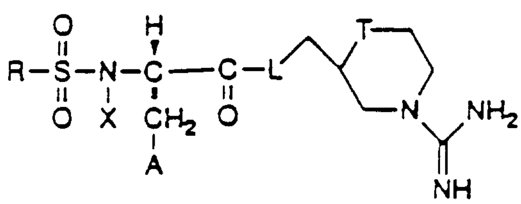

IC

où A est un aryle, un aroyle ou un hétérocyclyle, en particulier le phényle, le nitrophényle, l'indolyle, le 2,3-dioxo-1-indolinyle ou l'aminobenzoyle.

18. Procédé selon la revendication 1 ou 2, pour préparer les guanidines choisies à partir du groupe des composés suivants :
le (R)-N-[(RS)-1-amidino-3-pipéridinylméthyl]-α-(2-naphtylsulfonamido-2,3-dioxo-1-indolinepropionamide,
le (R)-N-[(RS)-1-amidino-3-pipéridinylméthyl]-α-(2-naphtylsulfonamido-o-nitrohydrocinnamamide,
le (R)-N-[(RS)-1-amidino-3-pipéridinylméthyl]-α-(o-nitrobenzènesulfonamido)indole-3-propionamide,
le (R)-N-[(S)-1-amidino-3-pipéridinylméthyl]-α-(p-iodobenzènesulfonamido)-indole-3-propionamide,
le (R)-N-[(S)-1-amidino-3-pipéridinylméthyl]-α-(p-iodobenzènesulfonamido)-p-nitrohydrocinnamamide,
le (R)-N-[(RS)-1-amidmo-3-pipéridinylméthyl]-3-(o-aminobenzoyl)-(2-naphtylsulfonamido)propionamide,
la N-[(R)-α-[[(S)-1-amidino-3-pipéridinyl]méthylcarbamoyl]phénéthyl]-N-(2-naphtylsulfonyl)glycine,
le (R)-N-[(S)-1-amidino-3-pipéridinylméthyl]-1,2,3,4-tétrahydro-2-(2-naphtylsulfonyl)-3-isoquinoléinecarboxamide,
le (S)-N-[(RS)-1-amidino-3-pipéridinylméthyl]hexahydro-β-(2-naphtylsulfonamido)-γ-oxo-1H-1-azépinebutyramide,

le (R)-N-[(S)-1-amidino-3-pipéridinylméthyl]-α-(2-naphtylsulfonamido)-2,3-dioxo-1-indolinepropionamide
l'acide 4'-[(R)-2-[[[(S)-1-amidino-3-pipéridinyl]méthyl]carbamoyl]-2-(2-naphtylsulfonamido)éthyl]-oxanilique,
le (S)-N-[[(RS)-1-amidino-3-pipéridinyl]méthyl]hexahydro-β-2-naphtylsulfonamido-γ-oxo-1(2H)-azocinbutyramide,
l'acide (2RS,4R)-1-[$N^4$-[[(S)-1-amidino-3-pipéridinyl]méthyl]-$N^2$-(2-naphtylsulfonyl)-L-asparaginyl]-4-méthyl-2-pipéridinecarboxylique,
l'acide 4'-[(R)-2-[[[(S)-1-amidino-3-pipéridinyl]méthyl]carbamoyl]-2-(2-naphtylsulfonamido)éthyl]-succinanilique.

**19.** Procédé pour fabriquer des préparations pharmaceutiques particulières pour utilisation comme médicaments, en particulier pour inhiber l'agrégation des plaquettes induite par la thrombine et la coagulation du fibrinogène dans le plasma du sang, caractérisé en ce l'on met sous une forme galénique une guanidine selon une des revendications 1 à 18 ou un sel de celle-ci.

**20.** Utilisation d'un composé selon une des revendications 1 à 18 pour préparer des médicaments pour le traitement ou la prophylaxie de maladies qui sont provoquées par l'agrégation des plaquettes induite par la thrombine ou la coagulation du fibrinogène dans le plasma du sang.

**21.** Composés des formules III, IV et V selon la revendication 1.